# EUROPEAN PATENT SPECIFICATION

(11) **EP 0 726 781 B1**
(45) Date of publication and mention of the grant of the patent: **22.08.2001**
(21) Application number: 95904770.5
(22) Date of filing: 29.11.1994
(51) Int. Cl.: A61K 51/06

(54) **IN VIVO AGENTS COMPRISING RADIOACTIVE METAL-ION CHELATES WITH ACIDIC SACCHARIDES AND GLYCOSAMINOGLYCANS, GIVING IMPROVED SITE-SELECTIVE LOCALIZATION, UPTAKE MECHANISM, SENSITIVITY AND KINETIC-SPATIAL PROFILES**
In Vivo Mitteln enthaltend radioaktive Metallionen - Chelaten mit Säure Saccariden und Glycosaminoglycanen gebende ein
AGENTS IN VIVO COMPRENANT DES CHELATES D'IONS DE METAUX RADIOACTIFS AVEC DES SACCHARIDES ET GLYCOSAMINOGLYCANES ACIDES, QUI AMELIORENT LA LOCALISATION SELECTIVE DES SITES, LE MECANISME DE FIXATION, LA SENSIBILITE ET LES PROFILS CINETIQUES DANS L'ESPACE

(30) Priority: 29.11.1993 US 160085
(43) Date of publication of application: 21.08.1996
(73) Proprietor: Access Pharmaceuticals Inc., Dallas, Texas 75207 (US)
(72) Inventor: RANNEY, David, F., Dallas, TX 75234 (US)
(74) Representative: Glawe, Delfs, Moll & Partner
(86) International application number: PCT/US94/13740
(87) International publication number: WO 95/14492

(56) References cited:
- EP-A- 0 055 028
- EP-A- 0 565 930
- WO-A-87/02893
- WO-A-91/15215
- WO-A-94/05203
- FR-A- 2 100 869
- FR-A- 2 554 348
- JP-A- 63 225 601
- CHEM. LETT. (1991), (7), 1189-92 CODEN: CMLTAG;ISSN: 0366-7022, 1991 AKIYAMA, MASAYASU ET AL '.beta.- Cyclodextrin bound retrohydroxamate ferrioxamines. Chiral iron(III) coordination and biological activity of synthetic siderophores'
- DATABASE CHEMABS CHEMICAL ABSTRACTS SERVICE, COLUMBUS, OHIO, US AN=109:31788, DOL, F. ET AL 'Pharmacokinetics of dermatan sulfate in the rabbit after intravenous injection' & THROMB. HAEMOSTASIS (1988), 59(2), 255-8 CODEN: THHADQ;ISSN: 0340-6245, 1988
- DATABASE CHEMABS CHEMICAL ABSTRACTS SERVICE, COLUMBUS, OHIO, US AN=72:97979, KRESSE, HANS ET AL 'Chemistry of the arterial wall. XV. Metabolic heterogeneity of carbon-14 and sulfur - 35 labeled glycosaminoglycans (acidic mucopolysaccharides) when incubated in vitro' & HOPPE-SEYLER'S Z. PHYSIOL. CHEM. (1970), 351(2), 151-6 CODEN: HSZPAZ, 1970

## Description

The present invention describes novel compositions, agents and their *in vivo* use which give improved selectivity, efficacy, uptake mechanism and kinetic-spatial profiles at sites of disease. It further describes compositions, agents and their use for improved selectivity, sensitivity, uptake mechanism and kinetic-spatial profiles of biomedical imaging, image contrast and spectral enhancement at sites of disease, including but not limited to magnetic resonance image (MRI) contrast enhancement. Novel compositions are prepared by (a) unique non-covalent chemical binding, further enhanced by (b) physical stabilization. Other compositions are prepared by covalent chemical binding. Binding is of cationic or chemically basic metal chelators to carriers comprising anionic or chemically acidic saccharides, sulfatoids and glycosaminoglycans, typically and advantageously of a hydrophilic or essentially completely hydrophilic nature. Binding of the active and carrier may also be by a combination of non-covalent, physical, and covalent means. Non-covalent binding can be carried out by means including but not limited to admixing cationic or basic metal chelators at appropriate ratios with anionic or acidic saccharide carriers, thereby forming solution-state and dry-state paired-ion salts, based principally on electrostatic binding of cationic (basic) group or groups of the metal chelator to anionic (acidic) group or groups of the acidic carrier. Such binding may be further stabilized by hydrogen bonds and physical factors, including but not limited to concentration, viscosity, and various means of drying, including lyophilization.

Carrier substances useful in this invention may include dermatan sulfates as defined in the claims. Due to an absence of water-diffusion barriers, favorable initial biodistribution and multivalent site-binding properties, oligomeric and polymeric, hydrophilic and substantially completely hydrophilic carrier substances are included among the preferred carriers for agents to be used for paramagnetic, T1-Type, selective MRI contrast of tumors, cardiovascular infarcts and other T1-Type MRI contrast uses. However, it will be apparent to those skilled in the art that amphoteric and hydrophobic carriers may be favored for certain biomedical imaging applications and therapeutic applications. Metal chelators useful in this invention include those which contain cationic, basic and basic-amine groups and which chelate metals and metal ions, transition elements and ions, and lanthanide series elements and ions. It will be apparent to those skilled in the art that essentially any single atomic element or ion amenable to chelation by a cationic, basic and amine-containing chelator, may also be useful in this invention.

For purposes of this invention, a cationic or basic metal chelator is defined and further distinguished from a metal-ion complex as follows: a cationic or basic metal chelator comprises an organic, covalent, bridge-ligand molecule, capable of partly or entirely surrounding a single metal atom or ion, wherein the resulting formation constant of chelator for appropriate metal or ion is at least about 10¹⁴. A chelator is further defined as cationic or basic if it or its functional group or groups which confer the cationic or basic property, and which include but are not limited to an amine or amines, is (are) completely or essentially completely electrophilic, positively charged or protonated at a typical pH employed for formulation. A formulation pH is characteristically selected to closely bracket the range of physiologic pH present in mammalian vertebrates. This typically includes, but is not limited to a pH in the range of pH 5 to 8. Amines may include primary, secondary, tertiary or quaternary amines or combinations thereof on the metal chelator. Herein, and as specified, a hydrophilic carrier is defined as a substance which is water soluble, partitions into the water phase of aqueous-organic solvent mixtures, or forms a translucent aqueous solution, complex, aggregate, or particulate dispersion under the conditions employed for formulation. A carrier is further defined as being anionic or acidic if it is completely or nearly completely nucleophilic, or if its functional group or groups capable of interacting with cationic, basic or amine metal chelators, is (are) completely or nearly completely negatively charged, anionic or ionized at the pH employed for formulation. Such anionic and acidic groups include, but are not limited to sulfates, phosphates and carboxylates, or combinations thereof on the carrier.

Agent compositions include, but are not limited to the'classes of cationic or basic, typically basic-amine metal chelator actives, or metal chelator actives including the chelated metal or metal ion, wherein these actives are further bound to anionic and acidic carriers comprising natural or synthetic carriers, including but not limited to hydrophilic anionic or acidic, natural or synthetic, native, modified, derivatized and fragmented, anionic or acidic saccharides, oligosaccharides, polysaccharides, sulfatoids, and glycosaminoglycans (GAGs).

Anionic and acidic saccharide and glycosaminoglycan carriers may contain monomeric units comprising glucose, glucuronic acid, iduronic acid, glucosamine, galactose, galactosamine, xylose, mannose, fucose, sialic acid, pentose, and other naturally occurring, semi-synthetic or synthetic monosaccharides or chemical derivatives thereof, comprising amine, sulfate, carboxylate, sialyl, phosphate, hydroxyl or other side groups. Glycosaminoglycans (GAGS) comprise essentially the carbohydrate portions of cell-surface and tissue matrix proteoglycans. They are derived from naturally occurring proteoglycans by chemical separation and extraction; and in certain instances, by enzymatic means [Lindahl *et* al. (1978). They include, but are not limited to those of the following types: heparin, heparan sulfate, dermatan sulfate, chondroitin-4-sulfate, chondroitin-6-sulfate, keratan sulfate, syndecan, and hyaluronate, and over-sulfated, hyper-sulfated, and other chemical derivatives thereof, as described further below. Strongly acidic glycosaminoglycans include all of those classes listed just above, except for hyaluronate, which contains only the more weakly acidic carboxylate groups and not sulfate groups. Natural sources of glycosaminoglycans include, but are not limited to: pig and beef intestinal mucosa, lung, spleen, pancreas, and a variety of other solid and parenchymal organs and tissues.

Sulfatoids comprise a second class of sulfated saccharide substances which are derived principally but not exclusively from bacterial and non-mammalian sources. Sulfatoids are typically of shorter chain length and lower molecular weight than glycosaminoglycans, but may be synthetically modified to give (a) longer chain lengths, (b) increased sulfation per unit saccharide, (c) various other chemical side groups, or (c) other properties favorable to the desired ligand-binding property and site-selective binding, uptake and accumulation property (or properties) *in vivo.* Sucrose and other short-chain oligosaccharides may be obtained from natural and synthetic sources.

These oligosaccharides can be rendered anionic or acidic by chemical or enzymatic derivatization with carboxylate, phosphate, sulfate or silyl side groups, or combinations thereof, at substitution ratios of up to about eight anionic or acidic substituent groups per disaccharide unit. Modified glycosaminoglycans may be derived from any of the types and sources of native glycosaminoglycans described above, and include: (1) glycosaminoglycan fragments, further defined as glycosaminoglycans with chain lengths made shorter than the parental material as isolated directly from natural sources by standard ion-exchange separation and solvent fractionation methods; (2) glycosaminoglycans chemically modified to decrease their anticoagulant activities, thereby giving "non-anticoagulant" (NAC) GAGs, prepared typically but not exclusively by (a) periodate oxidation followed by borohydride reduction; (b) partial or complete desulfation; and (c) formation of non-covalent divalent or trivalent counterion salts, principally including but not limited to salts of the more highly acidic sulfate functional groups, with principally but not exclusively: calcium, magnesium, manganese, iron, gadolinium and aluminum ions.

For purposes of this invention, a special class of such salts includes those salts formed by electrostatic or paired-ion association between the acidic or sulfate groups of acidic saccharide or glycosaminoglycan carrier, and the basic or cationic group or groups of the metal chelator or metal chelator including metal, as described above. Derivatized acidic saccharides and glycosaminoglycans are typically prepared by derivatization of various chemical side groups to various sites on the saccharide units. This may be performed by chemical or enzymatic means.

Enzymatic means are used in certain instances where highly selective derivatization is desired. Resulting chemical and enzymatic derivatives include, but are not limited to acidic saccharides and glycosaminoglycans derivatized by: (1) esterification of (a) carboxylate groups, (b) hydroxyl groups, and (c) sulfate groups; (2) oversulfation by nonselective chemical or selective enzymatic means; (3) acetylation, and (4) formation of various other ligand derivatives, including but not limited to (a) addition of sialyl side groups, (b) addition of fucosyl side groups, and (c) treatment with various carbodiimide, anhydride and isothiocyanate linking groups, and (d) addition of various other ligands.

If and when present, sulfate and sialyl side groups may be present at any compatible position of saccharide monomer, and on any compatible position of glycosaminoglycan monomers [Lindahl *et al.* (1978). Certain of the resulting derivatized acidic saccharides and glycosaminoglycans may have desired alterations of anticoagulant activities, site-localization patterns, clearance and other biological properties. As one. example of this relationship between certain classes of glycosaminoglycans and biological properties, dermatan sulfates with a native sulfate/carboxylate ratio preferably in the range of 0.7:1 to 1.8:1, more preferably between 0.9:1 and 1.5:1 and typically 1:1 , are reported to have relatively low binding to normal endothelial cells, avoid displacement of endogenous heparan sulfate from endothelial-cell surfaces, have relatively high selectivity to induced endothelia at sites of disease, including thrombus, and have rapid plasma clearance, principally by the renal route; whereas heparins and oversulfated dermatan sulfates with higher sulfate/carboxylate ratios of between 2:1 and 3.7:1, are reported to have relatively higher binding for both normal and induced endothelia, to displace relatively more endogenous endothelial heparan sulfate, and to clear more slowly than dermatans [Boneu *et al.* (1992).

As newly described and used in the present invention, the new special class of dermatan sulfates which contain selectively oversulfated oligosaccharide sequences, have the further unique advantages of higher potency combined with very low toxicity as carrier substances of associated or bound actives (*i.e*., dermatan sulfate-actives, DS-actives). This is related to their (a) relatively low sulfate/carboxylate ratios which range between 0.7:1 and 1.8:1, most preferably lying between 0.9:1 and 1.5:1, and most typically being 1:1; (b) very low anticoagulant activities -- related to very low factor Xa and USP heparin activity plus negligible binding to antithrombin III; (c) very low or absent platelet-aggregating, and hence thrombocytopenia-inducing properties -- related to their relatively low SO3-/COO- ratios in combination with a modal molecular of weight less than about 45,000 daltons and preferably less than about 25,000 daltons; (d) essentially complete absence of *in vivo* metabolism; and (e) very rapid blood and body clearance, all as further described below. These properties result in an extremely high in *vivo* safety profile with an absence of bleeding, metabolism and in *vivo* residua in normal tissues and organs. These properties and their resulting safety profiles clearly distinguish the dermatan sulfates from all other classes of glycosaminoglycans (GAGs) and other classes of acidic saccharides, oligosaccharides, polysaccharides and sulfatoid substances (taken together, comprising acidic and anionic saccharide substances), and they provide uniquely surprising and unexpected advantages for dermatan sulfates over these other classes of acidic and anionic saccharides. Most particularly, the dermatan sulfates show these surprising and unexpected advantages over other glycosaminoglycan polysulfates, with SO3-/COO- ratios in the range of between 2:1 and 3.7:1 and sulfur contents of greater than or equal to 10% (weight basis -- indicative of their much higher sulfate contents). Also, most particularly, the new special class of dermatan sulfates (as described at length below), which is enriched for selectively oversulfated oligosaccharide sequences without comprising oversulfated or polysulfated molecules overall throughout the entire chain length (the latter being characterized by SO3-/COO- ratios greater than or equal to 2.0:1 and sulfur contents greater than or equal 10%), have the further surprising and unexpected advantage of more strongly binding to the selectively induced receptors of endothelium, tissue matrix and target-cells at sites of disease (including tumors) by means of the complementary, selectively oversulfated oligosaccharide sequences of these new special dermatan sulfates. Hence, these new special dermatan sulfates exhibit surprisingly and unexpectedly more potent site localization and site-targeting potencies than would otherwise be expected based on their moderately low overall SO3-/COO- ratio and sulfation and on their related extremely low cellular and systemic toxicity properties and side-effect profiles.

In a special case unique to the present invention, derivatization of the acidic saccharide and glycosaminoglycan carriers may be accompanied by the basic metal chelator itself. The source and type of glycosaminoglycans, its chain length and sulfate/carboxylate ratio can be optimized to (1) provide optimal formulation characteristics in combination with different small and macromolecular diagnostic agents and drugs; (2) modulate carrier localization on diseased versus normal endothelium; (3) minimize dose-related side effects; (4) optimize clearance rates and routes of the carrier and bound diagnostic and therapeutic actives.

Non-covalent formulations of active and carrier afford markedly higher active-to-carrier ratios than those possible for covalent chemical conjugates. In the present invention, non-covalent binding affords a minimum of 15% active to total agent by weight [active / (active + carrier), w/w]; typically greater than about 30% (w/w); preferably at least about 50% (w/w); and frequently between about 70-99% (w/w). Covalent binding characteristically limits the percent active to (a) less than about 12% for non-protein small and polymeric carriers, (b) less than about 7% for peptide and protein carriers, including antibodies, and (c) less than about 0.5-2.0% for antibody fragments. This limitation is based on the number of functional groups available on carrier molecules which are useful in agent formulation and *in vivo* site localization.

It will be apparent to those skilled in the art that covalent active-carrier agent compositions of low substitution ratio may be useful for certain *in vivo* applications of typically narrow range, and that non-covalent active-carrier agent compositions of high substitution ratio may be useful for other *in vivo* applications of typically broader range. Generally, but not exclusively, covalent agents may be useful for radionuclide imaging or therapeutic applications in which only low total-body doses are needed, clearance of the non-targeted dose fraction does not cause undue toxicity, and high conjugate stability is required. Generally, but not exclusively, non-covalent agents may be particularly useful for the majority of diagnostic imaging applications and certain high-dose therapeutic applications, for which high total-body and site-localized doses are needed, and rapid clearance of the non-localized fraction of administered agent is desired in order to accelerate plasma clearance and to achieve low background levels for purposes of maximizing image contrast and minimizing systemic toxicity.

Rapid clearance is preferentially conferred by non-covalent physical formulations due to their capacity to give controlled dissociation or release of the active from the carrier. Such controlled release allows the diagnostic or therapeutic active, to dissociate from its carrier at a programmed rate which is consistent with rapid site localization of a significant fraction of the total administered dose. In instances where the carrier is polymeric and hence clears more slowly, this selectively accelerates clearance of the active.

It will be apparent to those skilled in the art that such controlled release can also be achieved for actives which are chemically conjugated to their carriers via chemical linkers, including peptide linkers, which are susceptible to cleavage by body enzymes. However, this latter means of facilitated clearance: (a) gives much longer clearance times than do physical formulations, (b) depends on endogenous enzyme levels and inhibitors which typically differ from subject to subject, from health to disease, and from one stage of disease to another. Hence, physical formulations have substantial advantages over chemical conjugates from the standpoints of both (a) high payload, and (b) accelerated clearance.

These properties of the present formulations represent additional substantial improvements over prior, non-selective and covalently conjugated active-carrier agents. The resulting agents are broadly useful for: (a) MRI contrast and spectral enhancement, Ultrasound contrast enhancement, and X-Ray contrast enhancement, where relatively high administered doses may be favored or required; (b) Nuclear Medical or Radionuclide imaging and therapy, where enhanced clearance of the non-targeted dose may be favored or required: and (c) certain high-dose, extended-release or sustained-effect therapy may be favored or required. Such therapeutic agents include but are not limited to those useful at a broad variety of organ sites and medical indications, for the treatment of: (a) acute vascular ischemia, acute infarct, acute vascular damage, shock, hypotension, restenosis, proliferation of neo-vessel, parenchymal cells or other pathological proliferations; and (b) the following classes of disease: vascular, parenchymal, mesenchymal, endothelial, smooth muscle, striated muscle, adventitial, immune, inflammatory, bacterial, fungal, viral, degenerative, neoplastic, genetic and enzymatic.

MRI contrast enhancement is one important indication for which high payload and controlled release of active are important unique advantages in addition to site selective localization (see below). A still further advantage is the hydrophilic form of carrier, which maximizes proximal water diffusion and binding of the paramagnetic active. This last property is required for optimal efficacy and minimal toxicity, because MRI paramagnetic T1-Type contrast agents require unimpeded water diffusion to within a very short distance of the localized metal ion in order to achieve effective paramagnetic relaxation and T1 contrast. Additionally, MRI image instrumentation and image acquisition are inherently both of low sensitivity; and these limitations remain even at the highest clinically acceptable field strengths and gradients and at the optimal radiofrequency pulse sequences.

MRI paramagnetic agents have been prepared as stabilized liposomes, which contain up to about 22% of active (w/w). However, their hydrophobic lipid bilayers markedly impede water diffusion into the liposome core active. This decreases their efficacy per unit dose relative to the hydrophilic controlled-release carriers of the present invention. There is an additional disadvantage of the reported MRI liposome formulations as follows: aside from localization in normal liver and reticuloendothelial-phagocytic organs, they have not demonstrated effective site-localization at sites of tumors, infarcts and other focal pathology within tissue sites.

For purposes of this invention, metal ions generally useful for chelation in paramagnetic T1-Type MRI contrast agent compositions and uses may include divalent and trivalent cations selected from the group consisting of: iron, manganese, chromium, copper, nickel, gadolinium, erbium, europium, dysprosium and holmium. Chelated metal ions generally useful for radionuclide imaging and compositions and uses, and in radiotherapeutic compositions and uses, may include metals selected from the group consisting of: gallium, germanium, cobalt, calcium, rubidium, yttrium, technetium, ruthenium, rhenium, indium, iridium, platinum, thallium and samarium. Metal ions useful in neutron-capture radiation therapy may include boron and others with large nuclear cross sections. Metal ions useful in Ultrasound contrast and X-Ray contrast compositions and uses may, provided they achieve adequate site concentrations, include any of the metal ions listed above, and in particular, may include metal ions of atomic number at least equal to that of iron.

For purposes of this invention, agents for therapeutic composition and uses in chelating internal body iron, copper or both, in order to make these metals unavailable locally (1) which are typically required for neovascularization, or (2) which cause and amplify local tissue injury [Levine (1993), include the carrier with basic metal chelator in one or both of the following forms: (a) carrier plus chelator without metal ion; and (b) carrier plus chelator with metal ion added and chelated in the composition at a formation constant lower or equal to that of the internal body metal which is to be chelated by metal ion exchange into the respective basic metal chelator of the composition (see below). Such weakly chelated metal ions of the composition may include one selected from the group consisting of: calcium, manganese, magnesium, chromium, copper, zinc, nickel, iron, aluminum, cobalt, gadolinium or other exchangeable ion. Metal ions useful for inclusion in compositions for other therapeutic uses may include the divalent and trivalent cations selected from the group consisting of magnesium, manganese, chromium, zinc and calcium, iron, copper and aluminum. It will be obvious to those skilled in the art that various ones of the preceding metal ions can be used in combination with basic metal chelators, for alternative indications than those specified above, and that metal ions other than those listed above may, under certain conditions, be useful in the uses and indications listed above.

The compositions described in this invention give surprising and unexpected improvements of performance and use which include:
(1) retained high association of active plus carrier during *in vitro* dialysis and *in vivo* targeting;
(2) selective binding of the active plus carrier to induced endothelia at sites of disease;
(3) following intravenous administration, very rapid (2-7 min) localization at the diseased site, due to rapid selective endothelial binding, envelopment and extravasation of the carrier plus metal chelator across disease-induced endothelia (including histologically non-porous endothelia);
(4) widespread uptake throughout the diseased tissue site;
(5) sustained retention (multiple hours to days) within the diseased site in combination with
(6) rapid plasma clearance (minutes) of the non-targeted fraction;
(7) moderately slow, polymeric diffusion rates within the diseased tissue matrix, allowing differentiation of functional tissue subregions based on differences in perfusion of viable and non-viable subregions;
(8) capacity to selectively image solid tumors or acute vascular and myocardial infarcts at body sites, as well as at brain and central nervous system sites, with substantially improved selectivity, sensitivity, improved delineation of tumor and infarct boundaries at both very short and prolonged post-injection intervals, and improved detection of small tumor metastases, including those at liver and lung sites.

Diagnostic and drug enhancement can be made to occur by a number of mechanisms, the principal ones being:
1. Effective TARGETING to tissue sites of disease;
2. STABILIZATION during both storage and plasma transit;
3. Prolonged RETENTION at the site of disease, giving a markedly increased area under the curve at the tissue site;
4. RAPID CLEARANCE of the non-TARGETED fraction, thereby reducing background signal in imaging applications and reducing normal organ exposure and systemic toxicity in therapeutic applications.

Five further significant advantages of the present compositions and uses are:
1. Simple formulations of active and carrier;
2. Stabilization of diagnostic and therapeutic actives on the shelf and during plasma transit;
3. Rapid site localization and sustained site retention;
4. Rapid clearance of the non-targeted fraction;
5. Availability of low toxicity carbohydrate carriers from natural sources and, where needed, modification or derivatization by straightforward synthetic means.

Acidic or anionic saccharides and glycosaminoglycans have unique mechanisms of site localization and retention *in vivo*. They bind to the body's endothelial determinants which are selectively induced on the microvascular barrier by underlying tissue disease. Previous approaches to site targeting were directed at antigenic determinants. However, because these determinants are typically located in sequestered sites within the tissues, in other words at sites across the endothelial barrier and not within the bloodstream and not on its endothelial surface, carriers and agents injected into the bloodstream had no effective means to recognize and localize in the region of these target antigens. Stated another way, previous approaches ignored the major problem of inappropriate carrier distribution which resulted from its failure to recognize the vascular access codes required for efficient extravasation at disease sites. Hence, these carriers failed to effectively load the relevant tissue sites with effective concentrations of their bound actives.

Acidic or anionic saccharides, including glycosaminoglycans, dermatan sulfates and the new special dermatan sulfates, localize at target sites by binding first to complementary receptors on disease-site vascular endothelium, induce very rapid (ca. 3-minute) extravasation of the carrier and associated active agent, and then widely permeate throughout the underlying tissue matrix, forming a depot reservoir of the carrier-agent selectively at the site of disease (including tumors -- even at sites up to several hundred micrometers distant from the typically irregularly spaced and perfused microvessels within the tumor matrix), and thirdly, bind to complementary receptors on the final target cells (including tumor cells), leading to induced tumor-cell internalization of GAG-actives (including DS-actives) (see Examples below)., The new special class of dermatan sulfates (described just above and more extensively below) appears to perform this complementary binding function via their selectively enriched oversulfated saccharide sequences, which correlate with an enriched heparin cofactor II activity of at least about 220 U/mg, and which appear to bind the positively charged, cationic and/or structurally complementary receptors or lectins that are selectively induced on disease-site endothelium, tissue matrix and target cells (including in tumors). For the new dermatan sulfates, these binding and targeting functions and potencies occur without either the overall high sulfation/polysulfation or the incumbent toxicity and safety disadvantages thereof (as otherwise described herein).

The biological address of a disease site can be viewed in a fashion similar to that of a postal address, wherein effective carrier substances must (1) first recognize the "state" address of the signal endothelium induced by proximal tissue disease; (2) next extravasate and load the "city' address of the extracellular tissue matrix with locally effective doses of the diagnostic and therapeutic actives; and (3) finally bind and load the "street" address of the target cells and antigens. Previous approaches to site delivery have attempted to recognize the "street" address without first recognizing the "state" and "city" addresses.

The reason that acidic saccharide and glycosaminoglycan systems work substantially better than previous antigen-recognition approaches, is that they recognize the newly induced signals which the body uses to attract and target white blood cells into sites of tissue disease. When disease strikes at a local site, it initiates a cascade of local mediators within the tissue matrix and at the endothelial-blood interface which signal the blood cells and central body systems that inflammatory and immune cells are required within the tissue site. These mediators include cytokines, chemoattractants, cytotoxins, induced cell-surface adhesions, selections and integrins, and various tissue-derived and blood-borne, soluble and cell-surface procoagulants. White cell accumulation begins within minutes and continues over days to weeks, depending on the nature, severity and persistence of local disease and the continued generation of tissue mediators and transendothelial signals.

As has now been reported and reviewed in detail [Ranney (1990); Ranney (1992); Bevilaqua *et al.* (1993); Bevilaqua *et al.* (1993); Travis (1993); Sharon *et al.* (1993), tumors, infarcts, infections, inflammatory diseases, vascular disorders, and other focal diseases, characteristically induce the release of such host mediators, or cytokines, from resident macrophages and local tissue matrix. In certain diseases, alien mediators such as bacterial lipopolysaccharides (LPS), viral RNA, and tumor-derived inducers, including EMAP II, and chemoattractants may also be released. Although additional mediators remain to be elucidated, the principal ones have now been defined and include: interleukin 1 (IL-1), tumor necrosis factor (TNF), vascular endothelial growth factor/vascular permeability factor (VEGF/VPF), transforming growth factor beta (TGF-beta), Lipopolysaccharide (LPS), single and double stranded nucleotides, various interferons, monocyte chemoattractant protein (MCP), interleukin 8 (IL-8), interleukin 3 (IL-3), interleukin 6 (IL-6), tumor-derived inducers and chemoattractant peptides (as above), various prostaglandins and thromboxanes. Certain ones of the preceding mediators induce the local generation and release of metalloproteinases, and these in turn, expose latent tissue binding sites, including intact and partially cleaved integrins, RDGS peptides, laminin, collagen, fibronectin, and cell-surface core-protein components of glycosaminoglycans.

Cytokines, including VEGF/VPF and monocyte chemoattractant protein (MCP); and tissue metalloproteinases and proteolytic tissue matrix fragments, directly induce the local endothelium to become adhesive for circulating white blood cells, including neutrophils, monocytes and lymphocytes. The induced endothelial adhesive molecules (adhesins) include: P-selectin (gmp-140), E-selectin (ELAM-1), intercellular cell adhesion molecule (ICAM-1), vascular cell adhesion molecule (VCAM-1), inducible cell adhesion molecule, (INCAM-110), von Willebrand's factor (vWF, Factor VIII antigen) (see below for disease states which activate these respective types of endothelial adhesins). Additional cascades become activated which indirectly amplify endothelial adhesiveness. These include (1) coagulation factors, especially fibronectin, tissue factor, thrombin, fibrinogen, fibrin, and their split products, especially fibronectin split products and fibrinopeptide A; (2) platelet-derived factors: platelet activating factor (PAF), glycoprotein IIb/IIIa complex; (3) white-cell (a) L-selectin, and (b) integrins, including VLA-4 (very late antigen 4); and (4) numerous complement factors.

The preceding pathologic processes and signals are involved, directly or indirectly as follows, in the binding and site localization of acidic carriers, including acidic saccharides (AC) and-glycosaminoglycans (GAGs) (Note that in the following outline, potential tissue binding sites are designated as "GAGs" and "ACs").
1. Local tissue diseases induce local cytokines and mediators, as described above. In particular, it is reported recently that the cytokine, vascular endothelial growth factor/vascular permeability factor (VEGF/VPF), is selectively induced by many or most tumors of human and animal origin [Senger *et al.* (1994) and is a 34-42 kDa heparin-binding and GAG-binding glycoprotein that acts directly on endothelial cells by way of specific endothelial receptors [Jakeman *et al*. (1993), to cause endothelial activation and induce additional new endothelial receptors which can bind GAGs (see below). VEGF/VPF is a chemically basic growth factor which is quite highly selective for endothelial cells versus fibroblasts and other cell types [Senger *et al.* (1994); Nicosia et *al.* (1994). It appears to be a key growth factor for stimulating the long-term endothelial angiogenesis in many or most human and animal tumors, and in AIDS-associated Kaposi's sarcoma [Connolly *et al.* (1989); Weindel *et al.* (1992). In certain instances, VEGF/VPF may also be important for the more transient and anatomically restricted angiogenic processes of wound healing and vascular restenosis [Senger *et al.* (1994); Miller *et al.* (1994); Nicosia *et al.* (1994); Berse *et al.* (1992). VEGF/VPF and platelet-derived growth factor, PDGF-BB, are reported recently to be the only species of the group of basic, GAG-binding growth factors which have significant angiogenic potency *in vitro*, *i.e.,* ones which are directly action in the absence of *in vivo* cofactors [Nicosia et *al*. (1994). The effects of VEGF/VPF are inhibited by antibodies directed against certain peptides on the external surface of the molecular [Sioussat *et al.* (1993)], and importantly, such inhibition suppresses the growth of animal tumors in vivo [Kim *et al.* (1993)]. Hence, VEGF/VPF both provides and induces receptor targets for binding of GAG carrier substances in tumors and potentially in other pathologic lesions.
2. These cytokines and mediators induce tissue chemoattractants, including VEGF/VPF, MCP (Yamashiro *et al.,* 1994) and IL-8, which comprise a family of arginine-rich, 8Kd, heparin-binding proteins reported to bind GAGs/ACs [Huber *et al.* (1991) ;
3. The cytokines and mediators of No. 1, above, induce the local endothelium to express P-selectin, the vascular cell adhesion molecular (VCAM-1), inducible cell adhesion molecule (INCAM-110), and von Willebrand's factor (vWF, Factor VIII antigen), which are reported binding determinants for GAGs/ACs [Bevilaqua et *al*. (1993); Bevilacqua *et al.* (1993)]; P-selectin is reported to bind GAGS [Bevilacqua *et al.* (1993)];
4. Integrins, including but not limited to VLA-4, are induced on circulating white blood cells, including lymphocytes, during various disease processes (see below); VLA-4 has a distinct binding site on the (induced) endothelial selectin, VCAM-1 (see No. 3, above); fibronectin, which is abundantly present in plasma and also available from tissue sites, has a distinct and separate binding site on VLA-4 [Elices *et al.* (1990)]; since fibronectin has specific binding sites for GAGs/ACs [Bevilaqua et al. (1993)], these amplification steps provide a strong additional mechanism for site localization of GAGs/ACs;
5. The chemoattractants, MCP and IL-8, lymphocyte integrin, VLA-4, platelet factor, PAF, and coagulation factors, thrombin, fibronectin and others, diffuse from local tissue and blood, respectively, bind to the induced endothelial selections, and amplify adhesiveness and activation at the initial endothelial P-selectin sites for GAGs/ACs [Elices *et al.* (1990); Lorant et al. (1993)];
6. Tissue metalloproteinases become activated and expose new binding sites for GAGs/ACs in the tissues which underlie the activated endothelia. These new tissue binding sites include as follows [Ranney (1990); Ranney (1992); Travis (1993); Bevilaqua *et al.* (1993)]:
   a. fibronectin fragments;
   b. collagen fragments;
   c. laminin fragments;
   d. RGDS peptides;
   e. Exposed core proteins of GAGs;
7. White blood cells are attracted to the site, become activated and release additional proteolytic enzymes, thereby amplifying No. 6 and increasing the exposure of binding sites for GAGs/ACs in the tissue matrix.
8. GAG/AC carriers selectively bind the induced and exposed determinants listed in Nos. 1-7, above, giving immune-type localization which is specific for induced binding sites (lectins) at the lectin-carbohydrate level characteristic of white-cell adhesion;
9. In cases where the carrier substance has multivalent binding to the target binding substance, including for example, cases in which the carrier is an acidic oligosaccharide or polysaccharide or an acidic glycosaminoglycan, multivalent binding of the endothelial surface induces rapid extravasation of the carrier and bound active, and results in substantially increased loading of the underlying tissue matrix, relative to that achieved by antibodies, liposomes, and monovalent binding substances, such as hormones and monovalent-binding sugars;
10. Adhesion of GAGs/ACs to induced and exposed tissue binding sites, reduces plasma backdiffusion of GAGs/ACs and their bound actives, thereby giving sustained retention within the tissue site;
11. Controlled release of the diagnostic or drug active from carriers comprising GAGs/ACs occurs gradually within the diseased site, thereby resulting in targeted controlled release;
12. Tumor cells, microbial targets and damaged cellular targets within the tissue site, may selectively take up the GAG/AC plus bound diagnostic or drug active, based respectively, on: induced tumor anion transport pathways, microbial binding sites for GAGs/ACs, and proteolytically exposed cell-surface core proteins [Ranney 07/880,660, 07/803,595 and 07/642,033] -- Fe uptake by hepatomas, Cr4S uptake by prostatic adenocarcinomas; [Kjellen *et al.* (1977)]
13. In cases where the carriers are hydrophilic or essentially completely hydrophilic, these carriers cause their bound actives to migrate (permeate) deeply into and throughout the tumor mass even at microanatomic sites distant from the tumor's typically irregularly spaced microvessels; and also to migrate out (permeate) into a small rim of normal tissue around each focus of disease, typically comprising a rim about 30-75 um thick; however, such carriers and/or their associated active substances (diagnostics or therapeutics) undergo selective uptake (internalization) by abnormal cells within tissue site and preferentially avoid uptake by normal cells within the site, thereby giving:
   a. In cases of diagnostic imaging applications: very sharp definition of the boundary between tumors or infarcts and the surrounding normal tissues;
   b. In cases of therapeutic applications:
      (1) protection against spread of disease at the rim;
      (2) relative protection of normal cells within and adjacent to the site of disease, from uptake of cytotoxic drugs.
14. In the case of hydrophilic carriers, including but not limited to GAGs/ACs, the non-targeted fraction of active is cleared rapidly and non-toxically, thereby minimizing:
   a. in imaging uses, background signal intensity;
   b. in all uses:
      (1) normal organ exposure; and
      (2) systemic side effects.
Regarding the above outline, the tumor-selective GAG-binding cytokines, VEGF/VPF and MCP, are now known to be present in all three of the following microanatomic locations: tumor-cell surface, tumor extracellular matrix, and local tumor neovascular endothelium. Hence, these cytokines provide receptor targets for GAG-agents at all three of the key tumor sites: tumor endothelium, tumor extracellular matrix, and tumor cells proper. the presence of these cytokines selectively on tumor -- endothelium, allows fore site-selective binding of intravascularly administered GAG-agents to tumor microvessels and very rapid (ca. 3-minute) selective extravasation of GAG-agents across the VEGF/VPF-"permeabilized" endothelium. Note: such "permeabilization" is recently shown to actually (a) comprise rapid transport by vesicular endosomes which are markedly enlarged (over the standard 120nm Palade vesicles characterizing normal endothelium) and markedly increased in number (over normal vascular endothelium) [Senger et al. (1993)]; and (b) comprise anatomically non-porous vascular endothelium, as assessed by macromolecular and particulate markers of true microfiltration porosity. The present of VEGF/VPF and MCP cytokines on tumor cell surfaces may account of selective tumor-cell internalization of GAG-agents, as shown in certain of the Examples below. Importantly, the presence of these cytokines plus the GAG-binding peptides of No. 6 (above) in the large extracellular volumes of the tumor matrix, accounts in part, for the large tumor-tissue reservoirs of GAG-associated agents (including metal chelates) which are observed by MRI contrast enhancement (see Examples below). The relatively slow (ca. 7-hour) backdiffusion of such agents into the bloodstream, further corroborates the present of such extracellular tissue-matrix receptors. Importantly, the combination of: (1) prolonged tumor retention of Gag-agents as an extracellular reservoir (depot); (b) tumor-cell internalization of a portion of this extracellular agent; and (c) very rapid blood and body clearance of the non-targeted portion, provides the following surprising and unexpected advantages for *in vivo* imaging (including MRI contrast enhancement) and therapy: (a) enhanced tumor selectivity; (b) prolonged, high "areas under the curve" (AUC's) in tumor; (c) short, low ACus in blood; (d) minimization of local and systemic toxicities. Additionally, involve the above outline, the following (A) cytokines and mediators; and (B) selections, integrins and adhesins are reported to be induced by various disease states in addition to that reported for tumors, above [Bevilaqua *et al*. (1993)]. Representative non-oncologic induction also occurs as follows.
A. Cytokines and mediators.
   1. MCP: Experimental autoimmune encephalomyelitis in mice [Ransohoff *et al.* (1993)];
   2. IL-8: Neovascularization: [Strieter *et al.* (1992)] ;
   3. PAF: Reperfused ischemic heart [Montrucchio et al. (1993)].
B. Selections, Integrins and Adhesins.
   1. ELAM-1:
      a. Liver portal tract endothelia in acute and chronic inflammation and allograft rejection [Steinhoff *et al. (1993)];*
      b. Active inflammatory processes, including acute appendicitis [Rice *et al.* (1992)].
   2. VCAM-1:
      a. Simian AIDS encephalitis [Sasseville *et al.* (1992)] .
      b. Liver and pancreas allograft rejection [Bacchi *et al.* (1993)].
   3. INCAM-110: Chronic inflammatory diseases, including sarcoidosis [Rice *et al.* (1991)].
   4. Integrin, beta 1 subunit cell adhesion receptor: inflammatory joint synovium [Nikkari *et al.* (1993)].
It is apparent from the above, that broad categories and many specific types of focal tissue disease may be addressed by the carriers and actives of the present invention, both for diagnostic and therapeutic uses, including tumors, cardiovascular disease, inflammatory disease, bacterial and viral (AIDS) infections, central nervous system degenerative disorders, and allograft rejection. It will also be obvious to those skilled in the art, that numerous additional disease states may be selectively addressed by the carriers disclosed in this invention.

The site selectivity of glycosaminoglycans (GAGs) appears to mimic an immune mechanism at the level of white-cell targeting rather than antibody targeting. Because antibodies have extremely high specificities, they characteristically miss major subregions of disease foci (typically as great as 60% of tumor cells are nonbinding). Recently, one of the GAG-binding determinants of endothelial P-selectin has been identified as sialyl Lewis x. Others are in the process of identification. Notably, the available nonvalent oligosaccharides specific for sialyl Lewis x suffer from two critical problems:
1. They are exceedingly expensive materials, available only by synthetic or semi-synthetic means, and hence, are not cost effective;
2. They do not bind effectively at diseased sites under *in vivo* conditions, apparently due to the inability as monomeric binding substances to displace endogenous interfering substances which are pre-bound at these sites.

There are two apparent benefits of the relatively broader range of GAG specificities and redundancy of GAG binding sites present on diseased endothelium, tissue matrix and cells:
1. GAGs allow a broader range of tumors and diseases to be targeted than that possible with antibodies (which typically target only a subset of histologic types -- even within a given class of tumor, and hence, are typically ineffective from both a medical and cost/development standpoint);
2. GAGs are projected to be effective over a greater time interval, from early onset of disease to progression and regression.

Despite the broader targeting specificity of GAGs over antibodies, their favorable clearance and avoidance of uptake by normal cells reduce systemic and local toxicities, even though more than one type of disease site may undergo targeted accumulation of the diagnostic/drug within its extracellular matrix.

The polymeric and multivalent binding properties of GAGs both are very important for optimal site localization of the attached diagnostic/drug. GAG molecular weights of generally ca. 8,000 to 45,000 MW, preferably 10,000 to 23,000 MW and more preferably 13,000 to 19,000 MW, are important in order to:
1. Restrict initial biodistribution of the diagnostic/drug to the plasma compartment and thereby maximize the quantity of agent available for site targeting;
2. Displace endogenous interfering substances which are pre-bound to diseased endothelium;
3. Induce active endothelial translocation of the GAG-diagnostic/drug into the underlying tissue matrix;
4. Afford rapid clearance and markedly reduced side effects of the attached actives.

### SUMMARY OF THE INVENTION

The present invention is defined in the claims. It encompasses novel agents comprising a radioisotopic metal-ion drelate having at least one excess basic or cationic group not involved in metal-ion drelation and being bound to selectively oversulfated dermatan sulfate, said oversulfated dermatan sulfate comprising oversulfated oligosaccharide sequences of L-idusonic acid -2-sulfate/galactosamine-4-sulfate or Ido-2-SO₃⁻,GalNac-4SO₃- disaccharides. The binding of the metal chelators to the carriers is stabilized by covalent or non-covalent chemical and physical means. In some embodiments, novel non-covalently bound compositions give uniquely high payloads and ratio of metal chelator to carrier, ranging from a low of about 15% metal chelator by weight, to a characteristic range of 70% to 90% metal chelator by weight. Specific embodiments comprise deferoxamine, ferrioxamine, iron-basic porphine, iron-triethylenetetramine, gadolinium DTPA-lysine, gadolinium N-methyl-1,3-propanediamine (N-MPD)-DTPA, gadolinium DOTA-lysine and gadolinium with basic derivatives of porphyrins, porphines, expanded porphyrins, Texaphyrins and sapphyrins as the basic or cationic metal chelators, which are in turn, bound to carriers, as defined in the claims.

Methods of magnetic resonance image (MRI) contrast enhancement are a particular embodiment of the present invention which confirm very rapid, carrier-mediated, site-selective *in vivo* localization and sustained site retention of metal-chelator compositions, based on stable binding of the metal chelator and carrier during in *vivo* plasma transit, allowing site localization following intravenous administration. Rapid and selective endothelial-site binding, facilitated rapid extravasation into underlying tissue sites, site accumulation, sustained site retention, together with rapid clearance of the non-site-localized fraction are also demonstrated by the use of the compositions of the present invention in the selective MRI contrast enhancement of tumors and cardiovascular infarcts.

Surprising and unexpected improvements of selectivity, mechanism of localization and cellular uptake, and MRI contrast sensitivity are shown for metal chelates having standard paramagnetic potencies. Further advantages of the use of the compositions and methods of the present invention are delineated in the examples *(infra)* including special histologic staining evidence which confirms the site-selective endothelial binding, extravasation, tissue matrix accumulation and cellular uptake mechanism. Selective localization and MRI imaging efficacy are also shown to occur when paramagnetic metal chelator actives are administered in carrier-bound form but not in free form.

In its most general embodiment, the present invention is an agent comprising a chelator for metal ions, said chelator having a cationic group and being bound to an anionic, hydrophilic carrier. In alternate embodiments, the chelator for metal ions which has a cationic group is bound to an anionic, hydrophilic carrier by non-covalent electrostatic binding. And, in certain alternate embodiments the invention comprises an agent comprising a basic chelator for metal ions, said chelator having a cationic group and being covalently bound to an anionic, hydrophilic carrier. In the particular embodiments of the invention in which the chelator is not covalently bound to the carrier, the said chelator may be basic.

In certain embodiments of the present invention, the agent which comprises a chelator for metal ions and having a cationic group bound to an anionic hydrophilic carrier may further comprise a chelated metal ion, and in particular it may further comprise a paramagnetic metal ion. The agents of the present invention, in particular those which comprise the chelator for metal ions non-covalently bound to the carrier may be further defined as being at least about 15 weight percent chelator. Preferably, the chelator has a formation constant for paramagnetic metal ions of at least about 10¹⁴.

Those agents of the present invention which comprise a metal ion will preferably comprise a metal ion selected from the group consisting of iron, manganese, chromium, copper, nickel, gadolinium, erbium, europium, dysprosium and holmium. In certain embodiments, the agents of the present invention may even comprise a metal ion selected from the group consisting of boron, magnesium, aluminum, gallium, germanium, zinc, cobalt, calcium, rubidium, yttrium, technetium, ruthenium, rhenium, indium, iridium, platinum, thallium and samarium. It is understood that other metal ions which are functionally equivalent to the listed metal ions are also included and would fall within the scope and spirit of the presently claimed invention.

In certain preferred embodiments of the invention, the agents comprise a carrier wherein said carrier is an essentially purified dermatan sulfate with a sulfur content of up to 9% (w/w) and with selective oligosaccharide oversulfation as defined in the claims.

In certain embodiments of the invention, the chelator is a chelator of iron ions. Preferably the chelator is a hydroxamate, and more preferably it is deferoxamine. In certain preferred embodiments the chelator together with the metal ion is ferrichrome, ferrioxamine, enterobactin, ferrimycobactin or ferrichrysin. In a particularly preferred embodiment, the chelator is deferoxamine.

In a certain embodiment, the invention may also comprise deferoxamine bound to a carrier being essentially purified dermatan sulfate with a sulfur content of up to 9% (w/w) and with selective oligosaccharide oversulfation as defined in the claims and may further comprise a metal ion. The agents of the present invention may also comprise a chelator which is a porphine, porphyrin, sapphyrin or texaphyrin and which may further comprise a metal ion, and preferably an iron ion or a gadolinium ion.

In a particularly preferred embodiment the agent of the present invention may comprise a chelator which is 5,10,15,20-Tetrakis (1-methyl-4-pyridyl) -21H,23H-porphine. In certain embodiments, the chelator may also be a polyaminocarboxylate or macrocyclic, and preferably a basic or amine derivative of diethylenetriaminetetraacetate, or more preferably a basic or amine derivative of 1,4,7,10-tetraazacyclododecane-N,N',N",N"'-tetraacetate (DOTA). In the agents of the present invention, the carrier may also be defined further as being complementary to endothelial determinants selectively induced at disease sites.

Alternatively, the invention is a spectral-enhancing agent to enhance images arising from induced magnetic resonance signals, the agent comprising Gd(III)diethylenetriaminepentaacetate covalently conjugated to dermatan sulfate with selective oligosaccharide oversulfation as defined in the claims. In another alternative, the invention is an agent for in *vivo* imaging, the agent comprising a basic chelator for metal ions and chelated metal ion, said chelator being bound by non-covalent electrostatic binding to a hydrophilic carrier as defined in the claims. The agent for enhancing body imaging preferably comprises deferoxamine, chelated Fe(III) and a glycosaminoglycan carrier as defined in the claims bound to said deferoxamine. The Fe(III) is a radiopharmaceutical metal ion, and most preferably the radiopharmaceutical metal ion is ⁵⁹iron or ⁶⁷gallium.

In an alternate preferred embodiment, the invention is an agent for enhancing body imaging, the agent comprising diethylenetriaminepentaacetate-lysine, chelated Gd(III) and a glycosaminoglycan carrier as defined in the claims bound to said diethylenetriaminepentaacetate-lysine. Alternatively, the invention is an agent for enhancing body imaging, the agent comprising DOTA-lysine, chelated Gd(III) and a glycosaminoglycan carrier bound to said 1,4,7,10-tetraazacyclododecane-N,N',N",N"'-tetraacetatelysine (DOTA-lysine). In a particular embodiment, the invention is an agent comprising ferrioxamine bound by non-covalent electrostatic binding to dermatan sulfate with selective oligosaccharide oversulfation as defined in the claims.

In an additional preferred embodiment, the invention is an agent for enhancing body imaging, including MRI imaging and spectral shift, the agent comprising gadolinium (III) chelated to N-methyl-1,3-propanediamine-diethylenetriaminepentaacetate (N-MPD-DTPA), the N-MPD-DtPA being bound or in association most preferably by paired-ion or other non-covalent means or alternatively preferably bound by covalent means to the new special class of dermatan sulfates containing selectively oversulfated oligosaccharide sequences.

It is understood that any of the agents of the present invention as described in the above paragraphs or in the appended claims may be defined further as being in a combination with at least one of a buffer, saccharide, sulfated saccharide, or salt, to produce an osmotic strength suitable for parenteral administration, and as being an aqueous solution or a lyophilized or dry preparation suitable for aqueous reconstitution having the desired osmotic strength, and wherein said agent is aseptic or sterile.

A preferred application of the invention is a method of enhancing magnetic resonance images or spectra in vertebrate animals comprising administering to said animal an effective amount of an agent of the invention which comprises the metal ion chelator, the carrier as described and a paramagnetic ion. The method of enhancing *in vivo* images arising from induced magnetic resonance signals, comprises the steps of administering to a subject an effective amount of an agent of the present invention which comprises a paramagnetic ion, exposing the subject to a magnetic field and radiofrequency pulse and acquiring an induced magnetic resonance signal to obtain a contrast effect.

Another application of the invention is a method of enhancing *in vivo* images, comprising the steps of administering to a subject an effective amount of an agent of the present invention which comprises a chelated metal ion, exposing the body to ultrasound or X-rays and measuring signal modulation to obtain a contrast effect.

Another application of the invention is a method of obtaining *in vivo* body images comprising administering to a subject an effective amount of an agent of the invention which comprises a metal ion wherein the metal ion is a radioisotope admeasuring scintigraphic signals to obtain an image.

Another application of the invention is a method of treating vascular disease, comprising administering to a subject a therapeutically effective amount of an agent of the present invention, and preferably an agent which comprises a metal ion.

### BRIEF DESCRIPTION OF THE DRAWINGS

The following drawings and figures are presented to illustrate preferred embodiments of the present invention and their uses in MRI contrast enhancement. These examples are purely illustrative, and do not in any way delimit the full scope of the present invention.

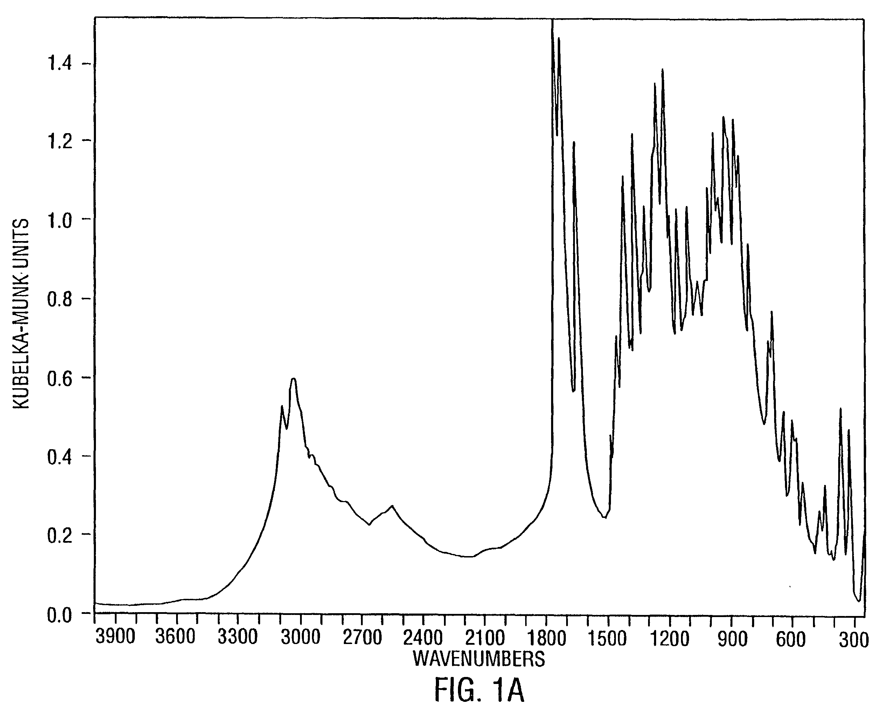

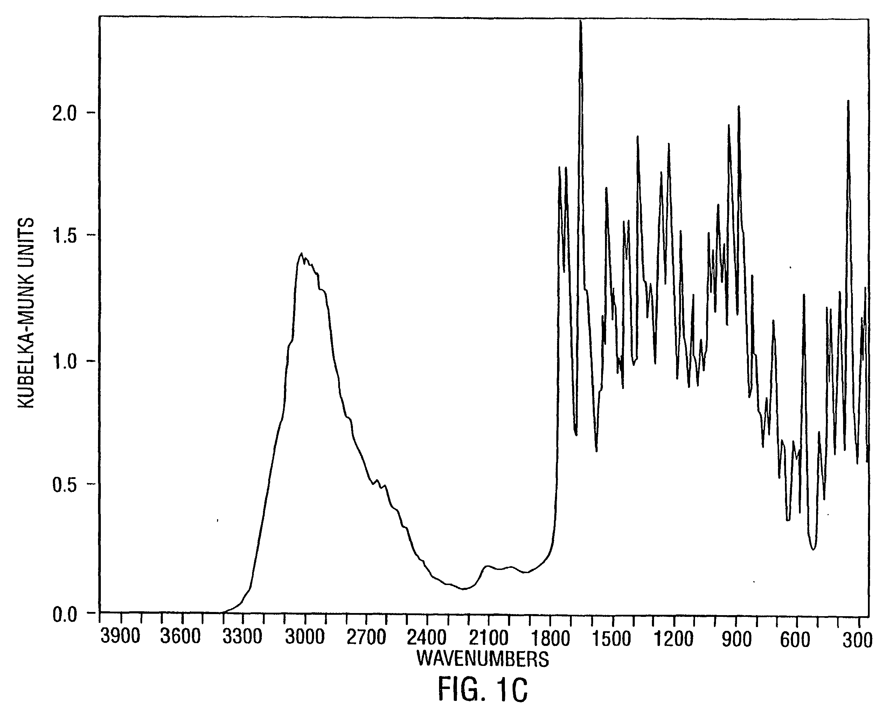

FIG. 1A is a control infrared spectrum of diethylenetriaminetetraacetate (DTPA) substrate (see Example 3).

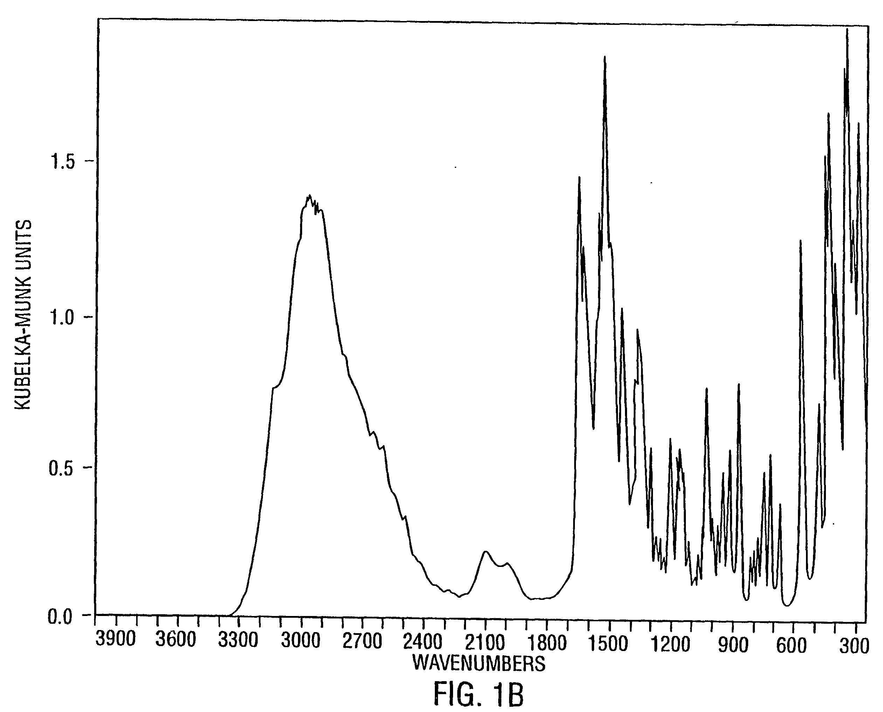

FIG. 1B is a control infrared spectrum of L-lysine.HCl substrate (see Example 3).

FIG. 1C is a control infrared spectrum of a physical mixture of these DTPA and L-lysine.HCl substrates without any chemical covalent linkage of the two substrates (see Example 3).

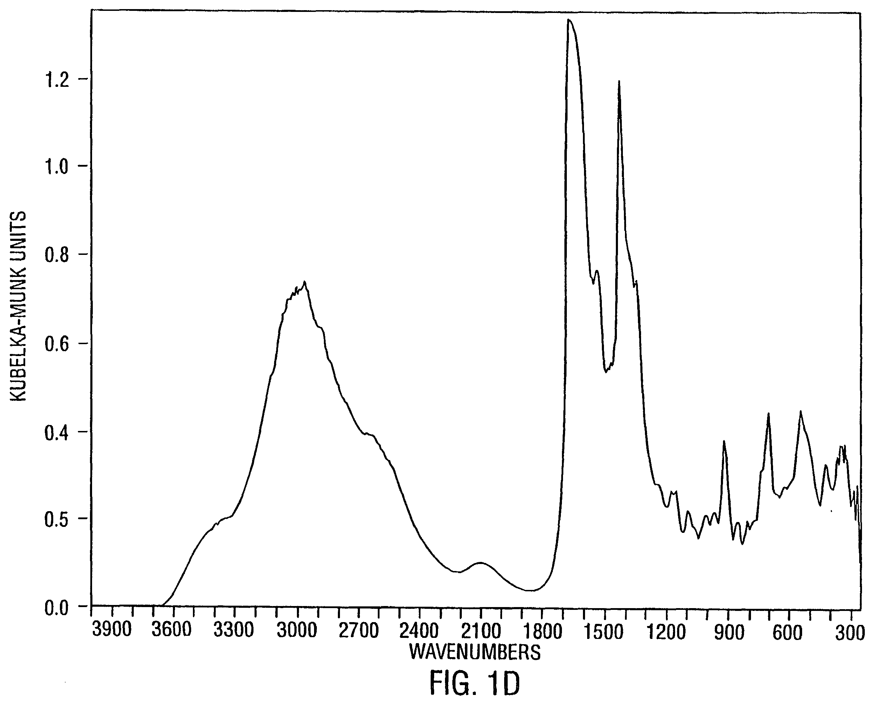

FIG. 1D is the experimental infrared spectrum of L-lysine covalently conjugated to DTPA by 1-ethyl-3-(3-dimethylaminopropyl) carbodiimide (EDC) linkage (see Example 3). Note the changes (height, width and loss of splitting) in signature peaks in the range of 1250-1700 wavenumbers, which indicate covalent conjugate formation.

For the following Figures (2-13), the dermatan sulfate carrier is of the new special class of dermatan sulfates with selectively oversulfated oligosaccharide sequences but without overall oversulfation (SO3-/Coo-ratio = 1:1 and sulfur content = 6.3 wt %; supplied by Opocrin S.P.A., Corlo Di Formigine, Italy, as "435 type").

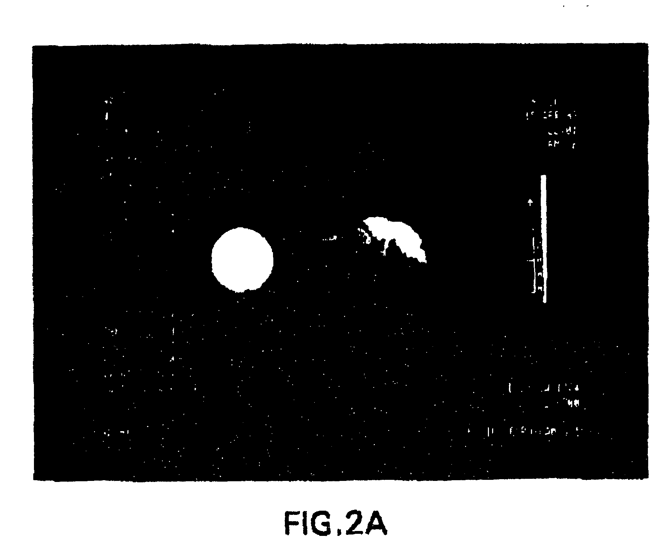

FIG. 2A, FIG. 2B, FIG. 3A, FIG. 3B, FIG. 4A, FIG. 4B, FIG. 4C, FIG. 4D and FIG. 8A, FIG. 8B and FIG. 8C show T1-weighted MRI images (TR/TE = 800/45, 550/23 and 600/45) performed at 1.0 and 1.5 Tesla, before (Pre) and after (Post) intravenous (i.v.) injection of Ferrioxamine:Dermatan Sulfate Selective Paramagnetic Contrast Agent, prepared as in Examples 2 and 5, and injected i.v. at a Ferrioxamine dose of 0.155 mmol/Kg into Fisher 344 female rats, with syngeneic breast adenocarcinoma inoculated previously into the liver, such that tumor diameters at the time of imaging are between 1.0 cm and 2.5 cm.

FIG. 2A. Precontrast image of liver (tumor not conspicuous).

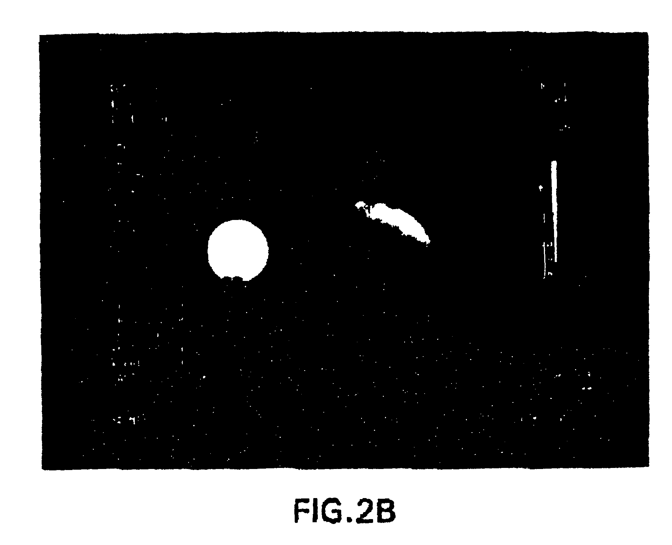

FIG. 2B. Liver image at 7 min postinjection (MPI) of the Selective Paramagnetic Contrast Agent, Ferrioxamine:Dermatan Sulfate (0.155 mmol/Kg) i.v., showing marked contrast enhancement of tumor in right lobe of liver, very sharp tumor boundaries against surrounding liver, and discretely demarcated darker central region of tumor necrosis -- allowing tumor perfusion and function to be spatially resolved and assessed within different, very small anatomical subregions.

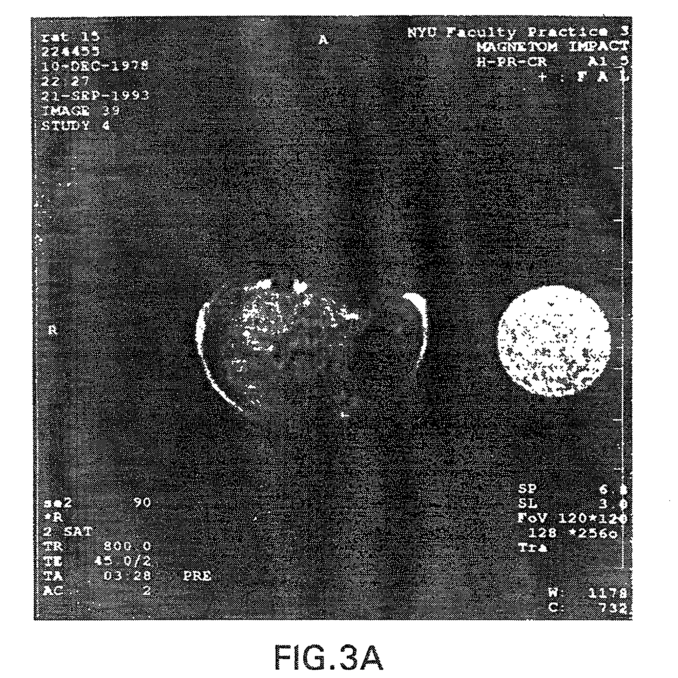

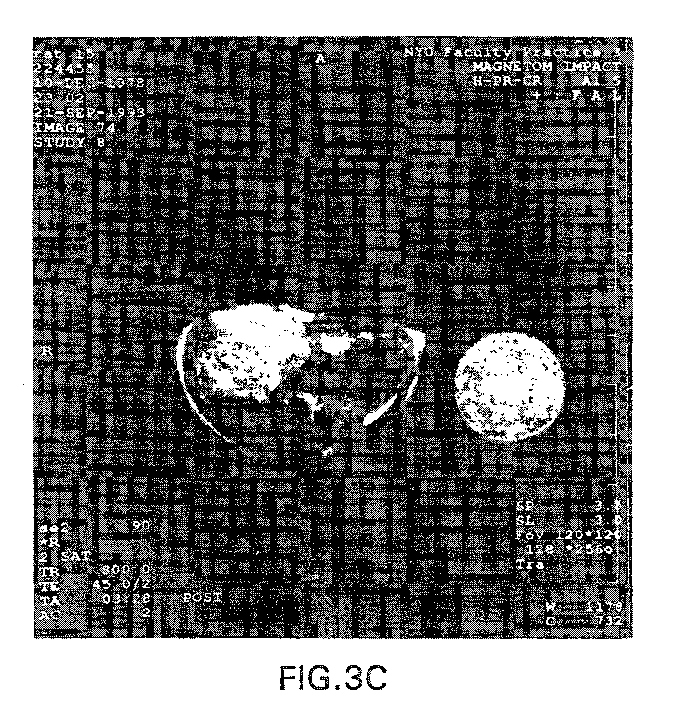

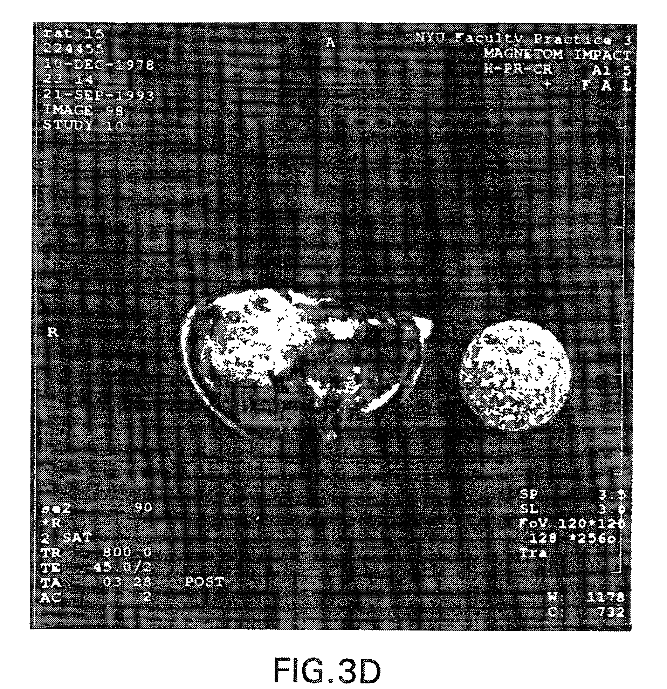

FIG. 3A. Precontrast image of liver (tumor is present but not conspicuous).

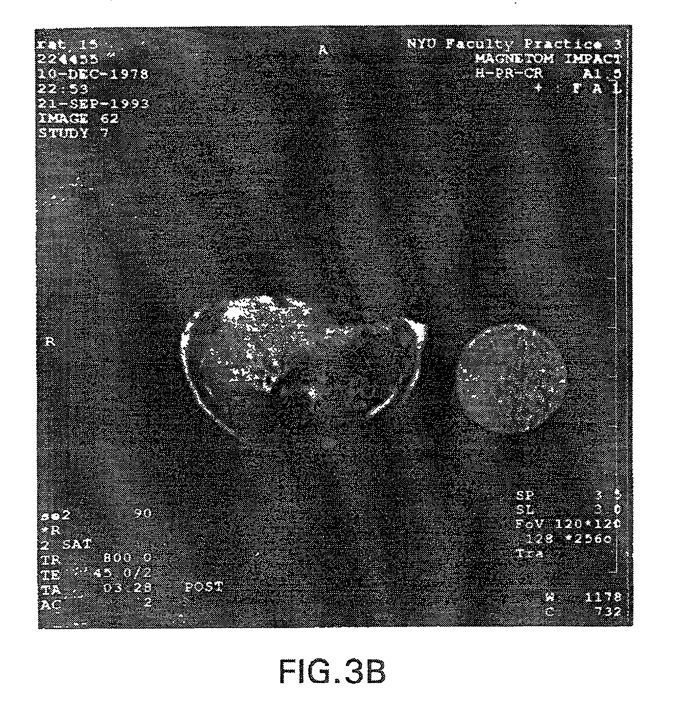

FIG. 3B. Liver image at 7 MPI of Ferrioxamine Active Alone (without any Dermatan Sulfate Carrier). Note that acute contrast enhancement is only very slight or nonexistent. This differs markedly from the pronounced tumor enhancement seen in FIG. 2B; and it indicates that binding of the Ferrioxamine active by the Dermatan Sulfate carrier is a requirement for tumor-site localization and tumor uptake of Ferrioxamine active.

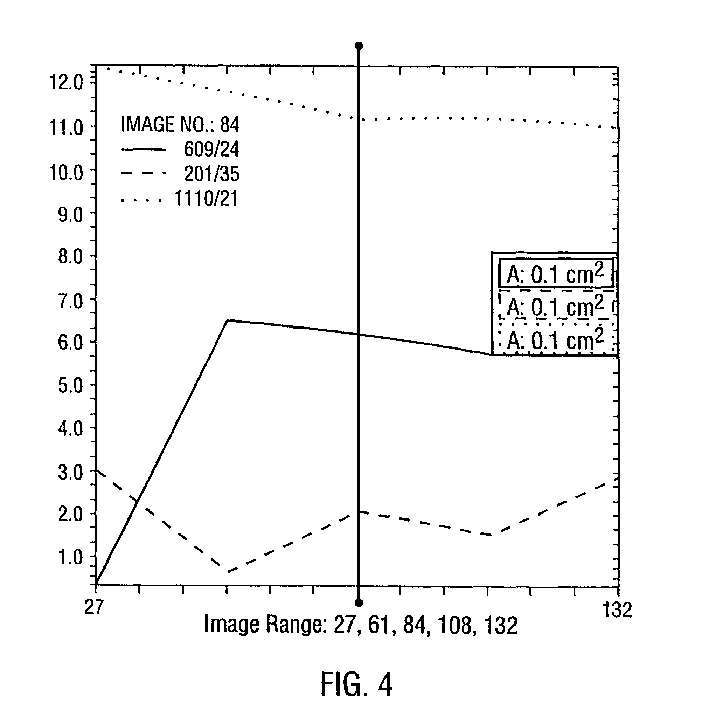

FIG. 4A. Precontrast T1 image (TR/TE = 800/45) of liver (breast tumor is present but not conspicuous).

FIG. 4B. Liver image at 21 MPI of Ferrioxamine:Dermatan Sulfate Selective MRI Contrast Agent. Note the marked enhancement of main tumor mass and distinct tumor borders. Also note the small, 2-mm, bright enhancement of tumor metastasis in left lobe of liver. This metastasis is completely non-visualized in the Precontrast T1 images.

FIG. 4C. Liver image at 30 MPI of Ferrioxamine:Dermatan Sulfate Selective MRI Contrast Agent. Note the sustained enhancement of main tumor and metastasis.

FIG. 4D. Liver image at 42 MPI of Ferrioxamine:Dermatan Sulfate Selective MRI Contrast Agent. Note: continued strong enhancement of main tumor and metastasis at prolonged post-contrast interval, at high, sustained sensitivity, and with continued delineation of tumor boundaries in both nodules (selectivity), plus delineation of the very small non-perfused region centrally within the 2-mm liver metastasis.

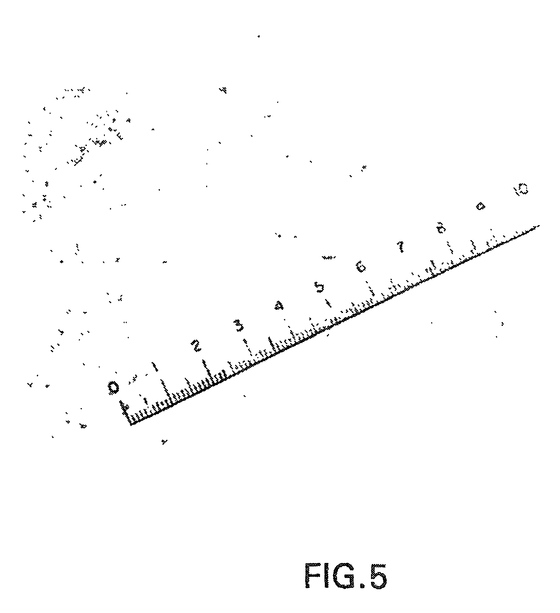

FIG. 5. Region-of-interest (ROI) analyses of MRI image intensities from a tumor animal analogous to that shown in FIG. 4A, FIG. 4B, FIG. 4C and FIG. 4D. Upper line = tumor ROI's; Lower line = liver ROI's; time points = Precontrast; and 12, 27, 44 and 64 MPI of Ferrioxamine:Dermatan Sulfate Selective MRI Contrast Agent. Note the Intensity Ratios of Tumor to Liver are: (a) at the Peak time of 12 MPI = 11:1; (b) as an average over the 27-64 MPI = 3.2:1 -- both (a) and (b) additionally indicating very good selectivity for tumor versus liver. Intensity fades only very gradually with time, unlike the kinetics reported for Gd:DTPA, which are very rapid and have a t1/2 at the site of ca. 12-20 min (images not shown).

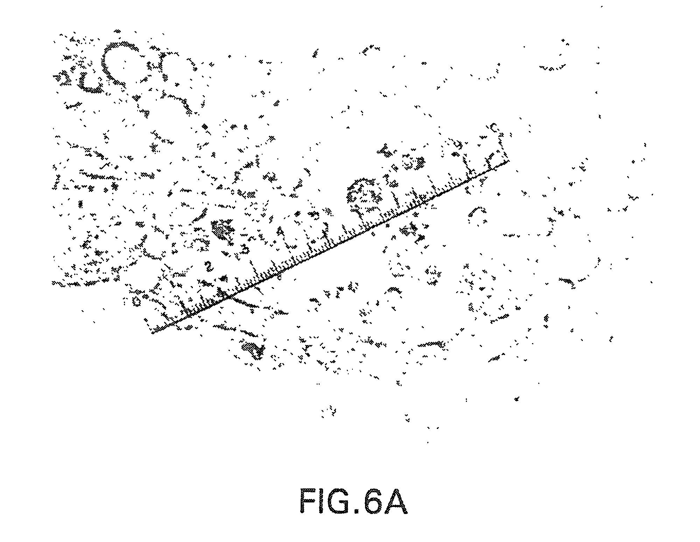

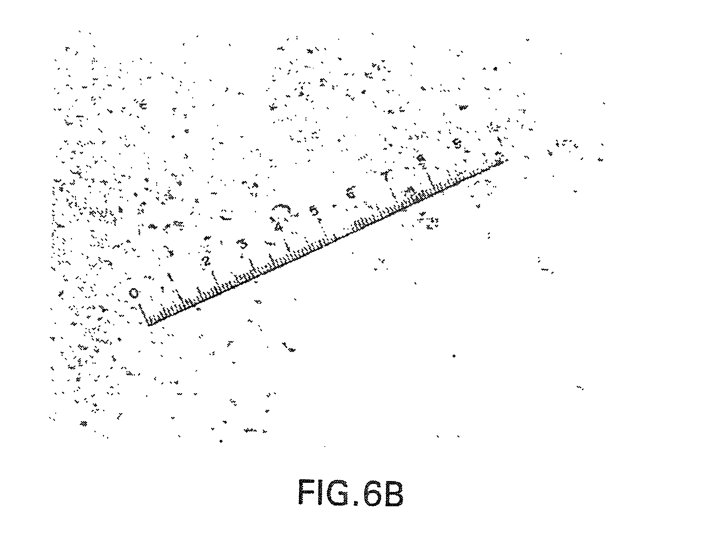

FIG. 6. Special histologic stain (heated ferroferricyanide reaction) of formalin-fixed section of sygeneic breast adenocarcinoma excised from liver inoculation site of Fisher 344 female rats: Outer Tumor Rim 7-10 MPI of Ferrioxamine:Dermatan Sulfate Selective MRI Contrast Agent. Note selective staining for ferrioxamine iron (a) strongly positive on and within tumor endothelium, (b) strongly positive in the subendothelia, (c) moderately positive in the extracellular matrix of tumor, and (d) lightly to moderately positive within tumor intracellular sites.

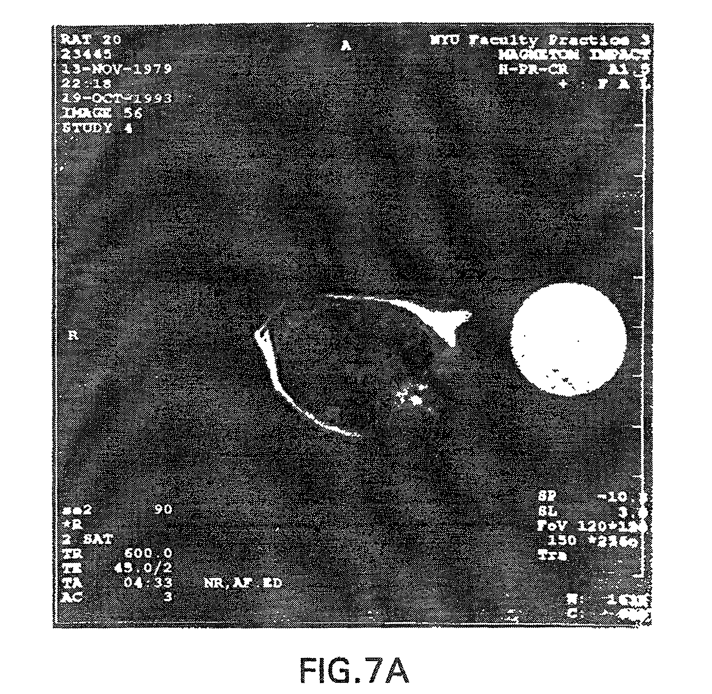

FIG. 7A. Same tumor, stain, conditions, and post-contrast time as FIG. 6, except tissue section is taken from Central Tumor, 7-10 MPI of Ferrioxamine:Dermatan Sulfate Selective MRI Contrast Agent. Significant staining positivity is present at all sites as in FIG. 6.

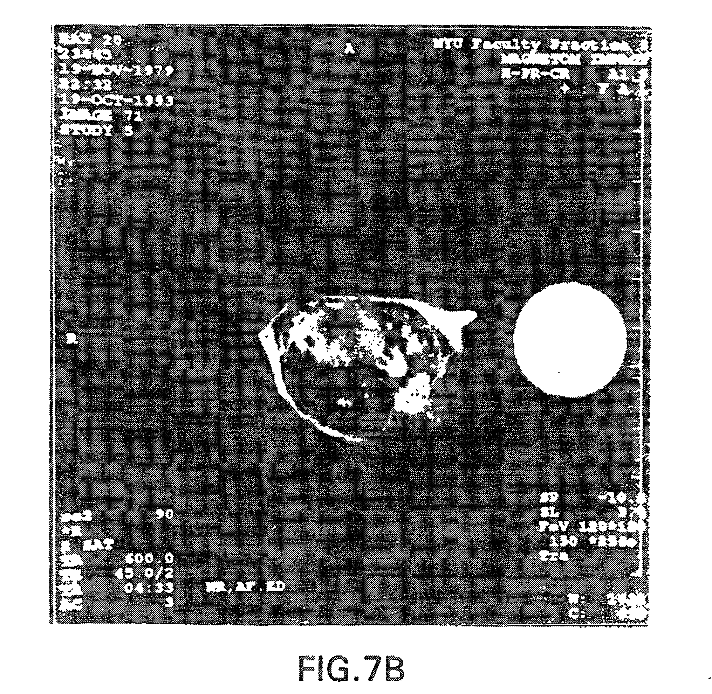

FIG. 7B. Identical to FIG. 7A, except a different animal with identical type and site of breast tumor, 7-10 MPI after i.v. Ferrioxamine Active Alone at a Ferrioxamine dose identical to FIG. 6 and FIG. 7A. Note the complete absence of staining positivity. This correlates directly with the results of MRI imaging with the full Agent (Active bound to Carrier) versus that with Active Alone (Active in free form) -- (refer to FIG. 2A and FIG. 2B versus 3).

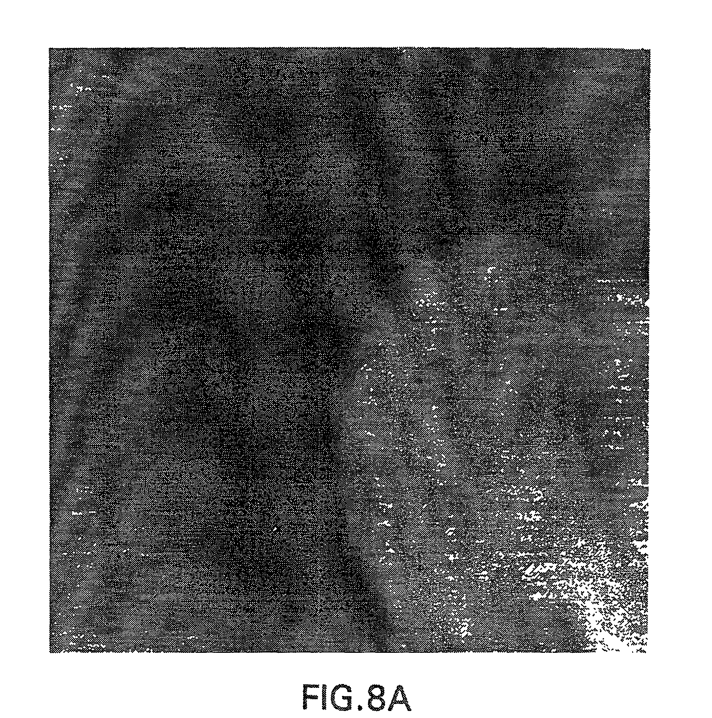

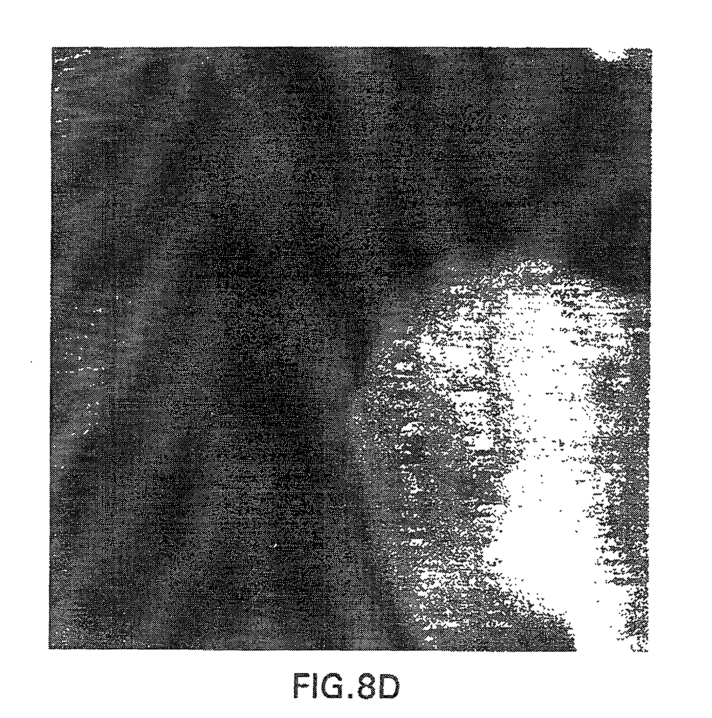

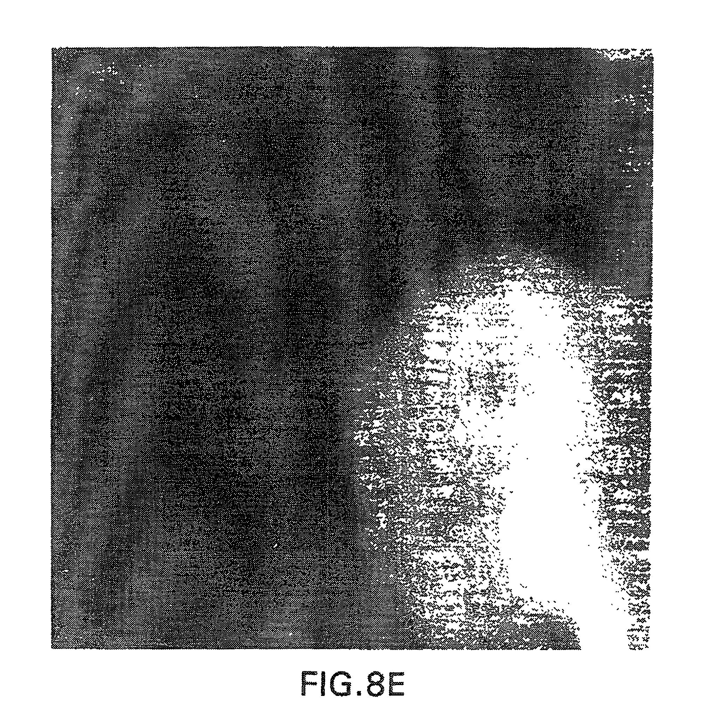

FIG. 8A. T1-weighted (TR/TE = 600/45) image of Lung Field in rat with primary liver breast tumor. Note that the lung metastases (2-mm to 3-mm nodules) are only faintly conspicuous Precontrast.

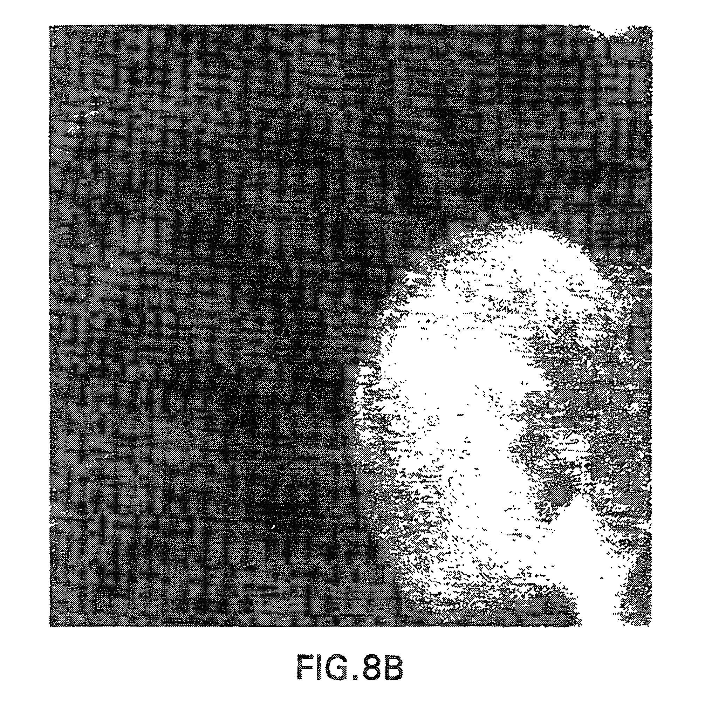

FIG. 8B. Lung Field of same rat at 12 MPI. Note the marked improvement in sensitivity of tumor detection (conspicuity) due to selective, bright enhancement of the lung metastases. Also note the sharpness of tumor boundaries.

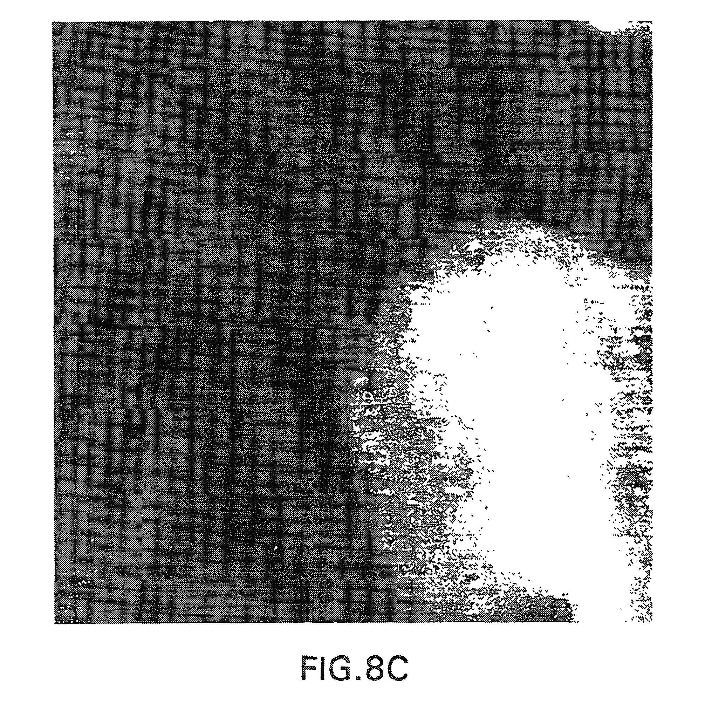

FIG. 8C. Same Lung Field at 17 MPI -- showing sustained enhancement and sustained sharpness of tumor boundaries. By comparison, the rapid diffusion rates of Gd:DTPA lead to rapidly fuzzy boundaries at early times; and thereby also decrease the sensitivity of detecting pulmonary metastases.

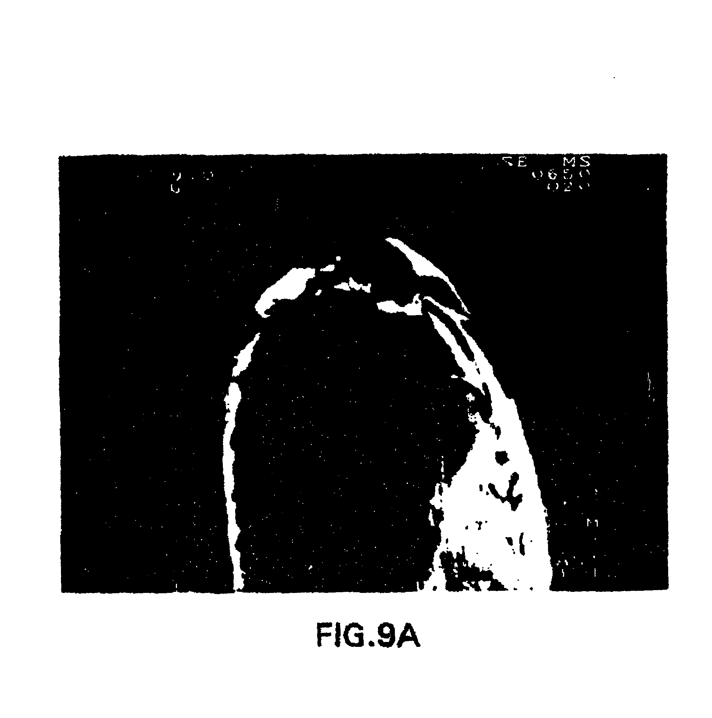

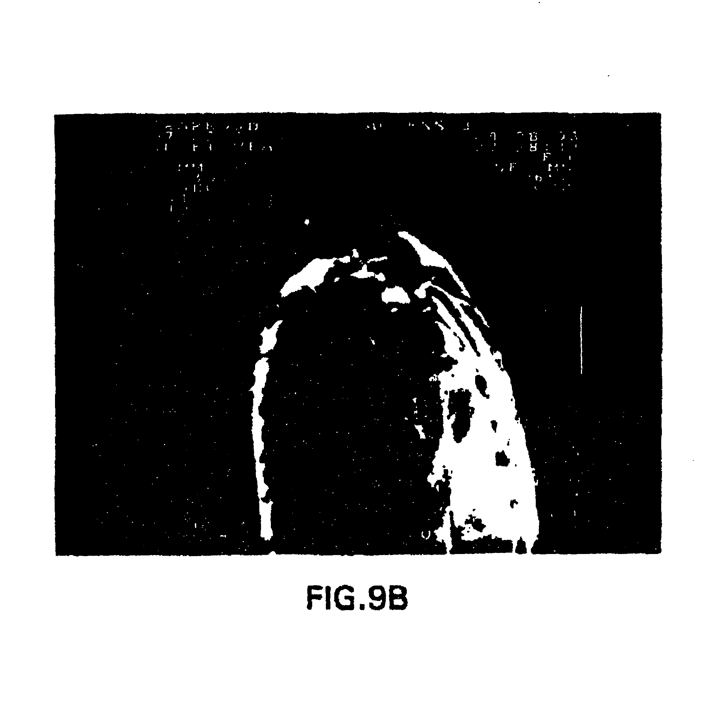

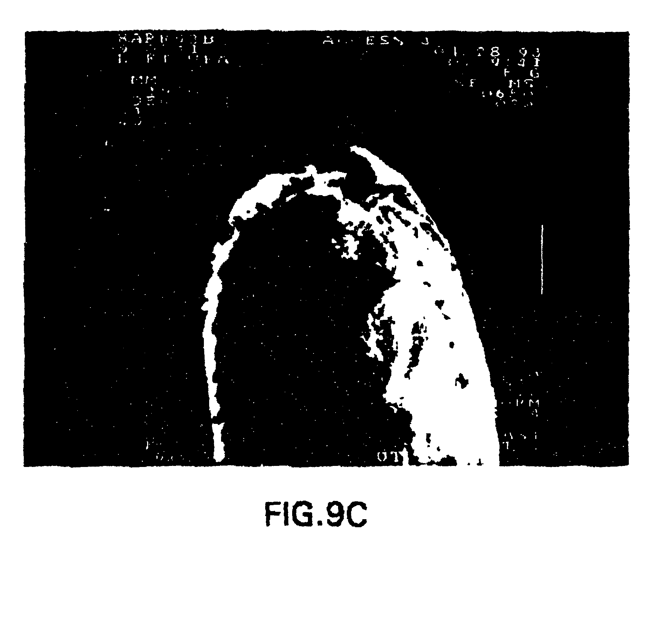

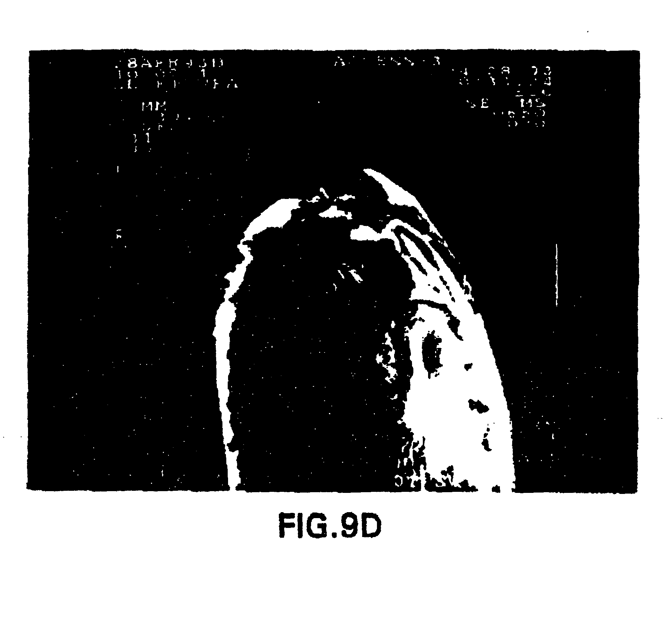

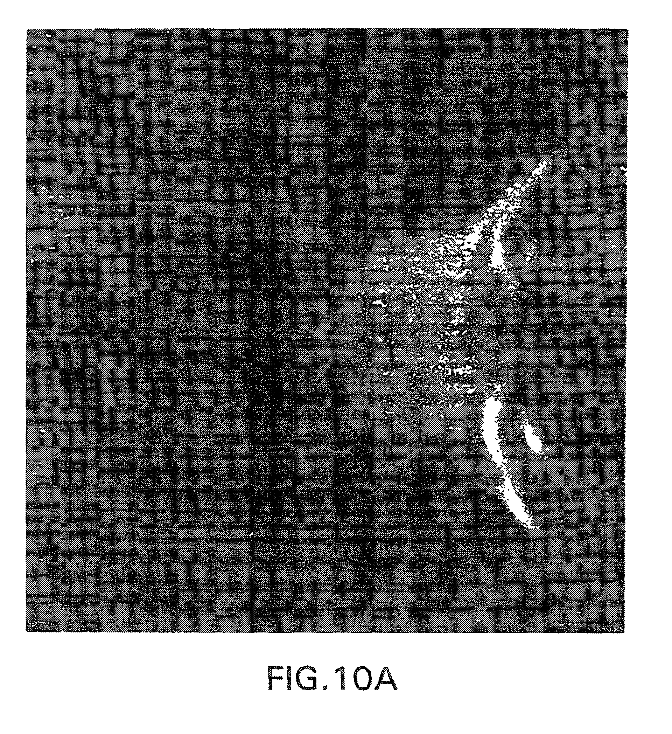

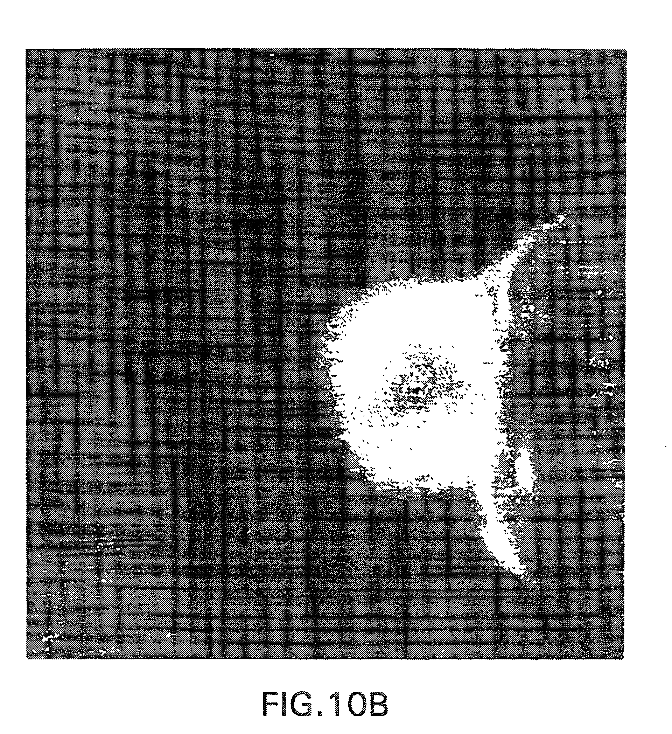

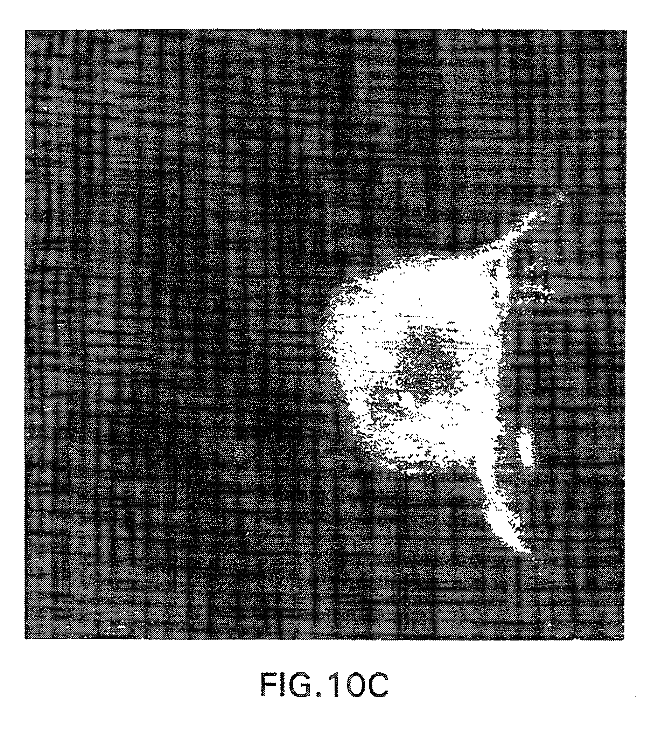

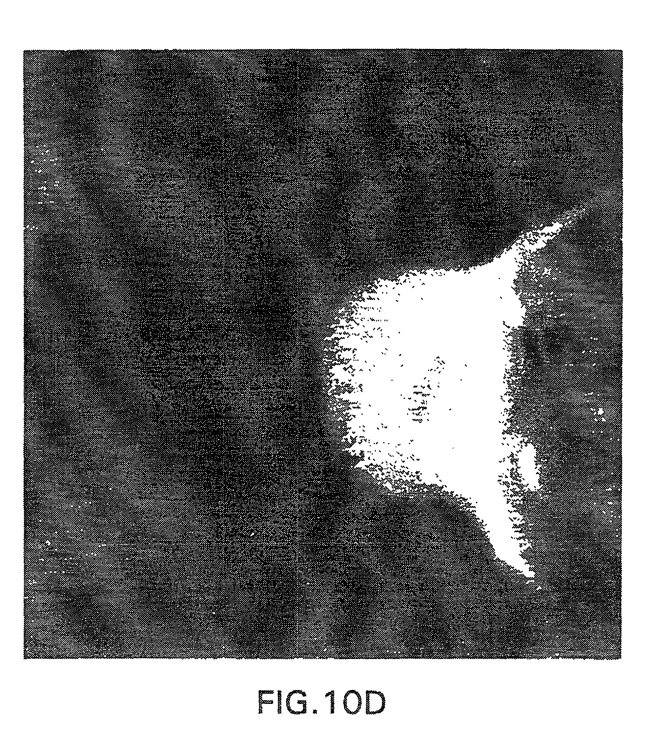

FIG. 9A, FIG. 9B, FIG. 9C, FIG. 9D, FIG. 9E, FIG. 10A, FIG. 10B, FIG. 10C, FIG. 10D and FIG. 10E show T1-weighted MRI images (TR/TE = 250/8) performed at 4.7 Tesla, before (Pre) and after (Post) intravenous (i.v.) injection of Ferrioxamine:Dermatan Sulfate Selective Paramagnetic Contrast Agent (FIG. 9A, FIG. 9B, FIG. 9C, FIG. 9D, FIG. 9E) prepared as in Examples 2 and 5, and injected i.v. at an Iron(III) dose of 0.155 mmol/Kg; compared to Gadolinium DTPA dimeglumine (FIG. 10A, FIG. 10B, FIG. 10C, FIG. 10D, FIG. 10E), injected i.v. at a Gd(III) dose of 0.100 mmol/Kg; each of these agents being administered to Copenhagen rats with syngeneic AT-1 prostate adenocarcinoma inoculated into previously prepared skin pouches [Hahn *et al.* (1993)], such that tumor diameters at the time of imaging are between 1.0 cm and 2.5 cm.

FIG. 9A. Precontrast image for Ferrioxamine:Dermatan Sulfate Selective Contrast Agent.

FIG. 9B. 7 MPI of Ferrioxamine:Dermatan Sulfate, liquid form at a ferrioxamine concentration of 0.166 mmol/mL. Note the strong enhancement of Outer Rim and Vascular array which fans out from the tumor pedicle.

FIG. 9C. Same as FIG. 9B, except 20 MPI. Note the sustained, discrete enhancement of elements in FIG. 9B.

FIG. 9D. Same as FIG. 9C, except 40 MPI. Note the sustained contrast and delineation of Outer Rim.

FIG. 9E. Same as FIG. 9D, except 60 MPI. Note the onset of contrast fading.

FIG. 10A. Precontrast image for Gd:DTPA dimeglumine Nonselective Contrast Agent.

FIG. 10B. 7 MPI of Gd:DTPA dimeglumine. Note that the Outer Rim is not well delineated, even at this very early post-contrast interval.

FIG. 10C. Same as FIG. 10B, except 20 MPI. Note the marked early contrast fading overall, with some agent sequestration seen at the central, poorly perfused (cystic) regions of tumor (as is typically reported for Gd:DTPA when used for imaging at body sites).

FIG. 10D. Same as FIG. 10C, except 40 MPI. Note that enhancement is nearly reverted to background levels.

FIG. 10E. Same as FIG. 10D, except 60 MPI. No residual contrast, except for central cystic regions.

FIG. 11A, FIG. 11B, FIG. 11C and FIG. 11D show T1-weighted MRI ECG-gated cardiovascular images performed at 0.5 Tesla, before (Pre) and after (Post) rapid intravenous (i.v.) infusion of Ferrioxamine:Dermatan Sulfate Selective Paramagnetic Contrast Agent prepared as in Examples 2 and 5, and injected i.v. at an Iron(III) dose of 0.155 mmol/Kg into German Shepherd dogs with acute, 90-min myocardial infarcts (ligature of proximal left anterior descending coronary artery) followed by reperfusion for ca. 90 minutes prior to contrast agent infusion.

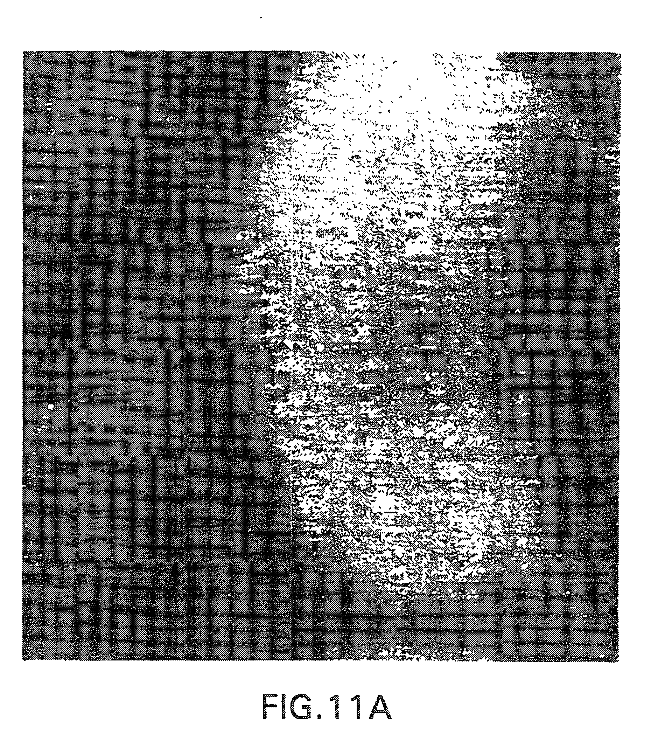

FIG. 11A. Precontrast image.

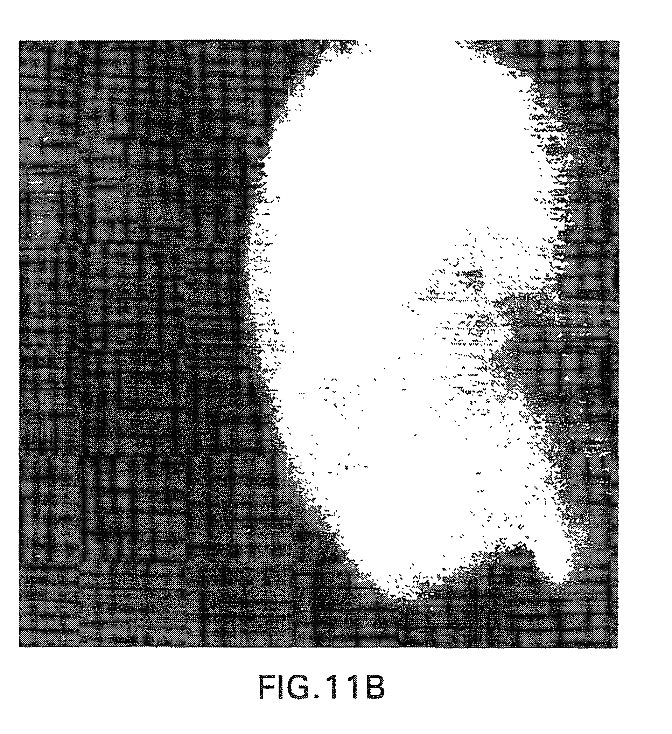

FIG. 11B. 7 MPI, showing strong enhancement of infarct by Ferrioxamine:Dermatan Sulfate Agent, and in particular delineating the boundary of the infarct -- putatively the boundary of the marginal zone. Note the central darker region -- putatively the irreversible central infarct zone.

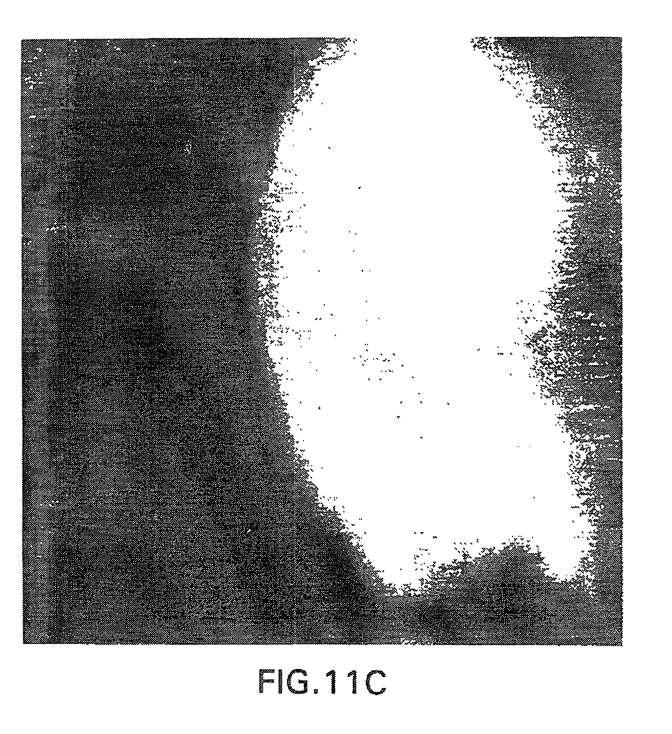

FIG. 11C. 20 MPI, showing sustained strong enhancement and zones as above.

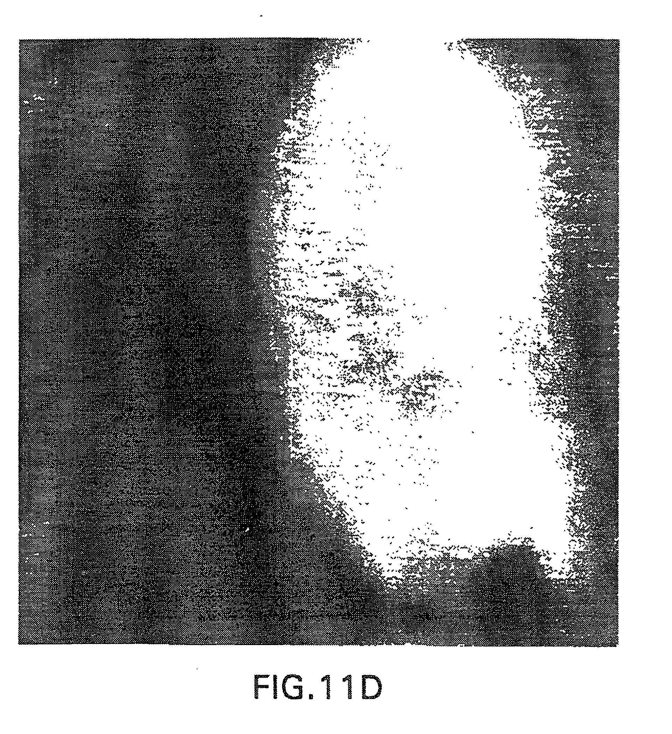

FIG. 11D. 40 MPI, same as 11C, except filling in of central zone; absence of significant overall contrast fading. NOTES: (1) injection of Ferrioxamine Agent Alone at 0.155 mmol/Kg, gives no detectable enhancement (images not shown); (2) infarct sizes and positions are documented by double dye infusion methods immediately after imaging.

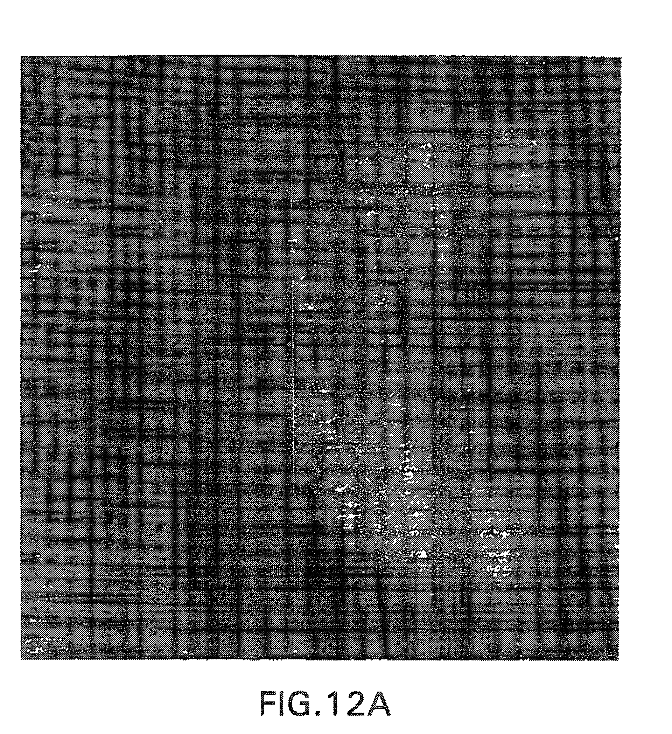

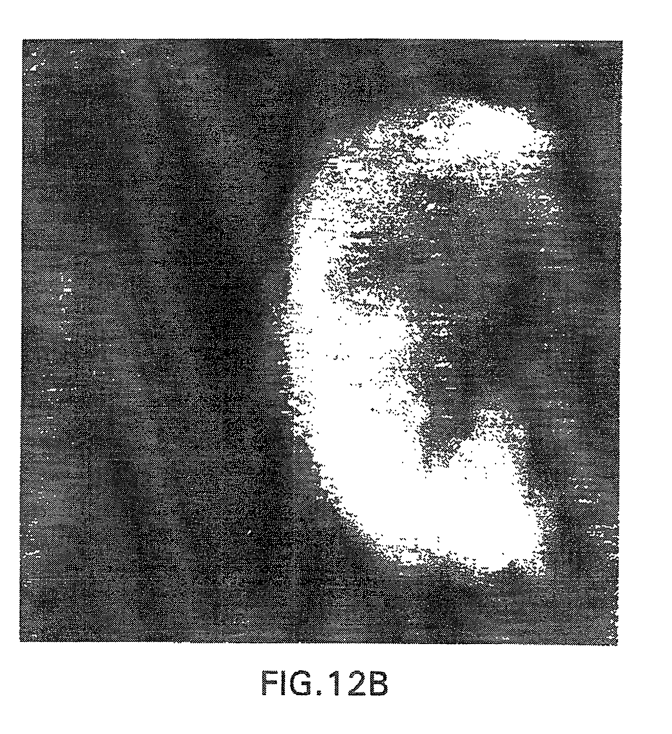

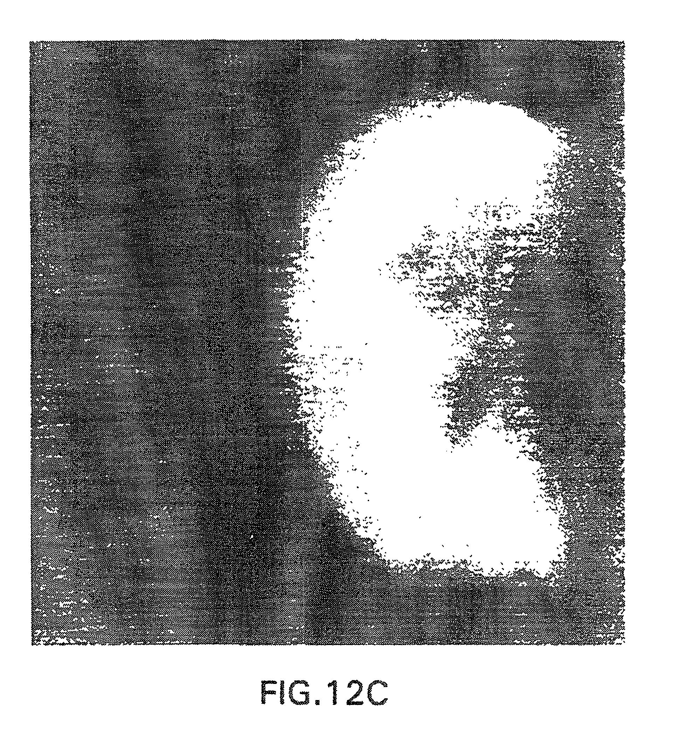

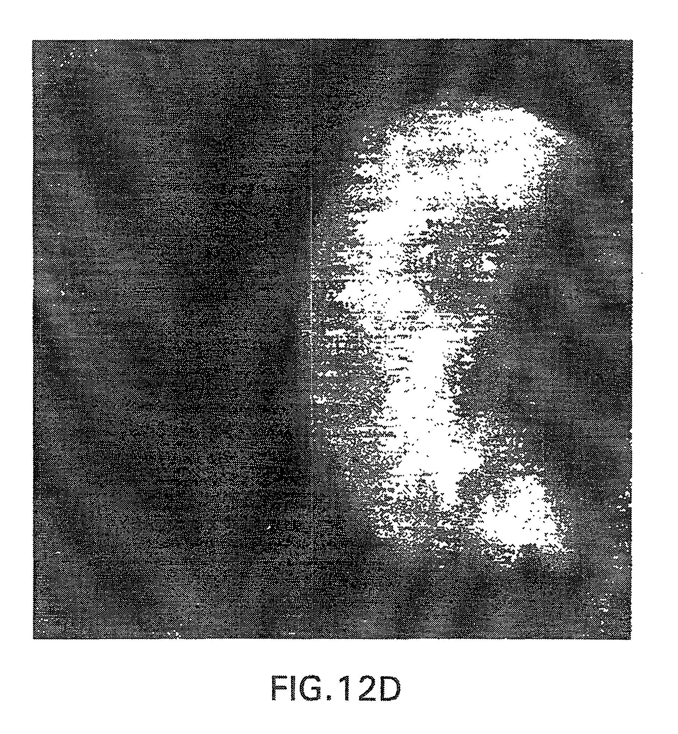

FIG. 12A, FIG. 12B, FIG. 12C and FIG. 12D show MRI 4.7 Tesla, T1-weighted images of Copenhagen rats with the AT-1 prostate tumor model (as in FIG. 9A, FIG. 9B, FIG. 9C, FIG. 9D, FIG. 9E, FIG. 10A, FIG. 10B, FIG. 10C, FIG. 10D, and FIG 10E), but rats are injected i.v. with Ferrioxamine:Dermatan Sulfate Selective Contrast Agent in the lyophilized (versus liquid) form, and the Agent is reconstituted with water just prior to administration at a higher concentration of 0.415 mmol/mL Fe(III) and administered at the usual dose of 0.155 mmol of Fe(III) per Kg.

FIG. 12A. Precontrast image for Ferrioxamine:Dermatan Sulfate Selective Contrast Agent.

FIG. 12B. 7 MPI of Ferrioxamine:Dermatan Sulfate, lyophilized reconstituted to a Fe(III) concentration of 0.415 mmol/mL. Note the very strong enhancement of the entire Outer Rim of tumor.

FIG. 12C. Same as FIG. 12B, except 20 MPI. Note the sustained, very strong enhancement and delineation of Outer Rim.

FIG. 12D. Same as FIG. 12C, except 40 MPI. Note the sustained very strong enhancement of Outer Rim with the Central Tumor now also starting to enhance brightly. Also note there is virtually no contrast fading at 40 minutes.

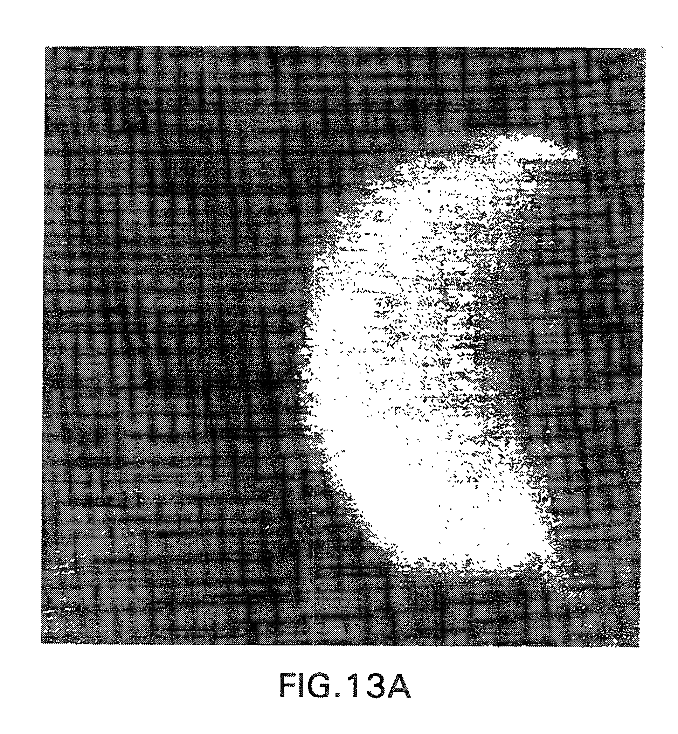

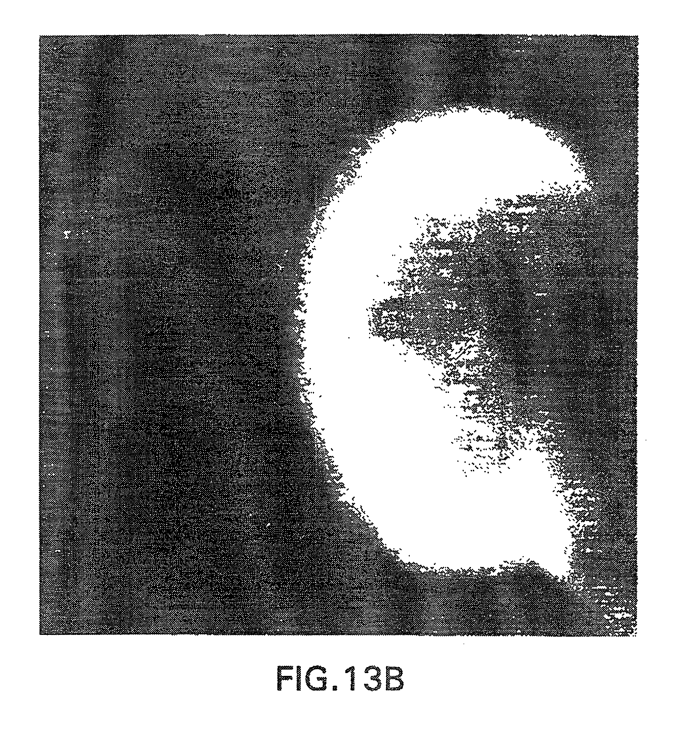

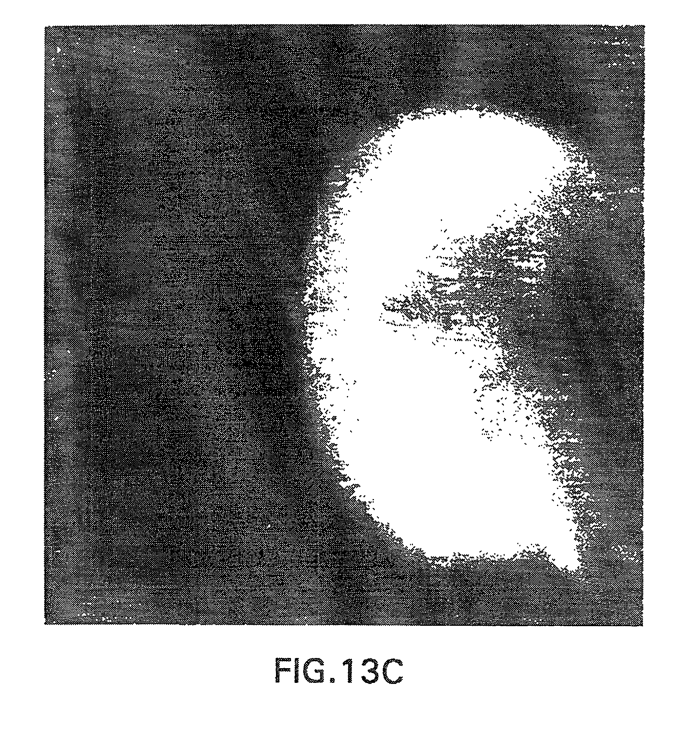

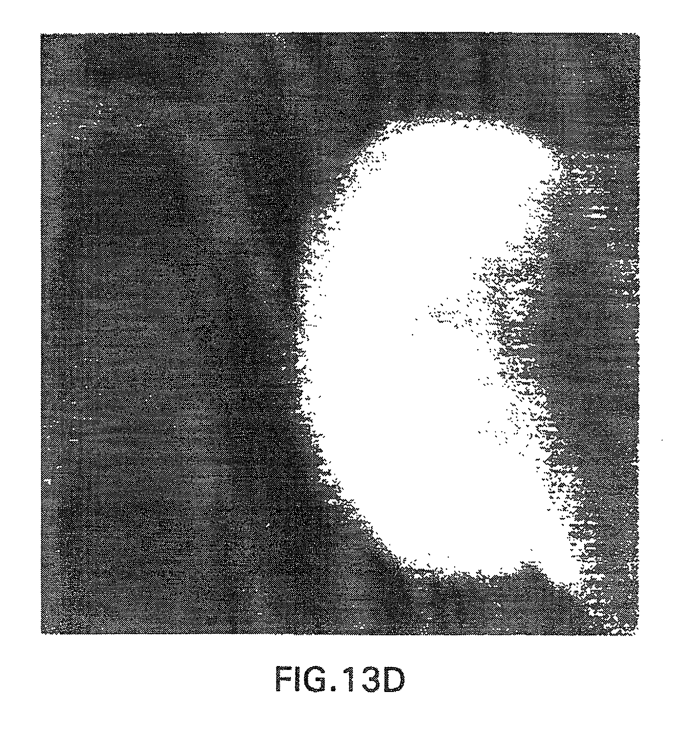

FIG. 13A, FIG. 13B, FIG. 13C, and FIG. 13D show MRI 4.7 Tesla, T1-weighted images of Copenhagen rats with the AT-1 prostate tumor model (as in FIG. 12A, FIG. 12B, FIG. 12C, and FIG. 12D), but rats are injected i.v. with Gd(III):DTPA-Lys:Dermatan Sulfate Selective Contrast Agent in liquid form pre-concentrated to 0.415 mmol/mL Gd(III) and administered at the usual dose of 0.155 mmol of Gd(III) per Kg.

FIG. 13A. Precontrast image for Gd(III) :DTPA-Lys:Dermatan Sulfate Selective Contrast Agent.

FIG. 13B. 7 MPI of Gd(III):DTPA-Lys:Dermatan Sulfate, at 0.415 mmol/mL. Note the exceedingly strong enhancement of the entire Outer Rim as well as Central Tumor. This is consistent with the higher paramagnetic potency of Gd:DTPA chelate, R1 = 4.3 [mmol.sec]-1, relative to ferrioxamine chelate, R1 = 1.5-1.8 [mmol.sec]-1.

FIG. 13C. Same as FIG. 13B, except 20 MPI. Note the sustained, very strong absolute enhancement Outer Rim. Also note additionally strong enhancement of the central vascular array (as differentiated from cystic sequestration).

FIG. 13D. Same as FIG. 13C, except 40 MPI. Note sustained enhancement of Outer Rim, with overall enhancement just beginning to fade at 40 minutes, but absolute enhancement remaining as bright or brighter in all regions relative to Ferrioxamine:Dermatan Sulfate.

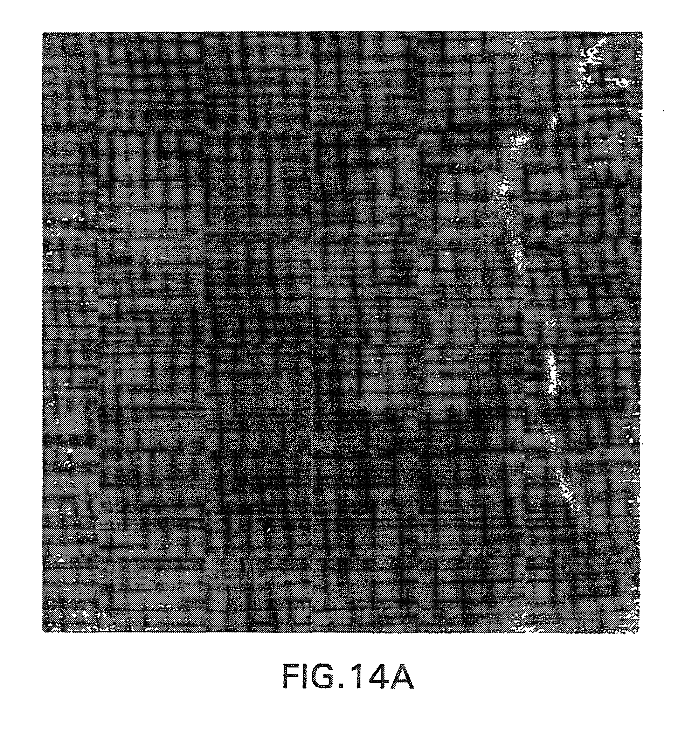

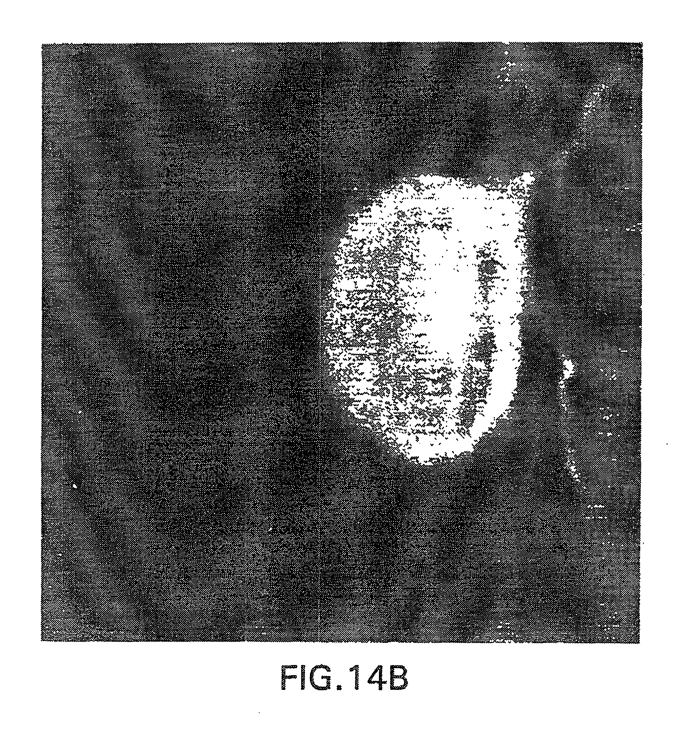

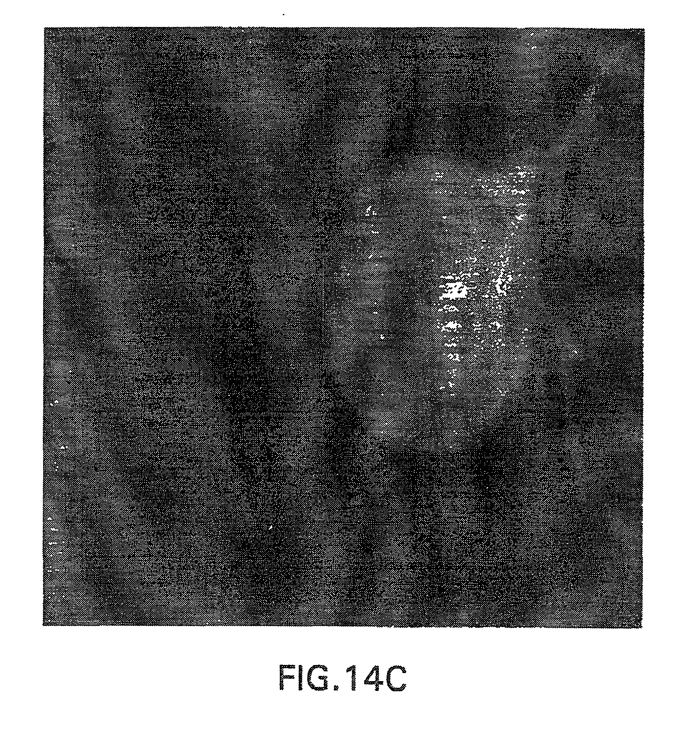

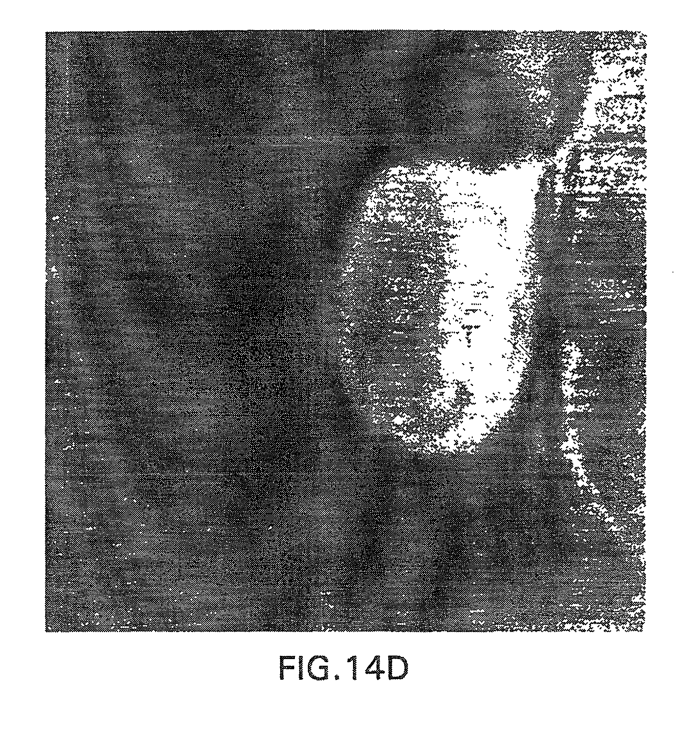

FIG. 14A, FIG. 14B, FIG. 14C and FIG. 14D show MRI 4.7 Tesla, T1-weighted images of Copenhagen rats with the AT-1 prostate tumor model (as in FIG. 13A, FIG. 13B, FIG. 13C, FIG. 13D), but rats are injected i.v. with Ferrioxamine Selective Contrast Agent, wherein the Active is non-covalently bound to Oversulfated Dermatan Sulfate, the Agent lyophilized and reconstituted with water just prior to administration at a concentration of 0.332 mmol/mL Fe(III) and administered at the usual dose of 0.155 mmol of Fe(III) per Kg.

FIG. 14A. Precontrast.

FIG. 14B. 7 MPI.

FIG. 14C. 20 MPI.

FIG. 14D. 40 MPI. Note the equivalent to slightly greater enhancement of Tumor Rim and greater definition of the vascular array at all times, in relation to Ferrioxamine bound to Native Dermatan Sulfate (above)

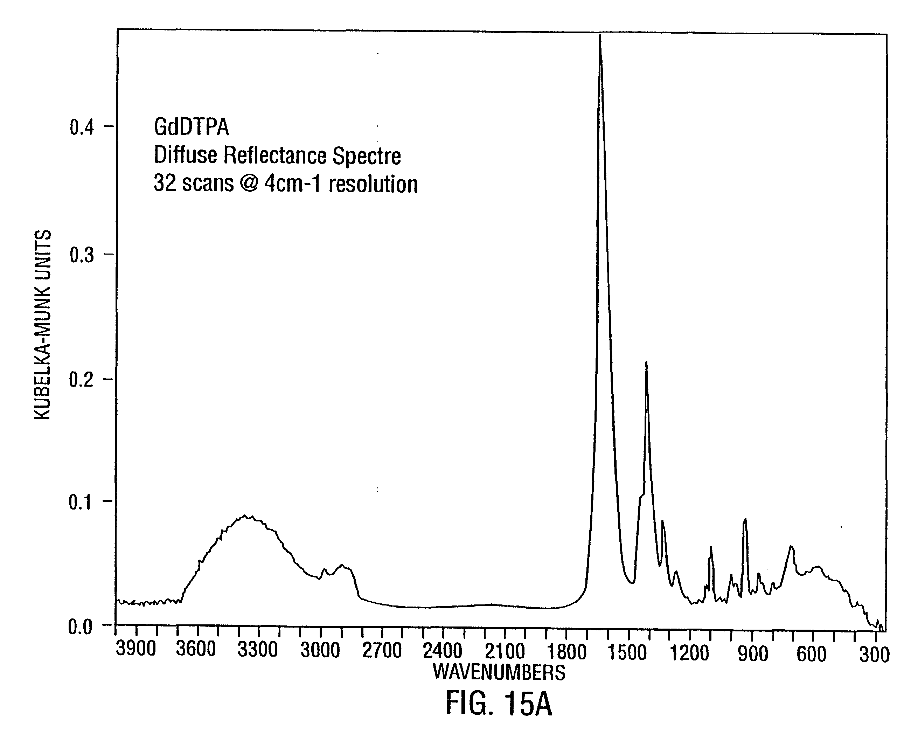

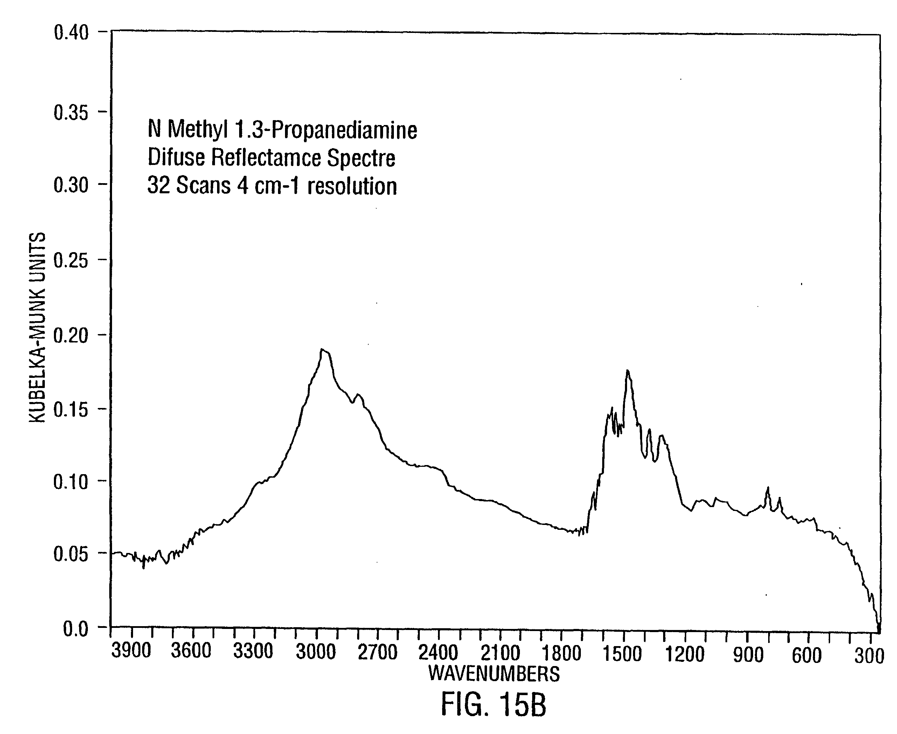

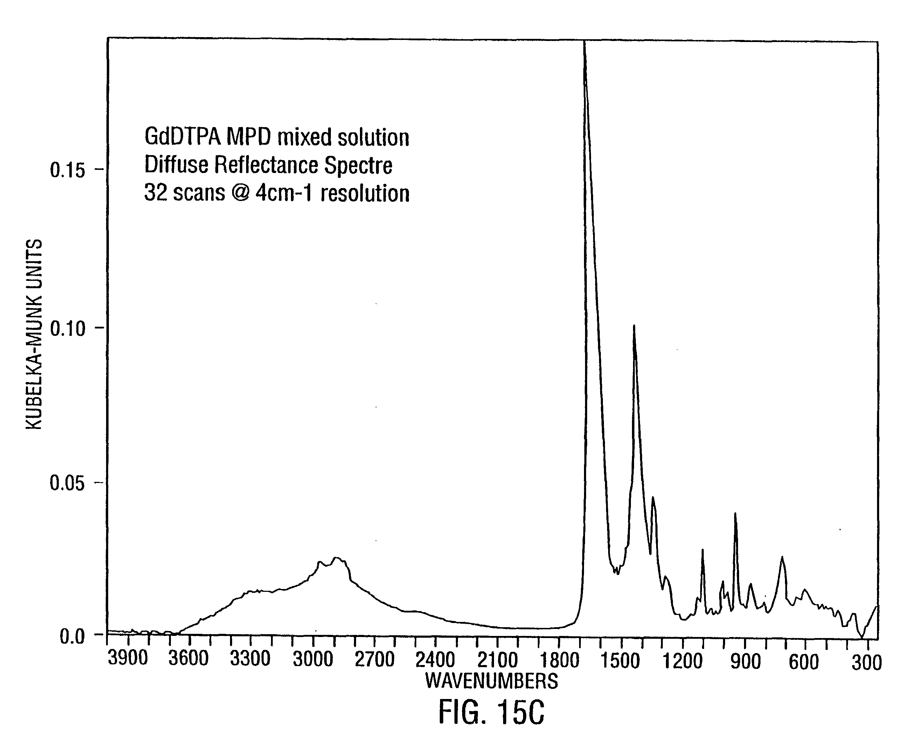

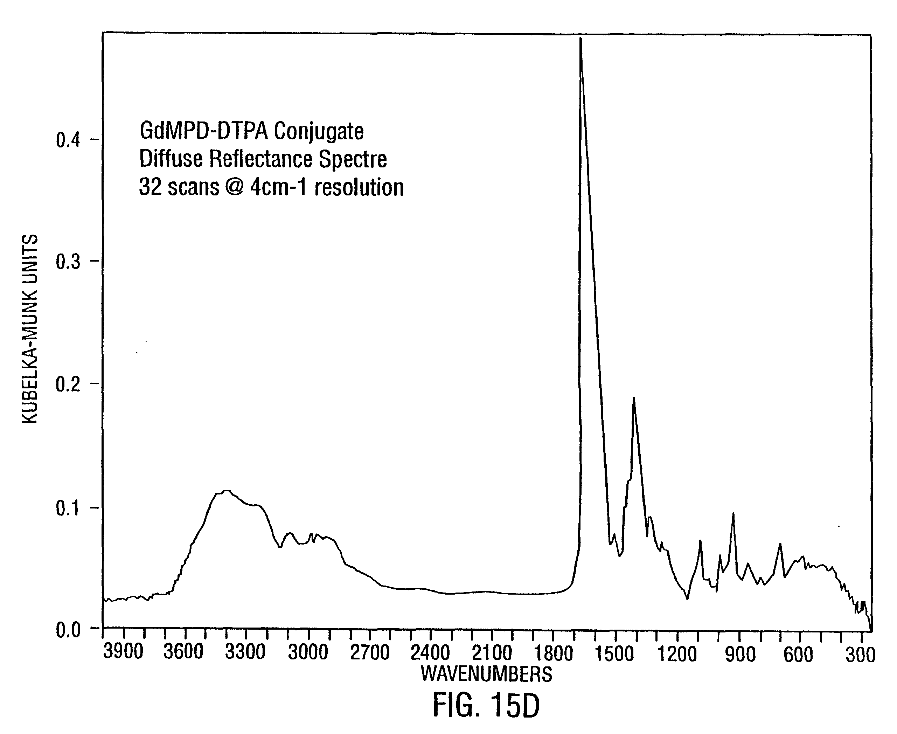

FIG. 15A, FIG. 15B, FIG. 15C, and FIG. 15D show MRI 4.7 Tesla, T1-weighted images of Copenhagen rats with the AT-1 prostate tumor model (as in FIG. 13A, FIG. 13B, FIG. 13C and FIG. 13D), but rats are injected i.v. with Ferrioxamine Selective Contrast Agent, wherein the Active is non-covalently bound to Oversulfated Chondroitin Sulfate, the Agent lyophilized and reconstituted with water just prior to administration at a concentration of 0.332 mmol/mL Fe(III) and administered at the usual dose of 0.155 mmol of Fe(III) per Kg.

FIG. 15A. Precontrast.

FIG. 15B. 7 MPI.

FIG. 15C. 20 MPI.

FIG. 15D. 40 MPI. Note the moderately greater enhancement of Tumor Rim and greater definition of the vascular array at 7 MPI, and the only slightly greater enhancement at the two later times, in relation Ferrioxamine bound to Native Dermatan Sulfate (above).

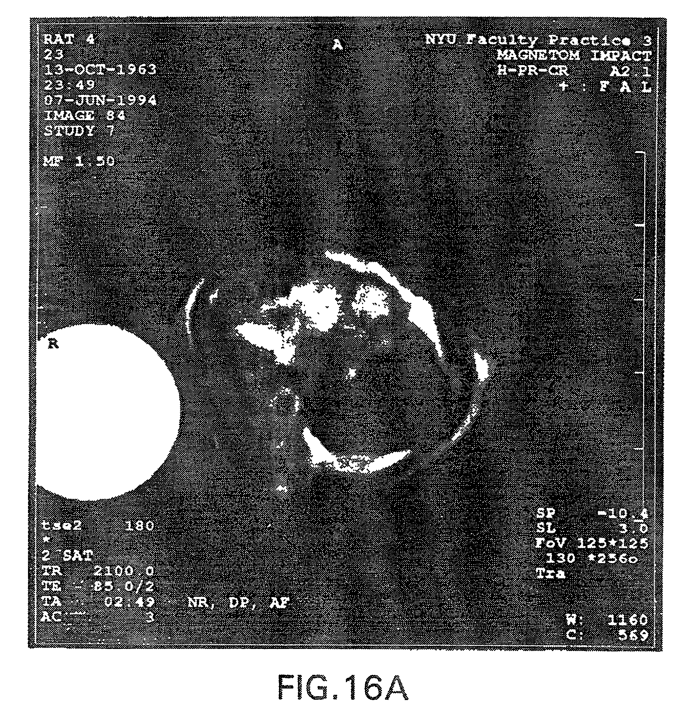

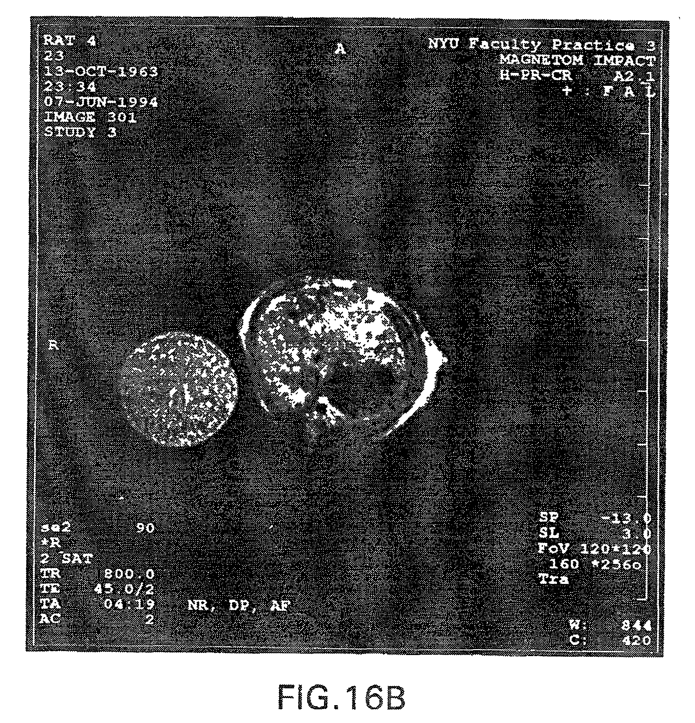

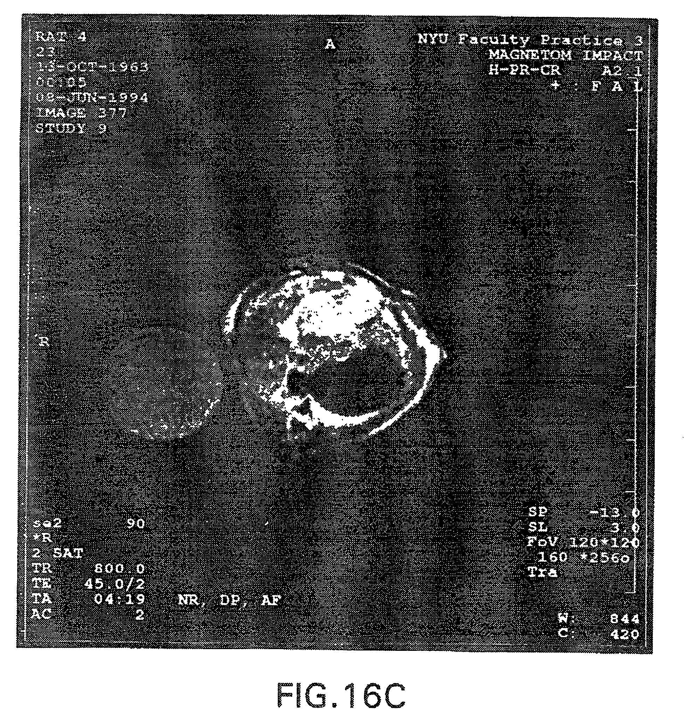

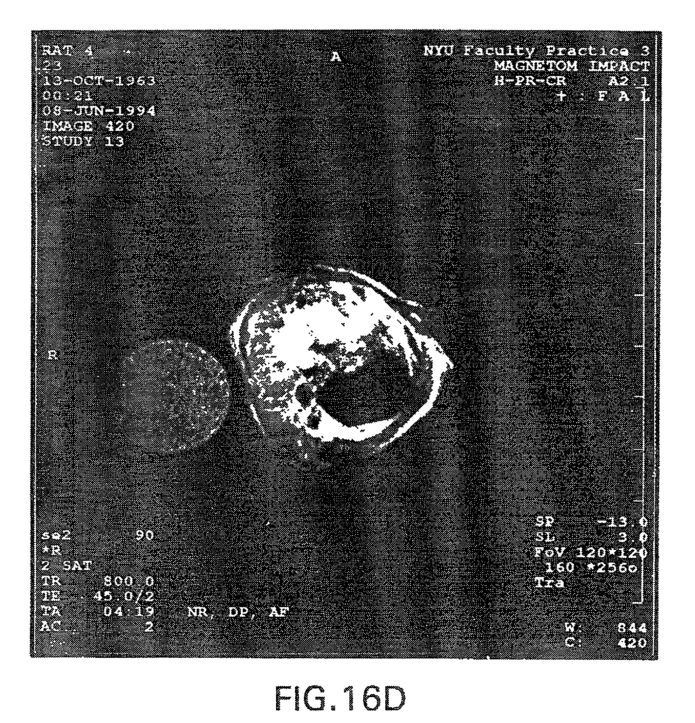

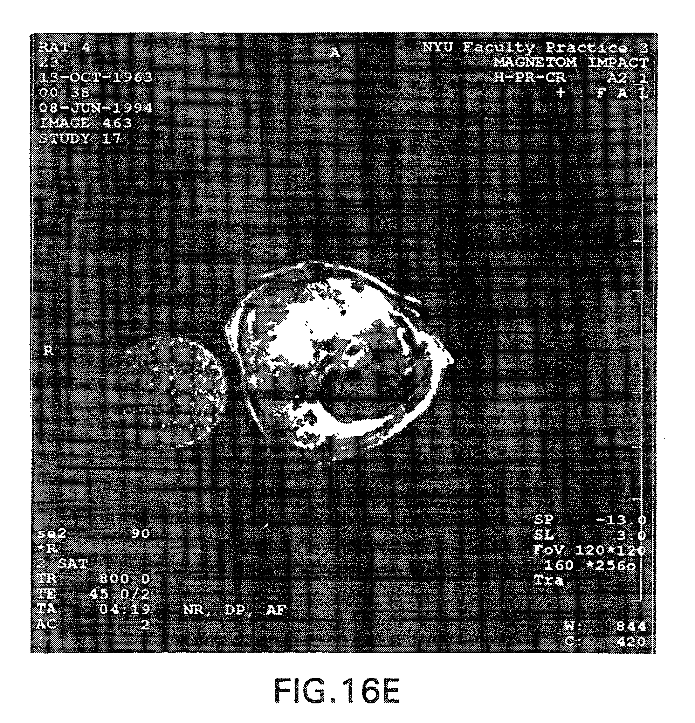

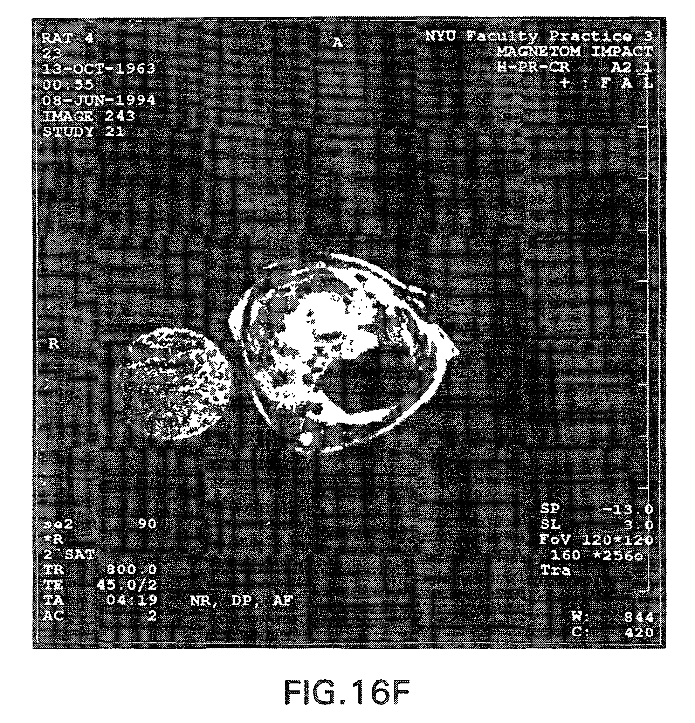

FIG. 16A, FIG. 16B, FIG. 16C, and FIG. 16D show MRI 4.7 Tesla, T1-weighted images of Copenhagen rats with the AT-1 prostate tumor model (as in FIG. 13A, FIG. 13B, FIG. 13C and FIG. 13D), but rats are injected i.v. with Ferrioxamine Selective Contrast Agent, wherein the Active is non-covalently bound to a non-anticoagulant GAG, Heparan Sulfate, the Agent lyophilized and reconstituted with water just prior to administration at a concentration of 0.332 mmol/mL Fe(III) and administered at the usual dose of 0.155 mmol of Fe(III) per Kg.

FIG. 16A. Precontrast.

FIG. 16B. 7 MPI.

FIG. 16C. 20 MPI.

FIG. 16D. 40 MPI. Note the very homogeneous enhancement of Outer Rim and Central Tumor at virtually all post-contrast times, in relation to the differential Rim enhancement achieved by essentially all of the other GAG carriers. This property may be useful in certain diagnostic and/or therapeutic applications.

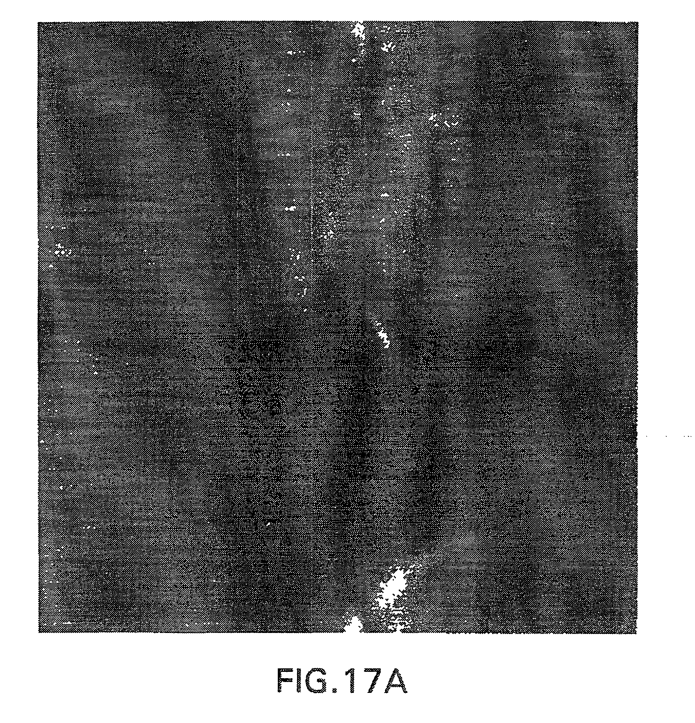

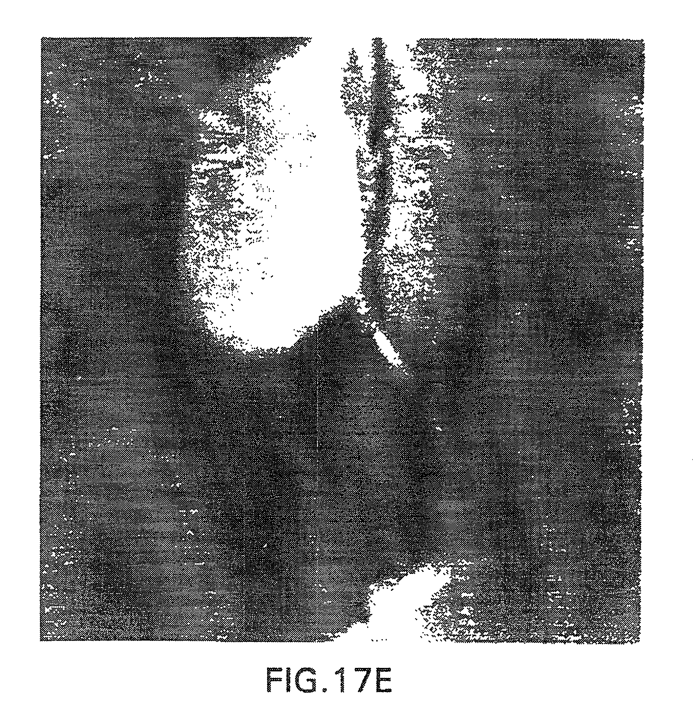

FIG. 17A is a control infrared (IR) spectrum of gadolinium diethylenetriaminepenaacetate (Gd:DTPA) (see Example 21).

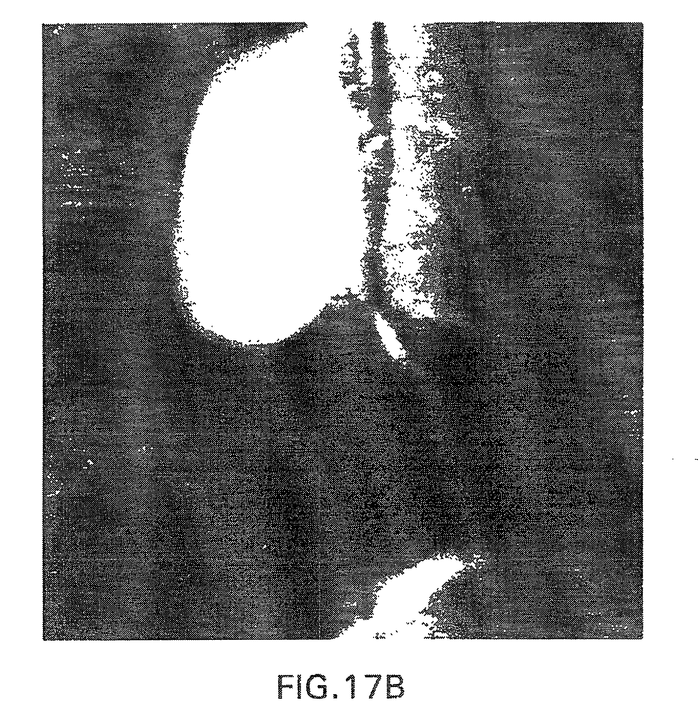

FIG. 17B is a control IR spectrum of N-methyl-1,3-propanediamine (MPD) (see Example 21).

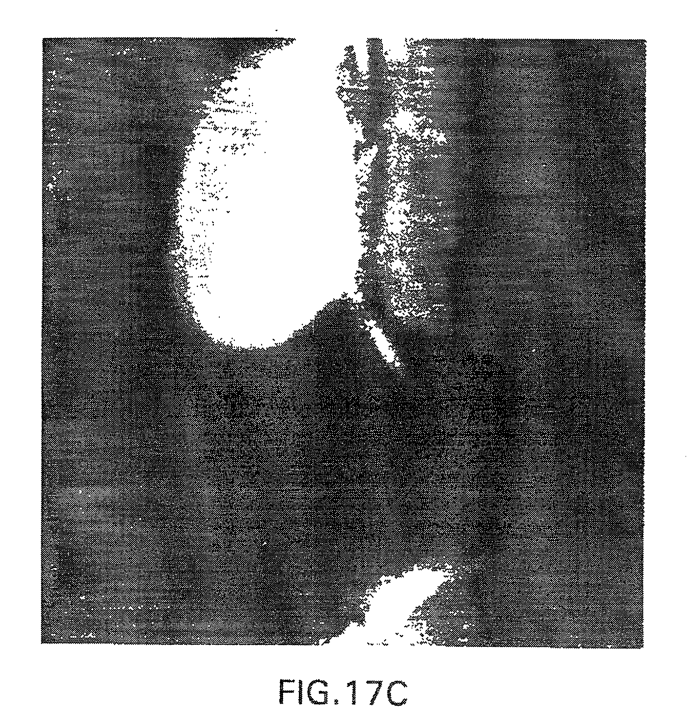

FIG. 17C is a control IR spectrum of a mixed (and dried) solution of the individual chemical components, Gd:DTPA and MPD (1:1 molar ratio).

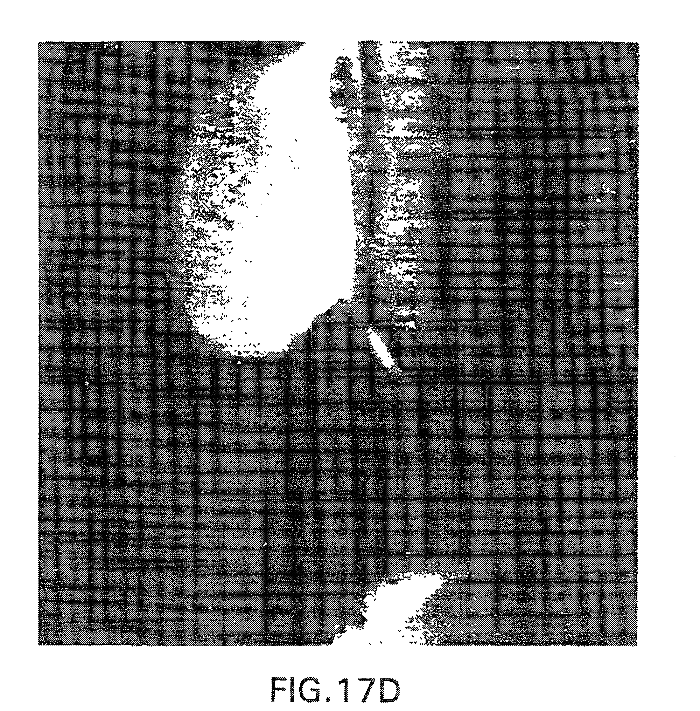

FIG. 17D is the experimental IR spectrum of MPD covalently conjugated at a 1:1 molar ratio to DTPA (as described in Example 21). Note the change in the height and splitting of the signature peak at 1400 wavenumber, and the change in the height and configuration of the broader stretching bands at 3300-3600 wavenumbers, which are indicative of covalent conjugate formation.

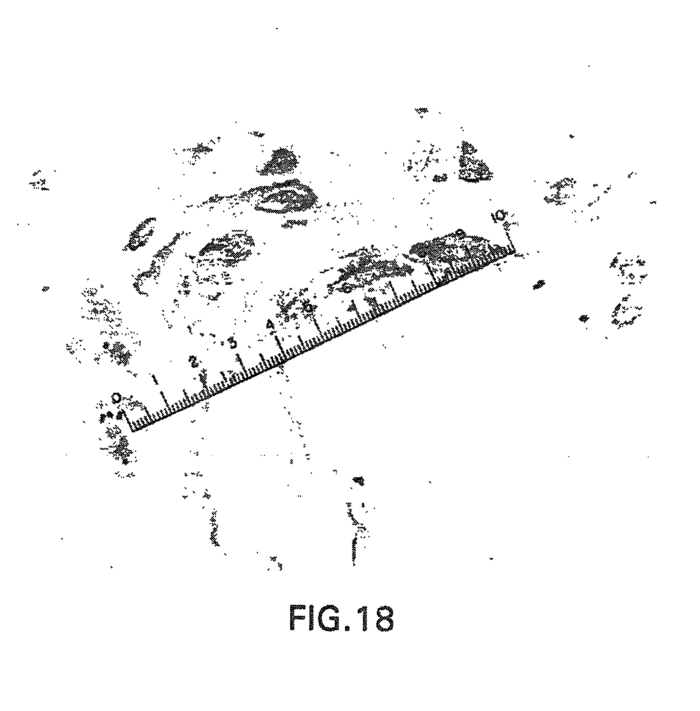

FIG. 18A shows a T2-weighted MRI scout image (TR/RE = 2100/85) of the liver regions of Fisher 344 female rats with syngeneic breast adenocarcinomas inoculated previously into the liver, such that tumor diameters at the time of imaging are between 1.0 and 2.5 cm, with the image acquired at 1.0 Tesla, just before performing the T-1 weighted series of images (shown below). This T2 image is performed in order to identify the approximate locations of 2 tumor nodules (right posterior liver) and 1 tumor infiltrate (central liver region), all tumor growths being confirmed at necropsy by gross visual inspection.

FIG. 18B, FIG. 18C, FIG. 18D, FIG. 18E and FIG. 18F show T-1 weighted images (TR/TR = 800/45) performed at 1.0 Tesla, before (Precontrast) and at various minutes after intravenous (i.v.) injection (Post-contrast, MPI) of Gd:MPD-DTPA:dermatan sulfate selective contrast agent, prepared as in Examples 21 and 22, and injected per Example 25, at a dose of 0.155 mmol/Kg into Fisher 344 female rats with syngeneic breast adenocarcinomas inoculated previously into the liver, such that the tumor diameters at the time of imaging are between 1.0 and 2.5 cm.

FIG. 18B. T1 Precontrast image of liver (tumor not conspicuous).

FIG. 18C. T1 liver image a 7 MPI, Gd:MPD-DTPA:dermatan sulfate selective contrast agent (0.155 mmol/Kg), showing extremely strong contrast enhancement of 2 solid tumor nodules (right posterior liver) and 1 irregular tumor infiltrate (central liver region), in the identical locations as those indicated by the T2-weighted scout image (FIG. 18A), but with much better definition of the tumor margins and much higher contrast gradients at the tumor margins. Note the moderately smaller size of tumor nodules and improved definition of the central tumor infiltrate, both due to an absence in the T1 mode of T2 imaging artifacts, namely an additional rim (corona) of water outside the actual tumor margin, which appears in the T2 pulse mode but not in the preferred T1 mode.

FIG. 18D and FIG. 18E. T1 Liver image at 20 and 40 MPI, Gd:MPD-DTPA:dermatan sulfate selective contrast agent (0.155 mmol/Kg), showing continued very marked contrast enhancement of the 2 solid tumor nodules (right posterior liver) and the 1 irregular tumor infiltrate (central liver region), with continued very highly demarcated tumor margins and essentially no contrast fading.

FIG. 18F. T1 Liver image at 20 and 40 MPI, showing continued very marked contrast enhancement of the 2 solid tumor nodules (right posterior liver) and 1 irregular tumor infiltrate (central liver region), with only a very slight degradation in the sharpness of tumor margins over 40 MPI, only a very minimal decrease (fading) of tumor contrast intensity in the 2 solid nodules (right posterior liver), a further brightening of the tumor infiltrate (central liver region), and a very slight background brightening of surrounding uninvolved liver.

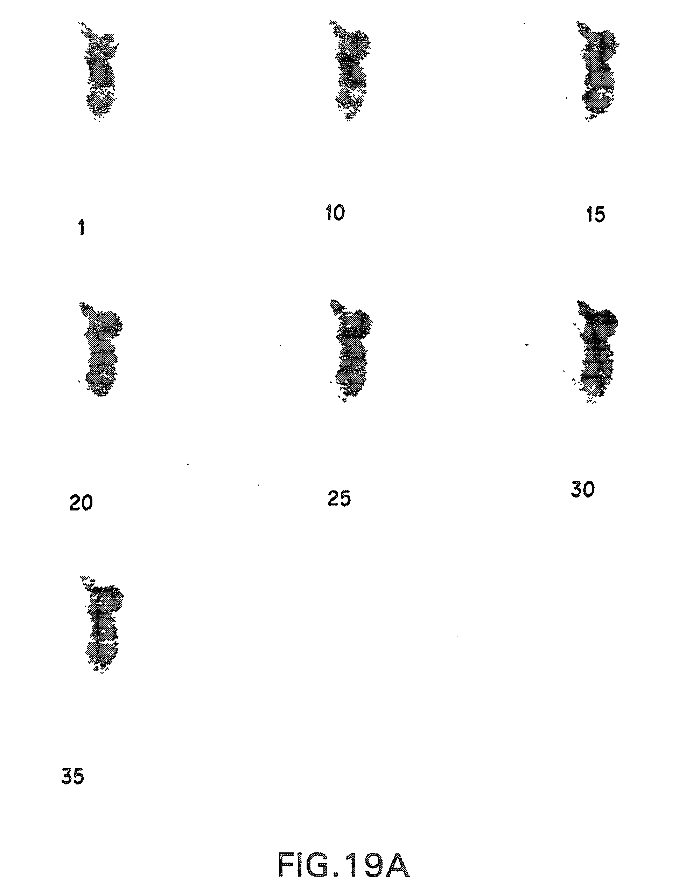

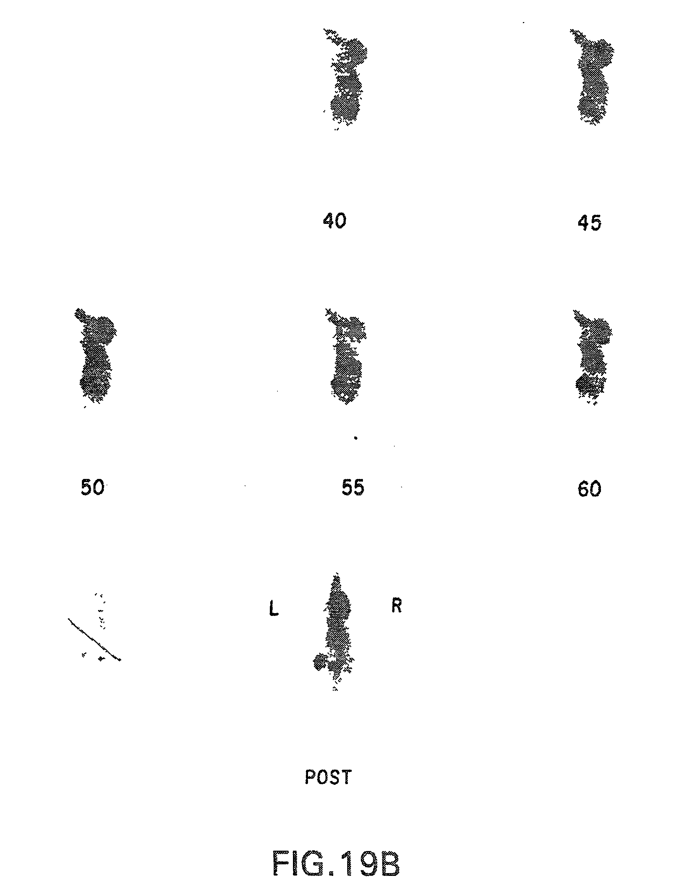

FIG. 19A, FIG. 19B, FIG. 19C, FIG. 19D and FIG. 19E show T1-weighted images at 4.7 Tesla (TR/TE = 250/8) of Copenhagen rats with syngeneic AT-1 prostate adenocarcinomas inoculated into previously prepared skin pouches [Hahn *et al.* (1993)], and imaged at diameters of 1.0-2.5 cm.

FIG. 19A. Precontrast image for Gd:MPD-DTPA:dermatan sulfate selective contrast agent, showing only the tumor and superficial back fat and back muscle, because a surface coil is used and not a whole body coil.

FIG. 19B. Post-contrast image, 7 MPI i.v. of Gd:MPD-DTPA:dermatan sulfate selective contrast agent, liquid form. Note the extremely strong enhancement of the entire tumor mass and the extremely strong gradient at the boundary between tumor and underlying normal tissue (image right).

FIG. 19C. Post-contrast image, 20 MPI i.v. of Gd:MPD-DTPA:dermatan sulfate selective contrast agent, liquid form. Note the extremely strong enhancement of the entire tumor mass and the extremely strong contrast gradient at the boundary between tumor and underlying normal tissue. Contrast has decreased slightly in the central tumor region, such that the tumor neovascular array is now very well visualized.

FIG. 19D and FIG. 19E. Post-contrast image, 40 and 60 MPI, of Gd:MPD-DTPA:dermatan sulfate selective contrast agent, liquid form. Note the still very strong enhancement of the tumor, and particularly the retention of an extremely strong contrast gradient at the boundary between tumor and underlying tissue. Contrast intensity in the central tumor and outer rim (image left, away from the animal) has decreased moderately, apparently due to progressive tumor accumulation in these regions, of such a high local concentration of the highly potent Gd:MPD-DTPA:dermatan sulfate [R1 = 7.8 (mmol.sec)⁻¹], that T2* effects are starting to produce competitive darkening of the central and outer tumor regions (image left; see also Example 26). The basal rim (image right), is relatively protected from this T2* darkening artifact, due to more rapid backdiffusion of the agent into plasma at this basal site. Hence, moderately lower doses are indicated.

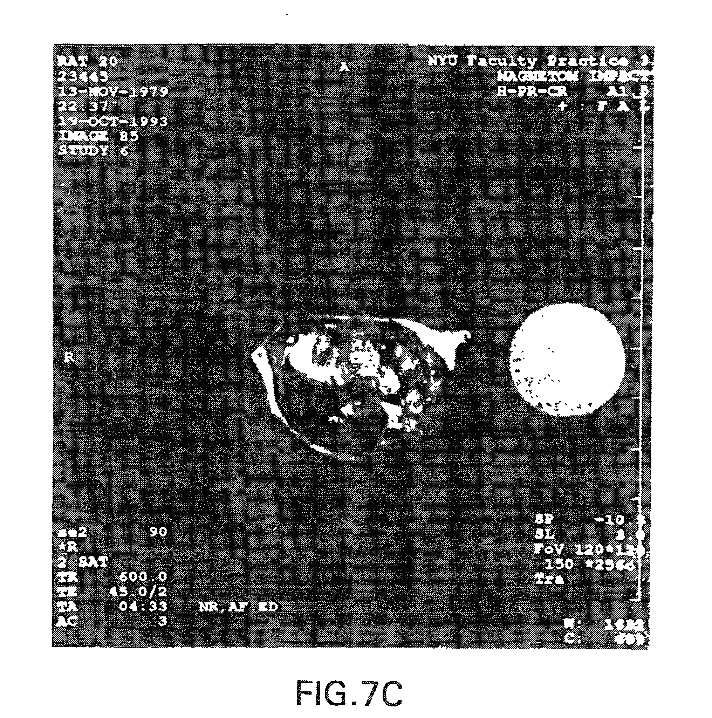

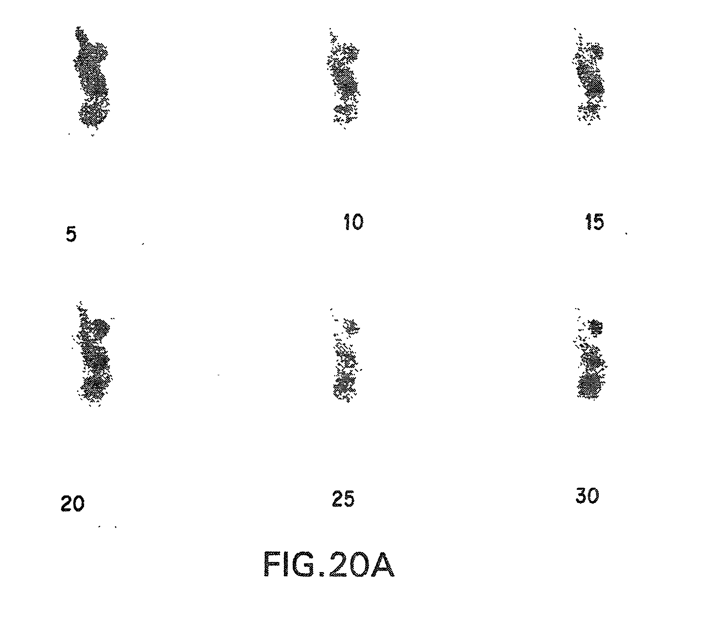

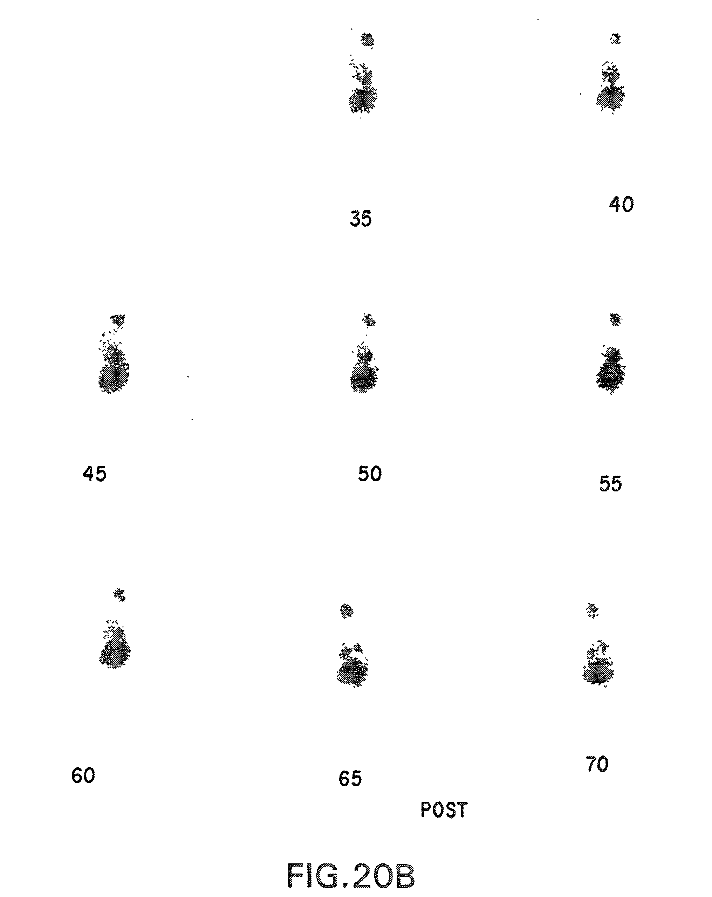

FIG. 20 shows a special histochemical stain (microwave augmented Prussian blue metal-ion stain) of AT-1 prostate adenocarcinoma (from Copenhagen rat), with the tumor tissue removed at 60 MPI just following the completion of MRI imaging, freshly frozen, sectioned and stained as above and as in Example 26 and FIG. 6 and FIG. 7. Note the selective staining positive for Gd(III) metal ion as follows: (a) very strongly positive within almost all tumor cells (tumor intracellular sites); (b) strongly positive at tumor-cell nuclei -- for many but not all tumor cells (e.g., see tumor cells underlying grid marker "9" and directly to the left of grid marker "10" at image right); (c) moderately positive neovascular endothelial cells (e.g., see directly above grid marker "8" at image top -- appearing as "railroad tracks": and directly under grid marker "2"); and (d) weakly positive to negative in subendothelial and extracellular matrix sites (= the spaces between tumor cells and endothelial ribbons). The low 60-minute staining of extracellular matrix may result from either or both of: (a) a more diffuse distribution of metal ions at 60 minutes (versus 7 minutes in FIG. 6 and FIG. 7A), diffuse metal ions being more difficult to visualize (due to their smaller optical staining niduses); or (b) plasma backdiffusion of a portion of the initially localized metal. These findings of metal-ion positivity in tumor endothelium, tumor matrix, tumor cells proper and tumor-cell nuclei, provide the basis for selectively localizing MRI and radionuclide diagnostic and therapeutic agents, and indeed, other types of active substances.

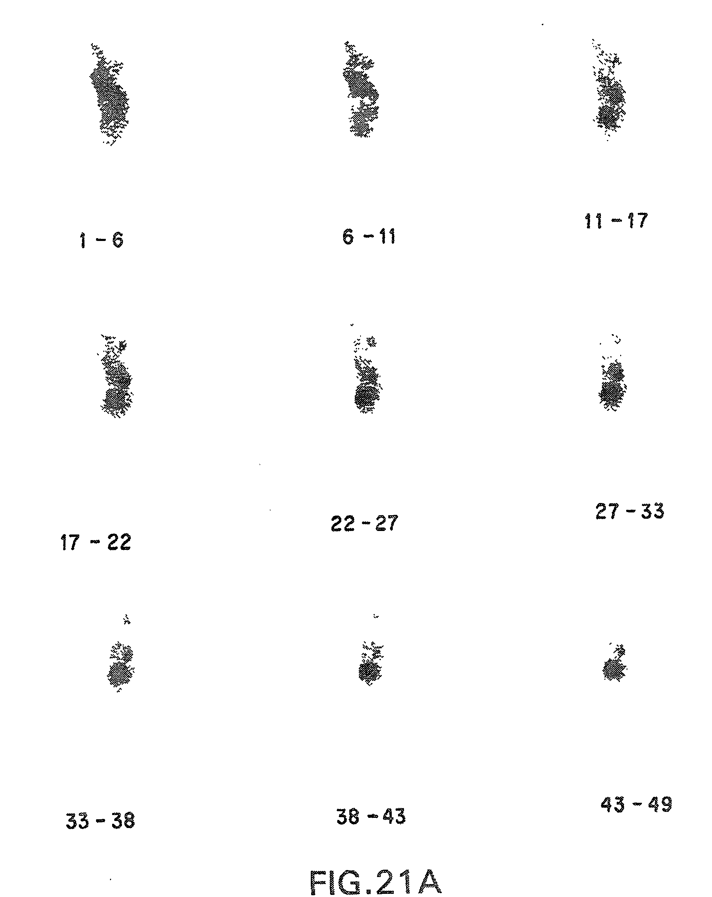

FIG. 21A, FIG. 21B, FIG. 22A, FIG. 22B, FIG. 23A, FIG. 23B, FIG. 24, FIG. 25A and FIG. 25B. Copenhagen rats with 2 to 5 cm AT-1 prostate adenocarcinomas grown in the skin pouch of the neck (as described above), injected intravenously with 200 to 300 uCi/rat of radionuclide metal, and where used, with ca. 0.35 umoles/rat of the respective chelator, and with gamma-camera images performed between 1 and 60 minutes of injection.

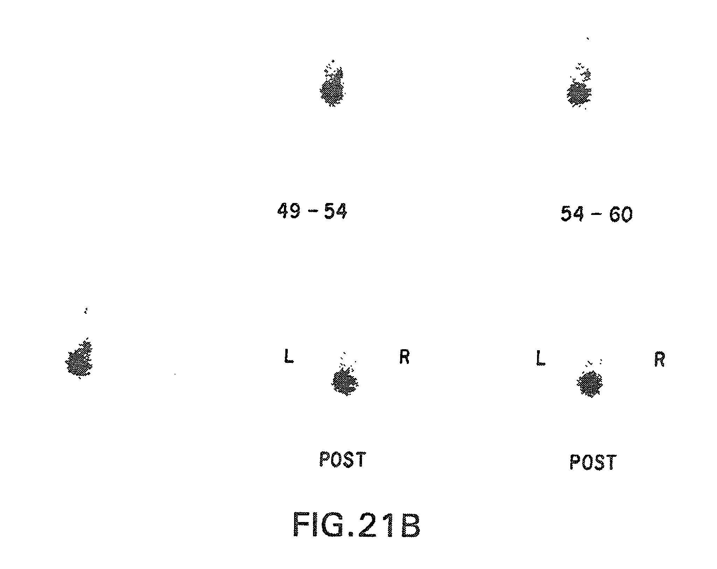

FIG. 21A and FIG. 21B show kinetic gamma-camera images (at the times marked) of a Copenhagen/AT-1 tumor rat injected with Ga-67 chloride. The blood pool is initially well labeled and the blood clearance rate is extremely slow, with very little clearance occurring by 60 minutes. Liver activity (background) begins to occur at an early time of 10-15 minutes and increases progressively over the 60 minute interval (as a function of Ga-67 binding to, and liver clearance of, transferrin and other plasma proteins).

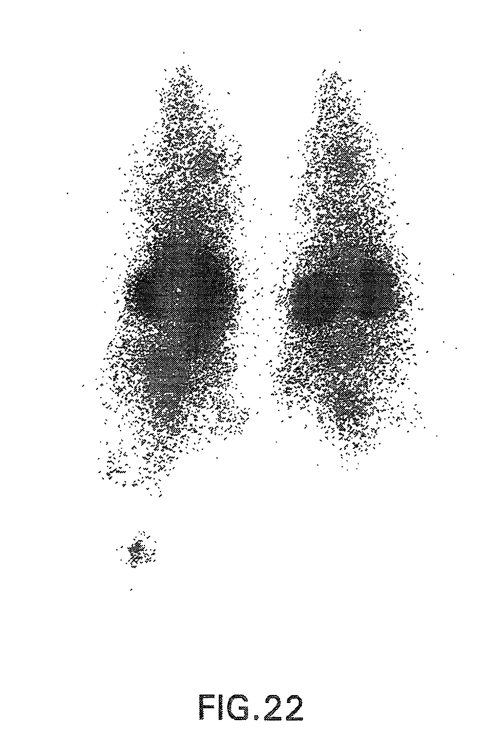

FIG. 22A and FIG. 22B show kinetic gamma camera images (at the times marked) of a Copenhagen/AT-1 tumor rat injected with Ga-67:DFo:DS (435 Type, Opocrin). The blood pool is initially well labeled, and blood clearance and tumor uptake are both very rapid, such that the tumor mass is very well visualized at all times after 10 minutes of injection, with exceedingly good tumor visualization between about 30 and 60 minutes (and also at later times -- images not shown). There is virtually no detectable liver uptake, clearance or background. Instead, clearance is entirely via the renal route, leading to early activity in the bladder region. Renal clearance is essentially complete by 48 hours (image not shown).

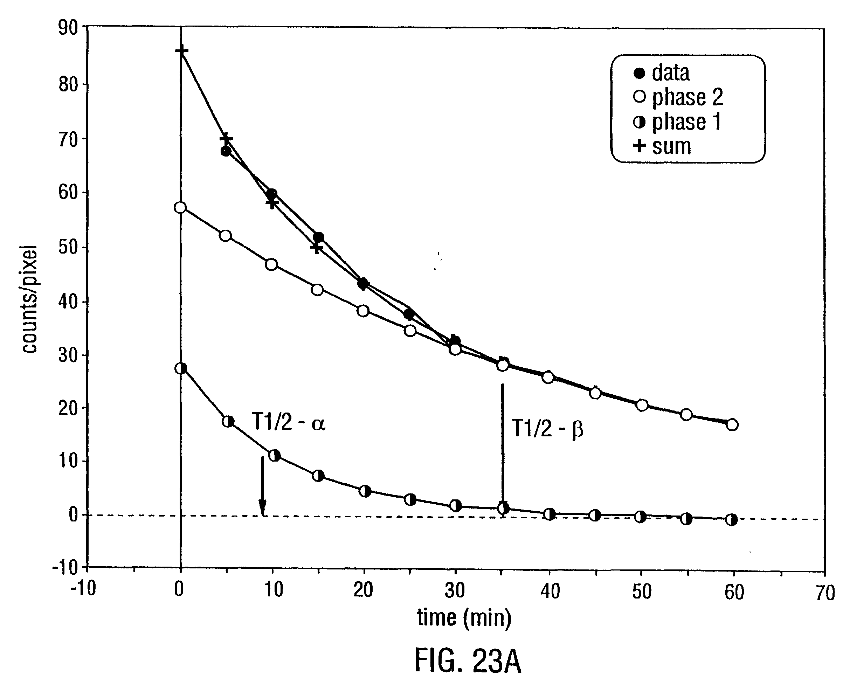

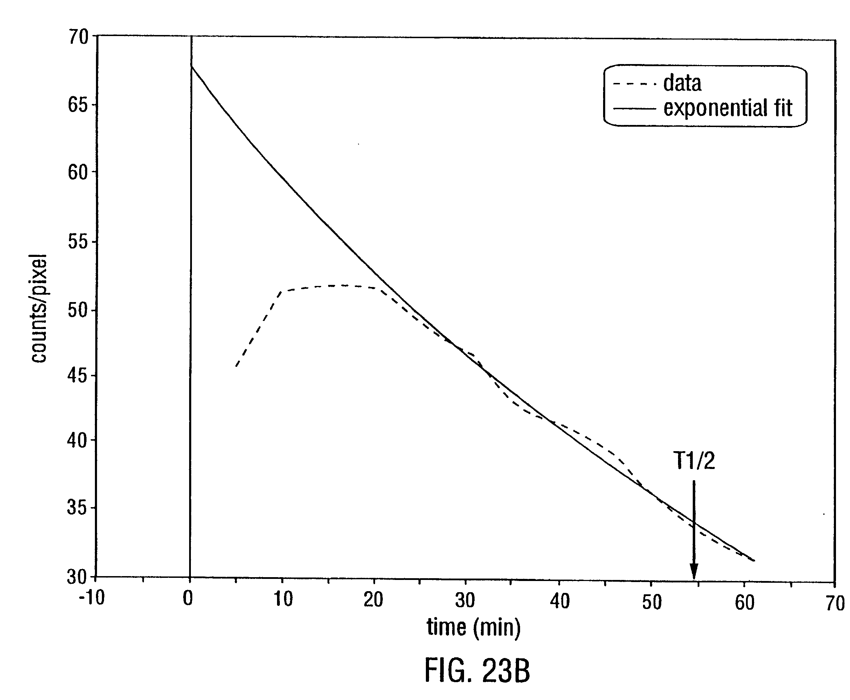

FIG. 23A and FIG. 23B show kinetic gamma camera images (at the times marked) of a Copenhagen/AT-1 tumor rat injected with Ga-67:DFo (without DS). The blood pool is initially well labeled, and blood clearance occurs very rapidly. Although a majority of the radionuclide agent clears very rapidly via the kidneys, a significant fraction also clears via the liver and generates a moderate liver background, which is detected as early as 11 minutes post-injection, and which increases further over the 60-minute imaging interval (as has been independently reported for DFo-metal ion complexes). Importantly, the tumor uptake is markedly lower and more transient than that observed for the full formulation with DS present (Ga-67:DFo:DS). By region of interest (ROI) analysis of image counts per pixel, there is only minimal tumor uptake over blood background at any post-injection time.

FIG. 24 shows a 3-hour gamma camera image of a Copenhagen/AT-1 tumor rat injected with In-111:MPD-DTPA:DS (435 Type, Opocrin) (right-hand panel); and with In-111:MPD-DTPA (without DS) (left-hand panel). The 3-hour post-injection image of In-111:MPD-DTPA:Ds shows continued very good visualization of the tumor mass, however the image of In-111:MPD-DTPA (without DS) shows lesser tumor uptake and much higher activities in the liver and bowel, which could obscure tumor detection if tumors were present in the liver or abdominal regions.

FIG. 25A and FIG. 25B. Clearance kinetics of Ga-67:DFo:DS (see Example 34, FIG. 22A and FIG. 22B (above), assessed by quantifying the gamma-camera counts per pixel, for regions of interest over the heart (for assessment of blood clearance) and the tumor mass.

FIG. 25A shows that the average blood clearance t1/2 is 18 minutes, with an even more rapid t1/2 alpha of 8 minutes, and a slightly slower t1/2 beta of 35 minutes. (The beta component and full backprojection are derived by full stripping of the alpha component.)

FIG. 25B shows a single-component clearance curve for the tumor, with a t1/2 of 54 minutes. This is 7 times longer than the blood t1/2 alpha and 3 times longer than the average blood t1/2. This is indicative of tumor retention of the initially localized agent.

### DETAILED DESCRIPTION OF THE PREFERRED EMBODIMENTS

The many innovative teachings of the present invention will be described with particular reference to the presently preferred embodiments, wherein these innovative teachings are advantageously applied to the particular issues of *in vivo* T1-Type MRI image contrast enhancement by site-selective localization and sustained site retention of paramagnetic metal chelates according to optimal spatial and kinetic profiles at the site, while simultaneously enhancing clearance and minimizing toxicity of the non-localized dose fraction. However, it should be understood that this principal embodiment is only one example of the many advantageous uses of the innovative teachings herein. For example, the various types of innovative compositions and methods disclosed herein can alternatively be used to selectively localize and enhance clearance of radionuclide imaging agents, X-ray contrast agents, ultrasound-acoustic image enhancing agents and a wide spectrum of therapeutic agents which are based on site delivery of metal chelates and in situ chelation of endogenous body metals. Of special interest to the therapeutic agents and uses embodied herein, are actives and indications useful in oncotherapy, cardiovascular infarcts, restenosis, atherosclerosis, acute thrombosis, microvascular disease, inflammation, and any other tissue diseases which have as part of their development or progression, a vascular component amenable to binding, adhesion, transport and/or modulation by the novel teachings, compositions and uses described herein. Hence, it will be obvious to those skilled in the art, that numerous additional compositions and uses are uniquely enabled by the present invention. The following examples are presented to illustrate preferred embodiments of the present invention, their uses in MRI contrast enhancement. These examples are purely illustrative, and do not in any way delimit the full scope of the present invention.

The present invention specifically describes the preparation and utilization of novel contrast agents for magnetic resonance imaging. These novel contrast agents consist of paramagnetic metal chelates, as distinguished from metal-atom complexes, wherein the presently disclosed chelates are bound to glycosaminoglycans as defined in the claim. Binding of the metal complex to the GAG may take the form of, but is not limited to, electrostatic interactions (ion-paired), hydrogen-bonding, Van der Waals interactions, covalent linkages, or any combination of these interactions. Following parenteral administration of these metal complex-glycosaminoglycan formulations, the technology described herein utilizes a biocompatible carrier molecule to deliver an associated biologically active substance to sites of vascular injury.

The present invention provides substantially improved MRI image and spectral enhancement compositions and methods, whereby the capacity of MRI hardware systems to detect tumors, cardiovascular diseases, and other diseases with a vascular or endothelial adhesive component are greatly enhanced. These improvements are presently accomplished by introducing a chelated paramagnetic metal ion selectively into tissue sites of interest, inducing selective (local) modulation of T1-Type, paramagnetic relaxation of water protons or other diffusible nuclei present within the site which are susceptible to orientation by fixed and gradient magnetic fields and to pulsed re-orientation by radiofrequency fields of appropriate resonant frequencies, thereby giving rise to detectable modulations of induced magnetic resonance signals, in the forms of either image contrast enhancement or spectral enhancement.

Past disclosures (Ranney: US serial No. 07/880,660, filed May 8, 1992, US Serial No. 07/803,595 filed April 3, 1992, and US Serial No.07/642,033 filed January 1, 1991] have dealt with the binding of magnetic agents which required: (a) magnetic potencies greater than that of the most potent single metal ion, gadolinium(III); (b) intramolecularly coupled, polyatomic metal-atom complexes stabilized by non-bridged ligands which have a stronger potential for chemical and physical instability than the stably, bridged-ligand chelated metal ions disclosed herein; and (c) divalent cationic charge on the "superparamagnetic" active for binding to anionic carriers, versus the presently disclosed requirement for only a monovalent cationic charge on paramagnetic metal chelator actives. It is understood, that for MRI uses, the metal chelator will also comprise an appropriate paramagnetic metal ion, for example, preferably iron(III) or gadolinium (III), however, for certain other diagnostic and therapeutic compositions and uses, the presently disclosed metal chelators may either comprise or avoid an appropriate metal ion. For the presently preferred MRI applications, basic metal chelators and metal chelators with electrophilic properties at formulation pH's are preferred, for example, ferrioxamine [Crumbliss, 1991], basic or amine derivatives of the polyaminocarboxylate chelator, diethylenetriaminepentaacetate (DTPA), and basic or amine derivatives of the macrocyclic chelator, 1,4,7,10-tetraazacyclododecane-N,N',N",N"'-tetraacetate (DOTA) [Li *et al.* 1993; Brechbiel *et al.* 1986]. In certain instances and with certain potent carriers bound to these and related actives, site localization may be so pronounced that the inherent potency *(in vitro* paramagnetic R1) of the paramagnetic metal ion may not be crucial to obtaining optimal site-localized image contrast or spectral enhancement effects. Hence, the present invention discloses pronounced T1 image contrast effects for the basic metal chelate, ferrioxamine, which by virtue of chelated Fe(III) ions, has a potency, or R1 relaxivity, of about 1.6-1.8 [mmol.sec]-1. Alternatively, basic metal chelates of Gd(III) maybe expected under certain but not all *in vivo* conditions, to have a potentially greater relaxivity, due to its greater *in vitro* R1 of about 4.0-4.3 [mmol.sec]⁻¹ when chelated by DTPA, and potentially moderately higher when chelated by DOTA [Geraldes *et al.* 1985], and as high as R1 ≥ 7.5 [mmol.sec]⁻¹ when Gd(III) is chelated to certain DTPA derivatives, including N-methyl-1,3-propane diamine-DTPA as one preferred embodiment of a group of preferred DTPA-amine and DTPA-basic derivatives which can both (a)"allow accelerated water diffusion and relaxation above that of DTPA; and (b) bind non covalently to acidic saccharides, including, preferably, glycosaminoglycans. Alternative metal ions may preferably include the divalent or trivalent cations, manganese, chromium and dysprosium; and less preferably, those ions of copper, nickel, erbium, europium, and holmium.

Preferred chelators of the present invention include those with a formation constant of at least about 10¹⁴ for strongly paramagnetic metal ions disclosed above, and including a basic or cationic group. These chelators preferably include ferrioxamine, basic or amine derivatives of DOTA, DTPA, porphines, porphyrins, sapphyrins or texaphyrins, which can preferably chelate Fe(III) and most preferably chelate Gd(III), as well as bind by principally paired-ion (electrostatic) means to the acidic groups of acidic carriers. For example, certain texaphyrins have an expanded macrocyclic ring which may, in certain instances, stably chelate Gd(III) [Sessler *et al*. '065; Sessler *et al*. '720; Sessler *et al.* '498. Whereas texaphyrins and sapphyrins are not exemplified in the present invention, it will be obvious to those skilled in the art, from the references cited just above, and from the presently disclosed and exemplified Fe(III) chelator, 5,10,15,20-Tetrakis(1-methyl-4-pyridyl)-21H,23H-porphine, that the related texaphyrins and sapphyrins and their basic, cationic and amine derivatives, as well as the presently disclosed porphine derivative and its analogues and basic, cationic and amine derivatives, would be included under the disclosures and teachings of the present invention, and may be used in combination with the presently disclosed acidic carriers. There are hybrid considerations of, among others: (a) paramagnetic potency of the metal chelate; (b) binding stability to the acidic carrier; and (c) formulation compatibility; and (d) biocompatibility and clearance *in vivo.* Hydrophilic chelators and carriers are usually, but not always preferred, due to their typically favorable formulation properties (absence of aggregation), biodistribution properties (absence of generalized binding to hydrophobic plasma and cell-membrane constituents during the process of localization) ; and clearance plus toxicity advantages. Alternative chelators may include the hydroxamates, ferrichrome, enterobactin, ferrimycobactin, ferrichrysin, and their basic or amine derivatives, all derivatives being defined as subsumed under the parent chelators listed above.

In all cases reported and tested, non-covalent binding of the basic amine chelator to the acidic carrier gives payloads of active agent which are markedly higher than those afforded by covalent conjugation. For example, preferred basic chelators, ferrioxamine and Gd(III) DTPA-lysine, and most preferred, N-methyl-1,3-propane diamine-DTPA (N-MPD-DTPA), are bound to their acidic glycosaminoglycan carriers at weight ratios of ≥ 70%. Alternative covalent active-carrier conjugates may be preferred in certain instances-, and preferred examples thereof are shown for MRI applications.

Specific embodiments of the present invention which have been tested *in vivo*, include, but are not limited to the presently exemplified preferred embodiments of: (a) deferoxamine, (b) ferrioxamine, (c) Gd(III) :DTPA-lysine, (d) N-methyl-1,3-propane diamine-DTPA, and (e) other basic metal chelates bound most preferably by non-covalent means, and also preferably by covalent means, as exemplified below, to dermatan sulfate as defined in the claims. These carrier substances being about low toxicity and optimal safety margins at the higher doses which typify MRI contrast agent administrations. These dermatan sulfates comprise relatively low SO3-/COO- ratios of preferably between 0.7:1 and 1.8:1, most preferably between 0.9:1 and 1.5:1, and typically 1:1; and additionally with relatively low sulfur content of preferably less than 9% (w/w), most preferably between 4% and 7% (w/w/), and typically 6.3-6.4% (w/w). This new special class of dermatan sulfates with oversulfation of only selected oligosaccharide sequences but without overall oversulfation of the entire molecule is described and defined above and below. Alternative preferred Agents obvious from the present disclosures, to those skilled in the art, may induce arginine and histidine basic derivatives of DTPA and DOTA, and also of the various texaphyrins, sapphyrins, porphines, porphyrins, EHPG, and by definition, most preferably for MRI applications, comprising their Gd(III) and Fe(III) metal-ions, and also preferably comprising their alternative paramagnetic metal ion chelates, as disclosed above.

The carrier substance used in the present invention is the class of new dermatan sulfates, enriched in uronic (L-iduronic) acid content and, in addition to its major monosulfated disaccharide sequence, (Ido-GalNAc₄SO₃), also characterized by an oligosaccharide sequence with a selectively high degree of sulfation, including the oversulfated saccharide sequences, (IdoA₂SO₃-GalNAc₄SO₃) and (IdoAGalNAc₄, 6SO₃) (as assessed by disaccharide analysis and as uniquely correlated with ¹H and ¹³C magnetic resonance spectra), enriched in heparin cofactor II activity, preferably greater than 220 Units/milligram, but low in factor Xa and antithrombin III activity and in overall anticoagulant activity (preferably less than 10% and most preferably less than 5% of standard heparin by USP anticoagulant assay), low in SO₃-/COO- ratio, preferably in the range of 0.7-1.8 and most preferably in the range of 0.9-1.5, and low in sulfur content, preferably less than 9% and most preferably in the range of 4 to 7%; and preferably having a modal molecular weight of between 10,000 and 23,000 daltons, and most preferably between 13,000 and 19,000 daltons -- the lower end of this molecular weight bracket generally being important in order for the carrier to be highly retained within the vascular compartment of normal organs after intravenous administration; and the higher end of this molecular weight bracket generally being important for effective disease site binding and uptake, while still affording the very rapid blood clearance by the renal route, which is important for rapidly achieving low blood imaging backgrounds and low body residua at early post-injection times.

The dermatan sulfates of the preceding paragraph may, in one case, be prepared by the methods of: (a) grinding and treating animal organs or tissues, including beef mucosa, swine skin or lung, and preferably for certain of the present uses, beef mucosa, with proteolytic enzymes including papain, at pH 5 to 7 for the shortest possible time to remove proteins; (b) passage over a strong anion (basic) exchange resin including a macroreticular styrene-divinylbenzene matrix functionalized with quaternary ammonium groups and having a particle size range of 0.3 to 1.3 mm; (c) eluting the sulfated polysaccharides with a neutral salt solution between of 0.7 and 2.0 molarity; (d) crystallization of the dermatan sulfate as a low-solubility salt of a bivalent or trivalent metal including copper, iron and calcium, and preferably copper; (e) reconversion to sodium salt via cation exchange resin including chelex 100 type (Bio-Rad 143-5852); selectively enriching for the oversulfated oligosaccharide sequences (above) by chromatography on a strongly basic anion exchange resin functionalized with quaternary ammonium groups, wherein the resin typically has a particle size of less than or equal to 10 micrometers and a cross-linkage of 2-8%; (f) concentrating the eluate by reverse osmosis; and (g) lyophilizing the resulting liquid to form a fine white powder. One example of the above dermatan species, which is not intended in any way to limit the scope of the present invention, comprises a subspecies of these dermatan sulfates (sulphates), as described [Mascellani, *et al.* WO 93/05074. (1993) ; Mascellani, *et al.* (1994)]. One of most preferred example of this subspecies of dermatan sulfate is the Type 435 beef mucosal dermatan sulfate (sulphate) manufactured and supplied by Opocrin S.P.A., Via Pacinotti, 3, I-41040 Corlo Di Rormigine, Italy. It has a modal molecular weight of approximately 17,500 to 18,000 daltons, as determined by charge suppressed molecular sieve chromatography with UV absorbance analysis, and a sulfur content of approximately 6.2 to 6.6% weight percent -- this low sulfur content occurring despite the selective enrichment in these dermatan sulfates of certain oligosaccharide sequences with a high degree of sulfation, including the oversulfated saccharide sequences, (IdoA₂SO₃-GalNAc₄SO₃) and (IdoAGalNAc₄, 6SO₃) whose enrichment correlates with high heparin cofactor II activity.

In the descriptions of the two preceding paragraphs, (a) enrichment for uronic (L-iduronic) acid content plus the preceding 2,4-disulfated disaccharide sequences in combination with (b) the preferred molecular weights in the range of 10,000 to 23,000 and most preferably 13,000 to 19,000 daltons, and (c) low SO₃-/COO-ratio, corresponding to a low overall sulfur content, typically in the range of 4.5 to 7% by weight, correlates with the surprising and unexpected advantages of: (a) *in vivo* potency of rapid disease-site binding, localization, uptake and deep penetration, *e.g.,* of tumor endothelium, tumor extracellular matrix and tumor cells; and (b) low side effects of induced platelet aggregation, anticoagulation and bleeding -- which are characteristically induced by the more highly sulfated and/or longer-chain (higher molecular weight) carriers, including sulfated, oversulfated and polysulfated glycosaminoglycans and natural and synthetic sulfated, oversulfated and polysulfated polysaccharides and sulfatoids -- most specifically those with a sulfur content of 10% or greater, and those with a USP heparin-type anticoagulant activity ranging from 15 to 145 USP units per milligram or greater.

The new special dermatan sulfate subspecies (as prepared by the special processes described above), when used as site-selective diagnostic or drug carrier substances, are clearly distinguished from all of the previous, older dermatan sulfates, *i.e.*, those (a) not having the special structures described above; (b) not prepared according to the above isolation and purification processes; or (c) not prepared by such alternative processes as would give comparable enrichment of the preferred oligosaccharide sequences and selective sulfations described above. These dermatan sulfates are also clearly distinguished, when used as above, from all of the prior older dermatan sulfates in that they are not only structurally different, but they are also essentially free of the contaminating heparins, heparan sulfates and heparinoids which bind normal endothelium, undergo various degrees of *in vivo* metabolism, and interfere with rapid and complete blood and body clearance [Dawes, *et al.* (1989), incorporated herein by reference]. It will be further obvious to those skilled in the art, that the new special dermatan sulfates described above, are, when used as site-selective diagnostic or drug carrier substances, even more distantly distinguished from the non-dermatan sulfate classes of glycosaminoglycans, namely: (a). chondroitin sulfates A and C -- which do not share the uronic (L-iduronic) acid sugars of dermatan sulfate [Walton, *et al.,* US Patent 4,489,065; Maeda, *et al.* (1993)]; (b) heparin -- which does share uronic (L-iduronic) acid structure but which has high anticoagulant activity and high binding to normal endothelium [Cremers, *et al.* (1994); Kalishevskaya, *et al.* (1988); (c) hyaluronic acid -- which is a non-sulfated glycosaminoglycan; (d) all of the polysulfated glycosaminoglycans and oversulfated sulfatoids, e.g., bacterial polysulfates including pentosan polysulfate -- all of which characteristically have sulfur contents of 10% or greater that create significant *in vivo* safety issues due to polysulfate-induced platelet aggregation and cell membrane perturbation/lysis, or act as cofactors for such cellular lysis and which can affect normal body cells as well as tumor cells and other pathological cells/organisms, such as that specifically described as direct toxic cofactor "opening" of tumor cells produced by chondroitin polysulfate, resulting from chondroitin polysulfate-induced membrane damage [Landsberger (1984)]. Hence, the new special dermatans preferred in the present invention are ones which do not themselves cause significant direct cellular or membrane damage, but instead induce rapid (3-to 7-minute) selective binding of disease-site endothelium, rapid (10 to 5-minute) endothelial cell transport, tumor uptake, deep matrix permeation and tumor-cell internalization of the attached diagnostic or drug active without the dermatan sulfate carrier itself or alone, damaging either the intermediate (e.g., endothelial) or final (e.g., tumor) target cells.

This new special class of dermatan sulfate is clearly distinguished from chondroitin sulfate Types A and C by its high content of L-iduronic (uronic) acid relative to the low or absent content in chondroitin sulfates A and C; and by its relatively lower modal molecular weight, most typically less than 25,000 daltons versus the chondroitin sulfates A and C, which typically equal or exceed 25,000 daltons modal molecular weight. The relatively lower molecular weight of the new special dermatan sulfates has at least three surprising and unexpected advantages when used as a carrier substance for bound or associated active substances: (a) very rapid blood clearance of the carrier and active, predominantly by the renal route, with a blood t 1/2 of typically about 20 to 120 minutes, increasing only very gradually as a function of increasing dose; (b) minimal to absent in vivo metabolism -- in major contrast to standard heparins, heparan sulfates and chondroitin sulfates A and C -- thereby giving extremely low residual *in vivo* deposition or retention of the carrier material; and (c) maximal, rapid vascular egress across disease-site endothelium -- including across induced and "permeabilized" endothelium, e.g., induced by Vascular Endothelial Growth Factor/Vascular Permeability Factor (VEGF/VPF) for maximal disease-site and tumor access, uptake and tumor-cell internalization of the bound or associated active substance.

Whereas, this new class of dermatan sulfates has been recognized as useful for conferring antithrombosis in the absence of (heparin-type) anticoagulant activity and bleeding side effects, it has not previously been recognized, nor would it be obvious to one skilled in the art, that this new special class of dermatan sulfates could confer the surprising and unexpected advantages of acting as a highly potent and effective *in vivo* carrier of noncovalently or covalently bound amine or chemically basic chelators or metal chelates, furthermore, to selectively localize them in sites of disease, including tumors, across non-permeabilized as well as "permeabilized" vascular endothelium and simultaneously to promote very rapid clearance of the non-targeted fraction of carrier plus active, highly preferentially by the renal route, in a fashion which increases only very gradually with increasing dose -- thereby conferring not only reduced side effects and low *in vivo* retention, but also the additional advantages of: (a) very low imaging backgrounds at very early times post-injection upon intravenous administration for the purpose of *in vivo* contrast enhancement by associated paramagnetic metal chelate; and (b) pronounced capacity for dose escalation with acceptable safety. These surprising and unexpected advantages are particularly important for use in paramagnetic enhancement of *in vivo* magnetic resonance images (MRI) because of low sensitivity of the imaging equipment and detection method, and hence, the need for injecting high intravenous doses of paramagnetic metal chelate (typically in the range of 0.1 to 0.3 mmol/kg) in order to deposit sufficient local-site concentrations of paramagnetic agent (ca. 50-100 micromolar). This further emphasizes the advantage of using a carrier material, including the new special dermatan sulfates, which can preferable allow a noncovalent method of binding the active to the carrier, and hence, can enable a high quantity of active to be bound per unit of carrier, preferably greater than 70% (weight % of active to [active + carrier]) versus typically 7 to 12% (w/w) for most covalently bound active-polymer systems, including antibody systems. Hence, the self-assembling, noncovalent formulation (as well as covalent formulation) properties of the new special dermatan sulfates provide an additional surprising and unexpected advantage of minimizing the quantity of dermatan sulfate carrier required to administer and selectively localize an effective *in vivo* dose of paramagnetic chelate.

The present invention describes the preparation and utilization of a novel MRI contrast agent for enhancement of solid tumors and cardiovascular infarcts. The contrast agents consist of cationic or basic paramagnetic metal complexes in association with carriers as defined in the claims. Of the paired-ion systems described below, most notable are those consisting of ferrioxamine with glycosaminoglycans, DTPA-lysine with glycosaminoglycans, N-methyl-1,3-propanediamine-DTPA with glycosaminoglycans, and most preferably, N-methyl,3-propanediamine-DTPA with the new special subspecies of dermatan sulfates described above.

It is envisioned that alternative diagnostic and therapeutic compositions and applications may be carried out using compositions substantially similar to those disclosed above. For example, alternative metal ions may be chelated for purposes of metal-ion exchange at the site. Hence, the present formulations may contain or comprise metal ions of manganese, aluminum, germanium, zinc, cobalt, calcium, platinum, or others. Alternatively, for purposes of radiation and radionuclide therapy, such compositions may contain or comprise metal ions of boron, cobalt, rubidium, yttrium, technetium, ruthenium, rhenium, indium, iridium, thallium, samarium or others. Specifically, and in some cases preferably, ⁵⁹Fe and ⁶⁷Ga [Hashimoto *et al.* 1983; Janoki *et al.* 1983] may be substituted as radionuclide forms of the non-radioactive metal ions, for purposes of nuclear medical imaging of tumors, thrombi, and other biomedical imaging purposes.

**TABLE 1**

| Advantages of Metal Ion Chelator and Anionic, Hydrophilic Carrier | | | | |
|---|---|---|---|---|
| Technology | Selective MRI Agent | Antibodies | PEG | Liposomes |
| Property | | | | |
| Drug Payload | High* 60-90%;** 77.5% | Very Low 5% | Low 10-30% | Low 15-20% |
| Localization in Tissue Sites | Yes | Very Low | No | No |
| Selectivity | Broad Immune (CHO-lectin) | Narrow Immune (Ab-antigen) | None | None |
| Time to Target | Very Rapid (several mins) | Slow (several hrs) | Slow (many hrs) | Very Slow (hrs-days) |
| Time to Clear Plasma & Body | Rapid | Very Slow | Very Slow | Extremely Slow (RES) |
| Applications | Broad (Tissue Sites) | Narrow (Intravascular) | Narrow (Enzymes) | Narrow (RES) |

| | | | | |
|---|---|---|---|---|
| *preferred | | | | |
| ** most preferred | | | | |

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. without departing from the spirit and scope of the invention.

In all of the following Examples, except as otherwise stated, all references to dermatan sulfate and native dermatan sulfate refer to the new special class of dermatan sulfates with oversulfation of only selected oligosaccharide sequences but without overall oversulfation (hypersulfation) of the entire molecule (as described and defined herein), and in particular refer to the new special "435 Type" of dermatan sulfate as supplied by Opocrin S.P.A., Via Pacinotti, 3, I-41040 Corlo Di Formigine, Italy. Examples 6,7,8 (with exception of agent (c)),10,11,12,13,14,15,16,21 are reference examples only and do not exemplify the claimed invention.

### EXAMPLE 1

### Preparation of Deferoxamine Free Base and Use in Formulation of Ferrioxamine

The free base of deferoxamine is used in certain instances, in order to minimize the residual salt content present in final formulations which utilize deferoxamine as a basic metal chelator. In these instances, deferoxamine is precipitated out of aqueous salt solutions by the addition of 2 N KHCO₃, as previously reported [Ramirez *et al*. (1973), incorporated by reference herein]. A saturated solution of deferoxamine (320 mg/mL at 25°C) is prepared by dissolving 4.0 g of deferoxamine mesylate salt in 12.5 mL of pharmaceutical-grade water. The solution is cooled to 4°C in an ice bath and 2.5 mL of 2.0 N KHCO₃ added. The glass container is scratched with a stainless steel spatula to initiate precipitation. The precipitate is collected by centrifugation, washed repeatedly with ice cold water, and filtered. The crude deferoxamine free base is purified by sequential recrystallization from hot methanol. The resulting pure deferoxamine free base is dried under a stream of nitrogen. The infrared spectrum of the deferoxamine as prepared is consistent with that referenced above.

Ferrioxamine is formulated from the deferoxamine free base by addition of ferric chloride at stoichiometric molar ratios of Fe(III) to deferoxamine free base. This results in chelated iron and minimizes residual mesylate and chloride ions.

### EXAMPLE 2

### Preparation of Ferrioxamine-Iron (III) Chelate

Batch quantities of the Fe(III) chelate of deferoxamine are prepared as follows. Deferoxamine mesylate (methanesulfonate) (Ciba-Geigy Limited, Basel, Switzerland), 390 g, is dissolved in pharmaceutical-grade water. Alternatively, the chloride salt of deferoxamine may be used. A highly purified slurry of ferric iron in the form of Fe(O)OH (13.44% w/v of Fe(O)OH particles, Noah Technologies Corporation, San Antonio, Texas), 372.9 g is suspended in 1899 mL of water and added to the deferoxamine with constant stirring. The resulting suspension is heated to 60° C for between 1 and 24 hours and the pH adjusted periodically to between 6.5 and 7.9 by addition of 0.10 N NaOH. Formation of the ferrioxamine complex is evidenced by development of an intense, deep reddish-brown color to the solution. Stoichiometric chelation of Fe(III) with deferoxamine is confirmed by in-process UV-Visible absorbance spectroscopy at 430 nm, against stoichiometrically chelated ferrioxamine standards. The batch solution is cooled to room temperature and centrifuged at 4500 rpm (≈2500 g) for 15 minutes to remove any unreacted or aggregated Fe(O)OH. This final batch volume is adjusted as desired, typically to a final volume of 2600 mL. Any remaining trace amounts of unreacted Fe(O)OH are removed and the solution also made aseptic, by passing the supernatant through a 0.22 *µ*m Millipore GV-type filter in a Class 100 laminar flow hood. The resulting batch is stored at 4°C in an autoclaved, sealed glass container until further use (see Examples below). The final concentration of ferrioxamine (DFe) is determined once again by UV-Visible absorbance spectrophotometry at 430 nm. The R1=1.6 (mmol.sec)⁻¹, based on ICP-AA measurement of Fe(III).

### EXAMPLE 3

### Preparation of the Basic Amine Chelator: Diethylenetriaminepentaacetate-Lysine (DTPA-Lys)

DTPA, 500 mg, is dissolved in 20 mL of pharmaceutical-grade water and heated to 60°C. L-Lysine hydrochloride powder, 931 mg, is added with constant stirring until dissolved. Alternatively, N-epsilon-t-BOC-L-lysine can be used to prevent reaction of the carbodiimide intermediate at the lysine epsilon amino group (see below), and when used, is dissolved in dimethylformamide:water (50:50, w/v). The solution pH is adjusted to 4.75 by addition of 0.1 N HCl. The water-soluble carbodiimide, 1-ethyl-3-(3-dimethylaminopropyl)carbodiimide HCl (EDC), 732.5 g, is dissolved in 2 mL water and its pH also adjusted as above. This EDC solution is added dropwise to the DTPA + lysine solution mixture (above) over 1 hour at 22°C with constant stirring and periodic adjustment of pH to 4.75, and the reaction allowed to proceed to completion over 2 more hours. When N-epsilon-t-BOC-L-lysine is used (see above), the N-epsilon-t-BOC group is hydrolyzed at this step, by acidification with hydrochloric acid to a pH of between 1.0 and 2.0, and stirring for 30-60 min. The pH is readjusted to 4.75 as needed, and the reaction solution is concentrated down to 5 mL by rotary evaporation at 60°C, and the DTPA-lysine (DTPA-Lys) derivative is precipitated by addition of 3 volumes of ethanol. Note: under these conditions, the ethanol:water ratio used, maintains the solubility of all individual substrates (above). The resulting precipitate is harvested by centrifugation at 2,500 x g, washed with ethanol, re-centrifuged, and dried over a stream of dry nitrogen. Covalent conjugation of lysine to DTPA is confirmed by infrared (IR) spectroscopy. The resulting reaction product has a faint yellow color.

### EXAMPLE 4

### Preparation of the Gadolinium(III) Metal Chelate of DTPA-Lys: gadolinium:DTPA-Lys [Gd(III):DTPA-Lys]

The gadolinium(III) chelate of DTPA-Lys, namely Gd(III):DTPA-Lys, is prepared by dissolving a known quantity of DTPA-Lys in water and adding a stock solution of gadolinium chloride, prepared at 0.1-1.0 M, as needed, until a stoichiometric quantity of Gd(III) has been added. The pH is adjusted to 7.0 by addition of 1.0 N NaOH. Alternatively, gadolinium oxide can be added and the reaction mixture stirred for 24 hours. In the case of gadolinium oxide, neutralization with 1.0 N NaOH is not needed. Each batch of Lys-DTPA conjugate is pretitrated and the final chelation product checked for stoichiometric addition of Gd(III), using a standard xylenol orange titration method [Lyle et al. (1963)], and further confirmed by quantitative ICP atomic absorption spectroscopy for gadolinium. The resulting Gd(III):DTPA-Lys is precipitated by addition of ethanol (3 volumes per volume of water), and the precipitate collected by centrifugation. This precipitate is rewashed with ethanol and centrifuged (as above), washed with acetone plus centrifuged, and the collected precipitate dried over a stream of dry nitrogen. The resulting product continues to have the same faint yellow color as noted in Example 3. The R1 of aqueous product = 4.2(mmol.sec)⁻¹ based on ICP-AA measurement of Gd(III).

### EXAMPLE 5

### Preparation of Paired-ion Agents of Ferrioxamine bound to Dermatan Sulfate Carriers; and Ferrioxamine to Depolymerized Dermatan Sulfate Carrier

Ferrioxamine:dermatan sulfate paired-ion agents are prepared by mixing appropriate ratios of the water solutions of ferrioxamine (see Example 2, above) with either: (a) dermatan sulfate of modal MW between approximately 5,000 daltons and 45,000 daltons (Opocrin, S.p.A., Modena, Italy, 435 type from beef mucosa modal MW=18,000 daltons; and Scientific Protein Laboratories, Waunake, Wisconsin, from porcine mucosa, modal MW=19,600 daltons); or (b) depolymerized dermatan sulfate of modal MW between approximately 2,000 daltons and 5,000 daltons (Opocrin S.p.A., Modena, Italy, 370 type from beef mucosa, depolymerized from 435 type starting material). A range of ratios of ferrioxamine to dermatan sulfate are prepared between a low of 1:99 (wt %) of ferrioxamine:dermatan sulfate or depolymerized dermatan sulfate; and a high of 30:70 (wt %) of ferrioxamine: dermatan sulfate or depolymerized dermatan sulfate). Using 0.1 to 1.0 N NaOH, the pH of the mixture is adjusted to between 5.5 and 8, the mixture is stirred continuously for 0.5 to 72 hours and the pH re-adjusted between 5.5 and 8, and typically to 7.5. This ferrioxamine:dermatan mixture is passed through a 0.22 *µ*m filter to remove any residual insoluble iron oxides and hydroxides, and to render the liquid agent aseptic. The aseptic agent is stored either as a liquid at 4°C, or as a lyophilized powder (see below). Further processing is carried out on the liquid, by filling into glass vials and autoclaving at 120°C for 15 minutes. Alternatively, further processing is carried out on the liquid by filling into glass vials, freezing at -50°C, and lyophilization to give an aseptic lyophilized powder. The lyophilized vials are reconstituted by adding sterile water and hand mixing for 1 to 5 minutes, to give a reconstituted liquid of desired concentration which is ready for injection. The resulting concentrations of ferrioxamine and dermatan sulfate are measured and vial quantities confirmed by standard reverse-phase HPLC and macromolecular size exclusion HPLC methods, respectively.

Multiple batches of Ferrioxamine:Dermatan Sulfate Agent have been prepared. *In vitro* test results for a representative batch are as follows: ferrioxamine:dermatan sulfate ratio: 77.5:22.5 (w/w); solubility of agent, 550 mg/mL; water:octanol partition, 17,600 (± 2,750):1; concentration of ferrioxamine, 0.166 mmol/mL; concentration of dermatan sulfate, 32 mg/mL; molecular weight of dermatan sulfate, Opocrin type 435, MN = 18,000 daltons; sulfate/carboxylate ratio of dermatan sulfate, 1.0 ± 0.15; ferrioxamine and dermatan purities, nominal ± 10%; pH, 6.5-7.9; viscosity, 3.8-4.2 centipoise; osmolality, 475-525 mOsm/Kg; R1, 1.5-1.8 [mmol.sec]-1; oversized particles, within USP guidelines for small-volume parenterals; Anticoagulant activity, less than 4.5 U.S.P. Units/mg (modified USP XXII assay); binding of ferrioxamine active to dermatan carrier, at least 92% retained (dialysis for 3 hours against 200 volumes, 500 daltons molecular weight cutoff).

*In vitro* stability of Ferrioxamine:Dermatan Sulfate Agent under accelerated conditions, indicate the following. (a) The liquid form is stable, by the preceding physicochemical and HPLC parameters for longer than 6 months at 4°C, 22°C and 40°C; is slightly unstable at 2 to 6 months at 60°C, and degrades significantly within 1 to 3 days at 80°C. (b) The liquid form can be autoclaved as above, with less than 3% degradation of ferrioxamine. (c) The lyophilized form is stable with respect to all parameters (above), including oversized particles; and is projected to be stable over storage periods of multiple years.

### EXAMPLE 6

### Preparation of Paired-ion Agents of Ferrioxamine bound to Heparin

Ferrioxamine:dermatan sulfate paired-ion agents are prepared by mixing appropriate ratios of water solutions of ferrioxamine (as in Example 5, above) with (a) beef lung heparin of modal MW between approximately 8,000 daltons; and (b) porcine heparin of modal MW between approximately 10,000 daltons and 20,000 daltons. A range of ratios of ferrioxamine to heparin or heparin fragment are prepared between a low of 1:99 (wt/wt) of ferrioxamine:heparin or heparin fragment; and a high of 30:70 (wt %) of ferrioxamine:fragment. Using 0.1 to 1.0 N NaOH, the pH of the mixture is adjusted to between 5.5 and 8, the mixture is stirred continuously for 0.5 to 72 hours and the pH re-adjusted between 5.5 and 8. This ferrioxamine:heparin mixture is passed through a 0.22 *µ*m filter to remove any residual insoluble iron oxides-hydroxides and render the liquid agent aseptic. The aseptic agent is stored at 4°C. As indicated, further processing is carried out by filling the aseptic liquid in glass vials, followed by freezing and lyophilizing, to render the agent as an aseptic lyophilized powder. The lyophilized vials are reconstituted by adding sterile water and hand mixing for 1 to 5 minutes, to give a reconstituted liquid of desired concentration which is ready for injection. The resulting concentrations of ferrioxamine and heparin are measured and vial quantities confirmed by standard reverse-phase HPLC and macromolecular size exclusion HPLC methods, respectively.

### EXAMPLE 7

### Preparation of Non-anticoagulant Heparin Carrier by glycine Derivatization

The anticoagulant activity of heparin can be reduced to almost negligible activity by derivatizing its carboxylate groups with glycine residues as reported [Danishefsky *et al.* (1971); Danishefsky *et al.* (1972)]. This non-anticoagulant heparin (Nac-heparin) can then be utilized as a modified glycosaminoglycan carrier. According to one present method of glycine conjugation, 0.75 g of heparin is weighed into a 100 mL beaker and dissolved in 25 mL of pharmaceutical-grade water. Glycine, 0.75 g, is added and the pH of the resulting solution adjusted to 4.75 with 0.10 N HCl. 1-ethyl-3-(3-dimethylaminopropyl)carbodiimide HCl (EDC), 0.75 g, is weighed into a separate vial, solubilized by adding a minimum amount of water, and the pH adjusted to 4.75 with 0.10 M HCl. Aliquots of the EDC solution are added to the mixture of glycine-glycosaminoglycan over a one hour period. After each addition of EDC, the pH is adjusted to maintain it at 4.75. After addition of all EDC, the reaction is allowed to proceed for an additional two hours with constant stirring and periodic pH adjustment. The glycine-heparin conjugate (Gly-HEP) is then precipitated by addition of 3 volumes of absolute ethanol. The precipitate is collected by centrifugation at 4500 rpm (≈ 2500 x g) for 15 minutes; and washed three times with 20-mL aliquots of ethanol with recentrifugation.

### EXAMPLE 8

### Preparation of Paired-ion Agents of Ferrioxamine bound to Glycosaminoglycans, Modified and Derivatized Glycosaminoglycans of: heparan sulfate, non-anticoagulant heparin oversulfated dermatan sulfate chondroitin sulfate, oversulfated chondroitin sulfate and the bacterial Sulfatoid, pentosan polysulfate

Ferrioxamine paired-ion agents are prepared with various glycosaminoglycan carriers by mixing appropriate ratios of water solutions of ferrioxamine (as in Example 5, above) with the following glycosaminoglycans: (a) heparan sulfate of MN = 8,500 daltons; (b) non-anticoagulant heparin SPL, ++ of MN = 10,500 daltons; (c) oversulfated dermatan sulfate of MN = 19,000 daltons; (d) chondroitin sulfate of MN = 23,400 daltons; (e) oversulfated chondroitin sulfate of MN = 14,000 daltons; and (f) pentosan polysulfate of MN = 2,000 daltons. The ratios of ferrioxamine to glycosaminoglycan and sulfatoid carriers are prepared to give a payload of [77.5:22.5 % (w/w) of ferrioxamine to carrier] (adjusted) by a scaling factor of [(mEq sulfates/mg of carrier as above) / (mEq sulfates/mg of beef lung heparin*)]. Using 0.1 to 1.0 N NaOH, the pH of the mixture is adjusted to between 5.5 and 8, the mixture is stirred continuously for 0.5 to 72 hours and the pH re-adjusted between 5.5 and 8. This ferrioxamine:heparin mixture is passed through a 0.22 *µ*m filter to remove any residual insoluble iron oxides-hydroxides and render the liquid agent aseptic. The aseptic agent is stored at 4°C. As indicated, further processing is carried out by filling the aseptic liquid in glass vials, followed by freezing and lyophilizing, to render the agent as an aseptic lyophilized powder. The lyophilized vials are reconstituted by adding sterile water and hand mixing for 1 to 5 minutes, to give a reconstituted liquid of desired concentration which is ready for injection. The resulting concentrations of ferrioxamine and heparin are measured and vial quantities confirmed by standard reverse-phase HPLC and macromolecular size exclusion HPLC methods, respectively.

Although not prepared in the present application, it is apparent that by combining the teaching of the present Example with those of previous disclosures 07/880,660, 07/803,595, and 07/642,033, ferrioxamine complexes can be similarly prepared with additional acidic saccharides, including sucrose octasulfate and sulfated cyclodextrins; with additional glycosaminoglycans, including keratan sulfate and hyaluronate; and with additional sulfatoids, including the bacterial sulfatoid, dextran sulfate.
* For beef lung heparin, mEq SO₃⁻/g carrier = 4.4.

### EXAMPLE 9

### Preparation of Paired-ion Agents of Gd(III):DTPA-Lys bound to Dermatan Sulfate Carrier

Gd(III):DTPA-Lys:Dermatan Sulfate paired-ion agents are prepared by mixing the water solutions of Gd(III):DTPA-Lys with dermatan sulfate of modal MW between approximately 5,000 daltons and 45,000 daltons (as in Example 5, above), and in particular, dermatan sulfate of MN = 18,000 (Opocrin, S.p.A., Modena, Italy, 435 type), to form a final solution ratio of 75:25% (w/w) of the Gd(III):DTPA-Lys active to the Dernatan Sulfate carrier. Several stable Agent variations of the resulting liquid have been prepared, wherein the concentration of Gd(III):DTPA-Lys ranges from 0.166 to 0.415 mmol/mL, and the respective concentration of dermatan sulfate ranges from 35 to 87.5 mg/ml. The T1 relaxivity (R1) of Gd (III) :DTPA-Lys = 4.2.

### EXAMPLE 10

### Preparation of a Basic Iron-porphine Chelate; and Paired-ion Binding to Heparin

The soluble, tetra-basic porphine, 5,10,15,20-tetrakis(1-methyl-4-pyridyl)-21H-23-Hporphine, 40 mg as the tetra-p-tosylate salt, is refluxed with Fe(II) chloride, 30 mg, for 2 hours in 20 mL of dimethylformamide. Evidence of iron complexation is observed in the form of a red to dark green color. Solvent was removed by evaporation, the solid product dissolved in water. The pH is adjusted to 7.5 to insolubilize excess ferric iron, followed by filtration of the iron-porphine product. A 2 mg/mL solution of iron-porphine complex and ca. 100% product yield is confirmed by inductively coupled plasma atomic absorption. A comparable reaction in water gives ca. 70% yield.

This iron-porphine complex is added to beef lung heparin dissolved in water, ca. 8 Kd, at ratios ranging from 1:20 to 20:1 (iron-porphine:heparin). This resulted in clear solutions without precipitates. Binding of iron-porphine to heparin is nearly 100% as evaluated by dialysis against water for 16 hours, using bags with molecular weight cutoffs of 3.5 Kd and 12 Kd. Iron-porphine alone is nearly completely dialyzed. UV-Visible spectrophotometric titration indicates maximum binding occurs at a molar ratio of 18:1 (iron-porphine:heparin). Since the beef lung heparin used is known to have approximately 18 available strongly acidic (sulfate) groups per mole (and per heparin chain), these results indicate strong ionic interaction and stable (to dialysis) binding of the basic tetraamine porphine complex to the sulfate groups of heparin.

### EXAMPLE 11

### Preparation of a Basic Triethylenetetraamine-iron Chelate; and Paired-ion Binding to Heparin and Sucrose Octasulfate

Soluble complexes of triethylenetetraamine and iron(III) are formed by dissolving 1.0 g of triethylenetetraamine.2HC1 (Syprine™) (Merck, West Point, PA) in water and adding a 1:1 mole ratio of iron chloride under acidic conditions (pH = 2) to give a clear yellow solution. Using 0.1 N NaOH, the pH is adjusted to 6.8, giving a red solution indicative of iron complexation. This solution develops a feathery red precipitate, indicative of intermolecular aggregation of the iron-triethylenetetraamine complex.
(a) To this resulting aqueous dispersion of complex is added beef lung heparin, to give final complex-to-heparin ratios of between 95:5 and 5:95 (by weight). At a ratio of 65:35 (complex:heparin) and higher ratios of heparin, heparin completely solubilizes the complex. This apparent solubilization is indicative of paired-ion binding between triethylenetetraamine-iron and heparin.
(b) To the aqueous dispersion of triethylenetetraamine-iron complex is added sucrose octasulfate (SOS), to give final complex-to-SOS ratios of between 95:5 and 5:95 (by weight). At a ratio of 65:35 (complex:SOS) and higher ratios of SOS, SOS causes the dispersion to become very much finer, indicative of paired-ion binding between triethylenetetraamine-iron complex and SOS. The absence of complete clarification of this SOS paired-ion system relative to that with heparin (above), is due to the much higher density of sulfates on SOS relative to heparin, which confers substantially increased intermolecular hydrogen bonding on the SOS system.

Although not directly exemplified, it will be apparent that polyamines with the homologous series CₓH_{x+y}N_{x-z}, which also form stable complexes with Iron(III), can also be used in place of triethylenetetraamine-iron complex and SOS in the present invention.

### Preparation of Covalent Conjugates of Deferoxamine Glycosaminoglycan Carriers

Substrates with electrophilic amine groups may be covalently conjugated reagents to nucleophilic carboxylate groups of acidic carriers, acidic saccharides and acidic glycosaminoglycans as reported [Danishefsky et *al.* (1971); Danishefsky et al. (1972); Janoki et al. 1983); Axen (1974); Bartling et *al. (1974);* Lin *et al.* (1975)]. The coupling reagents described in these references activate carboxylate groups toward nucleophilic attack. The mechanism involves formation of an activated intermediate resulting from reaction of the coupling reagent with the carboxylate residues on the carrier. The intermediate undergoes nucleophilic attack, typically by an amine functional group. This results in formation of a stable covalent conjugate, typically via an amide bond between the active and the carrier. Examples 12, 13, and 14 (below) describe the synthesis of ferrioxamine-heparin covalent conjugates, wherein the ferrioxamine is covalently bound to heparin via three different coupling reagents.

### EXAMPLE 12

### Preparation of a Covalent Ferrioxamine-Heparin Conjugate by 1-ethyl-3-(3-dimethylaminopropyl) Carbodiimide (EDC) Linkage

Aqueous ferrioxamine, 2.0 g, as prepared in Example 1, is adjusted to pH 4.75 by addition of 0.10 M HCl. Beef-lung heparin (Hepar-Kabi-Pharmacia, Franklin, OH), 0.75 g, is dissolved 5.0 mL of pharmaceutical-grade water and added to the ferrioxamine with constant stirring. The pH of the resulting solution is re-adjusted to 4.75 with 0.10 M HCl. The water-soluble carbodiimide, 1-ethyl-3-(3-dimethylaminopropyl) carbodiimide HCl (EDC), 2 g, is weighed into a scintillation vial, solubilized in a minimum amount of water, and the pH adjusted to 4.75 with 0.10 M HCl. Aliquots of EDC solution are pipetted into the mixture of ferrioxamine-heparin over a one hour period. After each addition of EDC the 0.10 M HCl is added to maintain the pH at 4.75. After addition of all EDC, the reaction is allowed to proceed for an additional two hours with constant stirring. The ferrioxamine-heparin conjugate is precipitated by addition of 3 volumes of absolute ethanol. This precipitate is collected by centrifugation at 4500 rpm (≈ 2500 x g) for 15 minutes and washed three times with 20 mL aliquots of ethanol plus centrifugation. The complex is further purified by redissolving in water and re-precipitating with 3 volumes of ethanol plus centrifugation. The final product is collected and dried over nitrogen. Ferrioxamine derivatization of heparin is confirmed by UV-visible absorbance spectroscopy of the ferrioxamine chelate at 430 nm and heparin analysis by size-exclusion HPLC chromatography.

### EXAMPLE 13

### Preparation of a Covalent Ferrioxamine-Heparin Conjugate by N-Ethoxycarbonyl-2-ethoxy-1,2-dihydroquinoline (EEDQ) Linkage

Beef-lung heparin (Hepar-Kabi-Pharmacia, Franklin, OH), 0.50 g, is weighed into a 3-necked 100 mL round bottom flask fitted with an inlet and outlet for N₂ purge. Anhydrous dimethylformamide (DMF), 20 mL, is added with constant stirring and the resulting suspension warmed to 50°C under a constant flow of nitrogen. A 30 mole excess (≈ 463.7 mg) of N-ethoxycarbonyl-2-ethoxy-1,2-dihydroquinoline (EEDQ) is added and the resulting suspension stirred at 50°C for 3 hours. The activated EEDQ-activated heparin is collected by centrifugation at 4500 rpm (≈ 2500 x g) for 10 minutes. The pellet is washed repeatedly with anhydrous DMF and then 3 times with acetone. The activated intermediate is dried under a stream of nitrogen.

An aliquot of ferrioxamine solution containing 766.3 mg of the iron complex, as prepared in Example 1, is pipetted into a 50 mL beaker and diluted to 25 mL with anhydrous DMF. In a separate 50 mL beaker, a known amount of EEDQ-activated heparin is suspended in 50 mL of anhydrous DMF with constant stirring. The DMF solution of ferrioxamine is pipetted slowly into the EEDQ-heparin suspension over a 5 minute period. The resulting suspension is stirred continuously for 3 hours at 40°C. After cooling to room temperature, the final product is collected by centrifugation, washed three times with anhydrous DMF, washed three times with acetone, and dried under nitrogen. Confirmation of conjugate formation is performed as in Example 12.

### EXAMPLE 14

### Preparation of a Covalent Ferrioxamine-Heparin Conjugate by Carbonyldiimidazole (CDI) Linkage

An activated intermediate of beef-lung heparin (Hepar-Kabi- Pharmacia, Franklin, OH) is prepared by weighing 3.0 g of heparin into a 50 mL round bottom flask and adding 25 mL of anhydrous dimethylformamide (DMF) with constant stirring. Carbonyl- diimidazole (CDI), 608.1 mg, (10 mole excess relative to heparin) is weighed into a separate vial and dissolved in 20 mL of anhydrous DMF. The DMF solution of CDI is added to the DMF-heparin suspension and stirred at 30°C for one hour. The CDI-activated heparin is collected by centrifugation, washed repeatedly with acetone to remove unreacted CDI and residual DMF, and dried under nitrogen.

The deferoxamine-heparin conjugate is prepared by weighing 1.0 g of the CDI-activated heparin into a 50 mL round bottom flask and suspending this in 25 mL of anhydrous DMF. Deferoxamine, 250 mg, prepared as in Example 1, is weighed into a separate round bottom flask and dissolved in 20 mL of anhydrous DMF. The deferoxamine free base solution is added slowly to the CDI-heparin suspension and stirred continuously for 16 hours at 75°C. The deferoxamine-heparin conjugate is collected by centrifugation at 4500 rpm (≈ 2500 x g) for 15 minutes, washed repeatedly with anhydrous DMF, washed repeatedly with acetone, and dried under nitrogen. The resulting product is dissolved in water, and its concentration determined by UV-Visible spectroscopy. A stoichiometric quantity of aqueous FeCl₃ is added and the resulting solution adjusted gradually to pH 6.5 and stirred for 2 hours. This results in a deep brown-red product. This ferrioxamine-heparin conjugate is separated from any residual substrates and intermediates by dialysis through a 2,000 MW cutoff bag against 150 volumes of water. The retentate is collected and concentrated by rotary evaporation. Confirmation of derivatization is performed as in Examples 12 and 13.

### EXAMPLE 15

### Preparation of a Covalent Heparin-Diethylenetriaminepentaacetate Conjugate (DTPA-heparin)

DTPA-functionalized carriers are prepared in aqueous media from the reaction of diethylenetriaminepentaacetic dianhydride (cDTPAA; Calbiochem-Bhering Corp.) and a molecule containing a nucleophilic functional group. Beef-lung heparin (Hepar-Kabi-Pharmacia, Franklin, OH), 1.5 g, is dissolved in 75.0 mL of 0.05 M HEPES buffer and the pH adjusted to 7.0 with 0.10 M NaOH. cDTPAA, 4.5 g (≈ 100 mole excess relative to heparin), is weighed out and divided into 20 equal (225 mg) aliquots. An aliquot of cDTPAA is added to the heparin solution every 3-5 minutes until all cDTPAA has been added. The pH of the solution is monitored continuously throughout cDTPAA addition and maintained at pH 7.0 with 0.10 M NaOH. After addition of the last aliquot of cDTPAA, the solution is stirred for an additional 30 minutes. The DTPA-heparin solution is dialyzed through 1000 MW bags against 150 volumes to remove non-conjugated DTPA. The resulting conjugate is concentrated by nitrogen-evaporation at 37°C and stored at 4°C.

### EXAMPLE 16

### Preparation of Gadolinium(III) and Iron(III) Chelates of DTPA-heparin Covalent Conjugate

The DTPA-heparin conjugate of Example 15 is further prepared in the form of paramagnetic metal chelates of the DTPA group with gadolinium(III) or Fe(III), by pipetting the required volume of DTPA-heparin into a 125 mL Erlenmeyer flask, adding a 1.5-to-10 mole excess of the paramagnetic metal ion oxide, as Gd₂O₃ or Fe(O)OH, and stirring for 24 to 36 hours at 37°C to obtain solubilization of the metal oxides sufficient for complete occupancy of the DTPA groups. The residual metal oxides are precipitated by centrifugation at 4500 rpm (≈ 2500 g), and the product separated from unreacted metal oxides by filtration through a Millipore 0.22 *µ*m GV-type filter, followed by dialysis against 150 volumes. The concentrations of chelated metal ion and heparin are determined by inductively coupled plasma (ICP) and size-exclusion HPLC, respectively. In the case of Gd(III), stoichiometric chelation is also confirmed by standard xylenol orange titration [Lyle *et al*. (1963)].

### EXAMPLE 17

### Toxicity Studies of Ferrioxamine:Dermatan Sulfate, 435 Type

Acute intravenous Toxicity Studies with 14-day recovery and necropsy are performed in male and female rats and male and female dogs. At standard i.v. injection rates of 0.075 mmol/Kg/min., significant signs generally occur only after 5-12.5 times the effective imaging dose of 0.155 mmol/Kg. The LD50 is much greater than 4.5 mmol/Kg and is limited by technical aspects of tail-vein infusion. At this rate, some rats can be infused with 10 mmol/Kg without untoward effects. At an artificially accelerated i.v. injection rate of 0.080 mmol/Kg, deaths in rats can be obtained, and the LD50 is between 2.5 and 3.0 mmol/Kg. Terminal necropsy reveals no abnormalities in any rats after i.v. injection of 2.2, 3.0 and 4.5 mmol/Kg (n =5 males and 6 females per dose level).

A pyramid acute i.v. toxicity study is performed in dogs at escalating doses of 0.5, 1.2 and 2.25 mmol/Kg and an infusion rate of 0.012 mmol/Kg/min in protocol studies. An acute symptom complex of hypotension can be obtained, which is minimal and reversible. No deaths occurred and terminal necropsy at 14 days revealed no abnormalities (n = 2 males and 2 females, all administered each of the three dose levels, with a 72-hour rest interval).

### EXAMPLE 18

### Ferrioxamine:Dermatan Sulfate Selective Contrast Agent: MRI Imaging of Lactating Breast Adenocarcinomas in Syngeneic Fisher 344 Female Rats; Plus Correlation with Special Histochemical Studies

As shown in FIG. 2A, FIG. 2B, FIG. 3A, FIG. 3B, FIG. 4A, FIG. 4B, FIG. 4C and FIG. 4D, T1-weighted MRI images (TR/TE - 800/45 and 550/23) are performed at 1.0 and 1.5 Tesla, before (Pre) and after (Post) intravenous (i.v.) injection of Ferrioxamine:Dermatan Sulfate, 435 type Selective Paramagnetic Contrast Agent (Example 5), at a Ferrioxamine dose of 0.155 mmol/Kg into Fisher 344 female rats, with syngeneic breast adenocarcinomas inoculated by trocar into the livers, such that tumor diameters at the time of imaging are between 1.0 cm and 2.5 cm. Tumors are not conspicuous on standard T1-weighted Precontrast images. Following injection of Ferrioxamine:Dermatan Sulfate Agent, the tumors (a) become rapidly and markedly enhanced at an early post-injection time (7 mins) (FIG. 2A-B); (b) display very sharp tumor boundaries against surrounding liver (FIG. 2A, FIG. 2B and FIG. 4A, FIG. 4B, FIG. 4C, and FIG. 4D), and discretely demarcated, darker central region of tumor necrosis (FIG. 2A, FIG. 2B) (allowing tumor perfusion and function to be spatially resolved and assessed within different, very small anatomical subregions); (c) exhibit sustained contrast for longer than 64 minutes postinjection (MPI) (FIG. 4A, FIG. 4B, FIG. 4C, FIG. 4D, MRI images; FIG. 5, quantitative region-of-interest, ROI, analysis) with continued very well defined tumor borders at prolonged imaging intervals. Correlation of these MRI images with microwave augmented iron stains of the freshly excised, 7 MPI tumors, indicate that tumor-site localization of the Ferrioxamine active occurs only when it is bound (non-covalently) to carrier (FIG. 6 and FIG. 7A) and not when administered in free form (Active alone) (FIG. 3A, FIG. 3B). As shown in FIG. 8A, FIG. 8B, and FIG. 8C, lung metastases of the liver tumor are rapidly and sensitively enhanced in very small 2-mm to 3-mm nodules at an early post-contrast interval; and this enhancement of the tumor at lung sites is also sustained for a prolonged period with high sensitivity plus retention of very sharp tumor boundaries against normal lung. The sustained intervals shown in FIG. 8A, FIG. 8B, and FIG. 8C are much longer than those typically reported for Gd:DTPA dimeglumine contrast enhancement at body organ sites.

### EXAMPLE 19

### Ferrioxamine:Dermatan Sulfate Selective Contrast Agent: MRI Imaging of Prostate AT-1 Carcinomas in Syngeneic Copenhagen Rats and Comparison with Gd(III)DTPA

As shown in FIG. 9A, FIG. 9B, FIG. 9C, FIG. 9D, FIG. 9E and FIG. 10A, FIG. 10B, FIG. 10C, FIG. 10D, and FIG. 10E, T1-weighted MRI images (TR/TE - 250/8) performed at 4.7 Tesla, before (Pre) and after (Post) intravenous (i.v.) injection of Ferrioxamine:Dermatan Sulfate, 435 type Selective Paramagnetic Contrast Agent prepared as in Examples 2 and 5, and injected i.v. at an Iron(III) dose of 0.155 mmol/Kg (FIG. 9A, FIG. 9B, FIG. 9C, FIG. 9D, FIG. 9E); compared to Gadolinium DTPA dimeglumine, injected i.v. at a Gd(III) dose of 0.100 mmol/Kg (FIG. 10A, FIG. 10B, FIG. 10C, FIG. 10D, FIG. 10E); each of these agents being administered to Copenhagen rats with syngeneic AT-1 prostate adenocarcinomas inoculated into previously prepared skin pouches [Hahn, *et al.* ], such that tumor diameters at the time of imaging are between 1.0 cm and 2.5 cm. Ferrioxamine:Dermatan Sulfate produces a rapid large enhancement of the Outer Rim of tumor and also of the Vascular Array which fans out from the tumor pedicle which carries a high majority of the tumor vasculature. Sustained contrast and delineation of these elements remains present through kinetic time points of 40 minutes. By comparison, following Gd:DTPA dimeglumine, the outer rim is not well delineated, even at the earliest post-contrast interval (7 MPI). Marked early contrast fading occurs overall in the tumor at 20 MPI, and some agent sequesters in the central, poorly perfused (cystic) regions of tumor (as is typically reported for Gd:DTPA when used for imaging at body sites). At 40 MPI, enhancement reverts to essentially background levels, and at 60 MPI, there is no residual contrast, except for central cystic regions.

### EXAMPLE 20

### MRI Contrast Enhancement of Acute Dog Myocardial Infarcts by Ferrioxamine:Dermatan Sulfate

As shown in FIG. 11A, FIG. 11B, FIG. 11C and FIG. 11D, T1-weighted MRI ECG-gated cardiovascular images are performed at 0.5 Tesla, before (Pre) and after (Post) rapid intravenous (i.v.) infusion of Ferrioxamine:Dermatan Sulfate, 435 type Selective Paramagnetic Contrast Agent injected i.v. at an Iron(III) dose of 0.155 mmol/Kg into German Shepherd dogs with acute, 90-min myocardial infarcts (ligature of proximal left anterior descending coronary artery) followed by reperfusion for ca. 90 minutes prior to contrast agent infusion. At 7 MPI, Ferrioxamine:Dermatan gives strong enhancement of the infarct zone, and in particular distinguishes the outer boundary of the infarct, which represents the putative marginal zone of the infarct amenable to potential recovery, from the central darker region, which represents the putative irreversible central infarct. Sustained strong enhancement and zonal demarcation is present through 40 MPI. Ferrioxamine injected without carrier at 0.155 mmol/Kg, gives no detectible enhancement. In these studies, infarct sizes and positions are documented by double dye infusion performed immediately after MRI imaging.

### EXAMPLE 21

### Comparison of MRI Tumor-imaging Potency In Vivo with Ferrioxamine Active Bound to Various Sulfated Glycosaminoglycans

Based on low anticoagulant activity, safety and projected site-localization potential, certain alternative glycosaminoglycan carriers and certain alternative physical forms of the resulting Selective MRI Contrast Agents are compared for their relative *in vivo* potencies of carrier-mediated tumor localization of bound Ferrioxamine. Because of its high spatial resolution and capacity to detect subtle quantitative differences in agent localization, the AT-1 prostate tumor model of Example 19 is used.

**Table 2**

| FIG. No. | Agent | Form Liquid/Lyo | [metal] mmol/mL | Dose mmol/kg | Relative Potency (scale of 1-6) |
|---|---|---|---|---|---|
| 19 | Gd:MPD-DTPA Dermatan-SO₃⁻ 435 type* | Liquid | 0.332 | 0.155 | 7 |
| 12 | Ferrioxamine Dermatan-SO₃⁻ 435 type * | Lyo | 0.415 | 0.155 | 4.0 |
| 13 | Gd:DTPA-Lys Dermatan-SO₃⁻ 435 type * | Liquid | 0.415 | 0.155 | 6 |
| 14 | Ferrioxamine Oversulfated Dermatan-SO₃⁻ | Lyo | 0.332 | 0.155 | 4.04.5 |
| 15 | Ferrioxamine Oversulfated Chondroitin-SO₃⁻ | Lyo | 0.332 | 0.155 | 5 |
| 16 | Ferrioxamine Heparan Sulfate | Lyo | 0.332 | 0.155 | 3.5 |
| | Ferrioxamine Dermatan Sulfate** | Lyo | 0.332 | 0.155 | 1.5 |
| The second and third last lines of the Table making a reference to Fig. 15 and 16 respectively relate to reference examples only. | | | | | |
| Carriers of shorter chain length than the glycosaminoglycans, namely pentosan polysulfate, are found to be less potent (typically only 2/6 on the scale above) and remain at the tumor site for intervals of less than about 20 minutes, whereas the GAGs shown in the table above, are much more potent and have considerably longer tumor site localization intervals. In comparing these carriers, there is a slight-to-moderate trend towards increased carrier potency based on carrier sulfate charge density. | | | | | |
| Lyo = Lyophilized powder form | | | | | |
| SO₃⁻ = Sulfate (*e.g.* SO₃⁻ = dermatan sulfate) | | | | | |

| | | | | | |
|---|---|---|---|---|---|
| * beef mucosa, purified, 18,000 daltons | | | | | |
| ** porcine mucosa, 19,600 daltons | | | | | |

### EXAMPLE 22

### Preparation of a N-Methyl-1,3 Propanediamine Derivative of DTPA (MPD-DTPA) and Chelation with Gadolinium (III)

The diethylenetriamine-pentaacetic acid anhydride (DTPA anhydride) solution is prepared by adding 180 ml of anhydrous dimethylformamide (DMF) into a 250 ml round bottom flask. The flask is fitted with a side arm addition funnel and contains a magnetic stir. While the DMF is stirring vigorously, 5 g (14 mmol) of DTPA anhydride (Sigma Chemical Co.) is added in 0.5 g portions over one hour. The resulting suspension is warmed to 60°C to 15 minutes or until the solution clears. The flask is removed from the heat and placed in an ice bath until the solution has equilibrated to 4°C.

The MPD-DTPA derivative is prepared by mixing 15 ml of DMF with 1.46 ml (14 mmol) of N-methyl-1,3 propanediamine (Sigma Chemical Co.) in the addition funnel. The MPD-DMF mixture in the side arm addition funnel is added to the cold (4°C), vigorously stirring DTPA anhydride solution, dropwise. A white precipitate forms throughout the addition. The suspension is allowed to stir overnight at room temperature. The MPD-DTPA derivative is collected by centrifugation at 2500g for 10 minutes and washed repeatedly with acetone (5 x 300 ml).

The product at this stage, in concentrated solution has a pH of 3.5, additional purification requires a solution pH of 7.0. The product MPD-DTPA derivative is dissolved in water and the pH is adjusted to 7 with 5 N NaOH. The product is lyophilized for 16 hours to dryness. The lyophilized material is dissolved in a minimum amount (40 ml) of warm (50°C) methanol for 15 minutes, cooled to room temperature, and precipitated with 10 volumes of acetone. The precipitate is collected by centrifugation at 2500g for 10 minutes. This material is again dissolved in warm methanol for 15 minutes, precipitated with 10 volumes of acetone and collected by centrifugation at 2500xg. The precipitate is washed repeatedly with acetone, dried under nitrogen and stored in a vacuum dessicator.

Formation of the MPD-DTPA conjugate is confirmed by infrared (IR) Spectroscopy (see FIG. 17A, FIG. 17B, FIG. 17C) and HPLC chromatograph. HPLC characterization is carried out using a cation exchange column (Dionex IonPac CS14, 4x250 mm, 8 micrometer, carboxylic acid) with a mobile phase consisting of 20 mM methanesulfonic acid in acetonitrile-water (99:1) at pH 1.8 and with UV detection at 220nm. This gives well separated, chromatographically pure (exceeding 99% purity) peaks for: (a) DTPA at 3.7 minutes; (b) N-methyl-1,1-propanediamine (20:1 molar ratio of MPD to DTPA required for detection, due to low UV absorbance of MPD) at 8.4 minutes; (c) the solution mixture of DTPA (or hydrolyzed DTPA anhydride) with MPD (1:1 molar ratio) at 3.7 minutes (only DTPA detected and MPD, due to very low extinction coefficient of MPD); and (d) MPD-DTPA conjugate (1:1 molar ratio) at 15.6 minutes. The product purity of (d) is greater than 93% by HPLC absorbance at 220 nm.

The chelating capacity of N-Methyl-1,3-propanediamine-DTPA (MPD-DTPA) is determined by titrating a small aliquot with 0.1 M GdCl₃ 5H₂O in 1 M ammonium acetate (pH 5.5) buffer, using Xylenol Orange (5%, w/v) as the colorimetric indicator of endpoint. Based on this titration, a stoichiometric quantity of 1 M GdCl₃ 5H₂O is added to a batch quantity of N-MPD-DTPA as follows: the bulk MPD-DTPA is dissolved in a minimum amount of water (ca. 300 mg/ml), 1M GdCl₃ 5H₂O is to the added while vigorously stirring, and the pH is adjusted from <4.0 to 7.0 with 5 N NaOH. The average chelating capacity is about 22% (by weight), with slight variation based on the extremely hygroscopic nature of the dry chelator.

### EXAMPLE 23

### Preparation of Paired-Ion Formulation of Gadolinium:MPD-DTPA:Dermatan Sulfate

The paired-ion formulation of gadolinium(Gd):MPD-DTPA:dermatan sulfate (using the new, special 435 Type dermatan sulfate, Opocrin) is prepared over a range of weight ratios from 10:1 to 1:10 of Gd:MPD-DTPA to dermatan sulfate, and is particularly prepared at one of the preferred ratios of 60% Gd:MPD-DTPA to 40% dermatan sulfate (w/w)(= a mole ratio of 43:1). These paired-ion formulations are prepared by dissolving the desired amount of dermatan sulfate at a concentration of 400 mg/ml and stirring in the Gd:MPD-DTPA as prepared in Example 22. This results in a hydrophilic, completely clear solution without any detectable molecular aggregates by laser light scattering analysis (Nicomp Instrument). Strong paired-ion binding between GdMPD-DTPA and dermatan sulfate is confirmed and evaluated by dialysis through a 500 MW cutoff bag for 3 hours, 150 volumes, and is assessed by ICP atomic absorption analysis of the retained Gd (mass balance = 95%). Very strong paired-ion binding is indicated by 73% retention of Gd within the bag for the Gd:MPD-DTPA:dermatan sulfate formulation prepared at 60:40% (Gd:MPD-DTPA to dermatan sulfate); compared to the much lower 23% retention within the bag for Gd:DTPA:dermatan sulfate when prepared at the same molar ratio of Gd:DTPA to dermatan sulfate.

Quantification of dermatan sulfate is performed by assessing the decrease in UV absorbance at 620 nm which occurs upon binding of the extremely strong binding (displacing) cationic dye, Azure A, as previously described [Klein *et al.* (1982: Grant *et al.* (1984).

The R1 potencies (T1 relaxivities) of (a) Gd:MPD-DTPA alone and (b) the 60:40% (w/w) paired-ion formulation of Gd:MPD-DTPA:dermatan sulfate, are evaluated using an IBM PC20 Minispectrometer, and both are determined to be 7.8 mmol⁻¹s⁻¹ (based on parallel determinations of Gd concentration by ICP atomic absorption). The equality of R1's for the Gd chelate alone and Gd chelate bound to dermatan sulfate, indicate that binding of the chelate to dermatan sulfate does not interfere with water diffusion and paramagnetic relaxation. Furthermore, the absence of R1 prolongation indicates an absence of increase in rotational correlation time, and hence, further corroborates that the size of the Gd:MPD-DTPA-dermatan sulfate molecular complex is relatively small (likely less than about 50,000-60,000 daltons). This further confirms a basis for the surprising and unexpected advantages of high tumor accessibility across even the relatively more (anatomically and filtration) intact portions of tumor neovascular endothelium, and also the very rapid renal clearance, both of which are observed in intact animals (see below). This result correlates with the absence of detectible molecular aggregates by laser light scattering (above). The remarkably high R1 of this new formulation is repeated multiple times and appears to correlate with enhanced water diffusion of the new Gd:MPD-DTPA conjugate (and also for the full dermatan sulfate product) in relation to Gd:DTPA with the MPD side group (R1 = ca. 4 [mmol. sec]⁻¹. The stability Kd of Gd:MPD-DTPA is greater than 10¹⁷.

### EXAMPLE 24

### Acute Murine Toxicity of Paired-Ion Formulation of Gadolinium:MPD-DTPA:Dermatan Sulfate

One of the formulations of EXAMPLE 22, Gd:MPD-DTPA:dermatan sulfate (at a 60:40 wt % of Gd:MPD-DTPA to dermatan sulfate; 435 Type dermatan sulfate, Opocrin) was tested for acute toxicity by intravenous tail-vein injection into 20-gram, male Balb/c mice (n = 6). When injections were performed over 10-12 minutes, the average LD50 = 11.0 mmol/kg (of Gd and chelator), with 3 mice surviving at an average of 9.9 mmol/kg and 3 mice dying at an average of 12.2 mmol/kg. When injections were performed more rapidly, over a 2-3 minute interval, the LD50's were moderately lower in dose. These results compare favorably to those of Gd:DTPA (dimeglumine), for which LD50 = 4.0 mmol/kg.

### EXAMPLE 25

### Acute Blood Clearance of Radiolabeled Paired-Ion Formulations of: 67Ga-labeled Deferoxamine:Dermatan Sulfate; and 111In-labeled MPD-DTPA:Dermatan Sulfate

In order to assess if dermatan sulfate carriers could confer their own very rapid and complete blood clearance properties to attached active substances (including non-covalently bound chelates), the formulations of Examples 2, 5, 21 and 22 (above) are modified such as to bind the radioactive single-photon-emitting (SPECT) metals, 67Ga or 111In, in place of the non-radioactive metal ions, Fe(III) or Gd(III).

For the 67Ga experiments, approximately 1.55 umole of deferoxamine (DFo)-dermatan sulfate (77.5:22.% wt %; DS Type 435, Opocrin) is labeled with approximately 800 uCi of 67Ga, by converting the 67Ga from a chloride to a citrate form and incubating it for 10 min at room temperature with DFo:dermatan sulfate at pH 5.5-6.5, injecting Copenhagen-strain rats intravenously in the tail vein with 0.39 umoles of DFo:dermatan sulfate to which is chelated ca. 200 uCi of 67Ga, obtaining serial gamma camera images over a 1-hour interval (and again at 24 and 48 hours), and analyzing the heart, upper abdominal region and pelvic regions of interest (ROI's) for blood, liver and renal clearances, respectively. The blood clearance t1/2 average = 18 minutes, with a very rapid t1/2 alpha component of 8 minutes plus a t1/2 beta component of 35 minutes. No liver clearance is observed at all. Renal clearance is very rapid, accounting for all of the discernable clearance and leading to rapid bladder activity. There is no significant residual activity in the snout, skeletal axis or regions of bone or bone marrow. In a control experiment, injection of 67GaDFo alone (without dermatan sulfate) also results in very rapid blood clearance, however, a significant fraction of the agent (ca. 30%) cleared quite rapidly (10-30 minutes) into the liver and bowel, producing high organ backgrounds in the liver and colon.

In a separate experiment wherein the Copenhagen rats had AT-1 prostate adenocarcinomas (1.0-4.5 cm in diameter) implanted in the back of the neck, the tumors become very rapidly (ca. 5 minutes) active (bright) with radionuclide agent, and the tumor counts per pixel exceed those of the blood and liver at all times after 15 minutes of injection, resulting in rapid, sensitive detection of the tumors. This corroborates the MRI imaging results in the same tumor model (Example 19).

In another experiment, the dose of DFo:dermatan sulfate is increased 100X from 1.55 umole/kg to 155 umol/kg (0.155 mmol/kg) while maintaining the dose of radionuclide constant at 200 uCi per rat, in order to assess the effects of MRI doses, dose augmentation and potentially therapeutic doses, on clearance half times. By visual assessment, clearance is very nearly identical to the 100-fold lower dose of agent (above), with only a very minimal, ca. 5-minute prolongation.

In a further separate experiment, 111In is converted to the acetate form at pH 5.5-6.5, used to radiolabel MPD-DTPA:dermatan sulfate (60:40 wt % MPD-DTPA:dermatan sulfate, 435 Type, Opocrin). Clearance times and organ clearance patterns (renal versus liver) are comparable to those of 67GaDFo:dermatan sulfate (above); and when tested, tumor uptake is also rapid and distinct.

These surprising and unexpected advantages of: (a) very rapid clearance over a 100-fold (or greater) dose eschelation, for two different actives non-covalently bound (by paired-ion binding) to dermatan sulfate; and (b) avoidance of liver and bowel clearance in the presence but not the absence of dermatan sulfate carrier, provide major advantages for low MRI and radionuclide imaging backgrounds in the blood and especially additionally, in the critical and difficult body regions of liver and mid-abdomen. Upon bladder catheterization, the pelvic region is also observed without substantial background interferences. Additionally, significant therapeutic regimens are enabled because of the only very gradual increase in blood and body clearance times with major dose increments of at least 2 orders of magnitude. These clearance properties, coupled with the selective (tumor) uptake properties shown in this Example and above, provide even further surprising and unexpected advantages for augmenting the differential between selectivity versus body residual and systematic toxicity.

### EXAMPLE 26

### Gadolinium:N-methyl-1,3,propanediamine-DTPA:Dermatan Sulfate (Gd:MPD-DTPA:DS) Selective Contrast Agent: MRI Imaging of Lactating Breast Adenocarcinomas in Syngeneic Fisher 344 Female Rats

As shown in FIG. 25A, FIG. 25B, FIG. 25C, FIG. 25D, FIG. 25E AND FIG. 25F, T1-weighted MRI images (TR-TE = 800/45) are performed at 1.0 Tesla, before (Pre) and after (Post) intravenous (i.v.) injection of Gd:MPD-DTPA:DS (DS = 435 Type, Opocrin) at a dose of 0.155 mmol/kg into Fisher 344 female rats with syngeneic breast adenocarcinomas inoculated by trocar into the livers, as in Example 18 (above). A T2 scout image (TR/TE = 2100/85) is performed in advance of the T1 image contrast series, in order to identify the approximate location(s) of tumor nodule(s) (FIG. 18A). This reveals 2 solid tumor nodules (right posterior liver) and one irregular tumor infiltrate (central liver region), all tumor sites subsequently being confirmed by gross visual inspection. These nodules are unidentifiable in the T1 (800/45) Precontrast (Pre) image (FIG. 18B), however following injection of Gd:MPD-DTPA:DS, all three tumor nodules: (a) become rapidly and exceedingly strongly enhanced at an early post-injection time of 7 minutes (FIG. 18C); (b) display rapid and prolonged (through 60 minutes) sharp tumor boundaries against the surrounding uninvolved liver (FIG. 18C, FIG. 18D, FIG. 18E), and exhibit prolonged (sustained) contrast through 60 minutes (FIG. 18F), with only a very slight degradation of the contrast gradient at the tumor boundaries at 60 minutes postinjection (MPI). In this animal model, the MRI contrast enhancement produced by Gd:MPD-DTPA:DS, is markedly greater (more potent on a dose basis) than that produced by the ferrioxamine:dermatan sulfate agent of Example 18; and is slightly to moderately greater (more potent on a dose basis) than that produced by Gd:DTPA-lysine:dermatan sulfate (prepared per Examples 3, 4 and 9; see also FIG. 13A, FIG. 13B, FIG. 13C, FIG. 13D, Example 21 and Table 2 for relative potency); both of the preceding agents containing less potent metal chelates, namely, with R1's of 1.6 and 4.2, respectively, compared to an R1 of 7.8 [mmol.sec]⁻¹ for Gd:MPD-DTPA:DS of the present Example. Also, the images of the present Example show all the following, surprising and unexpected advantages over Gd:DTPA (dimeglumine), as well as over all the reported liver-specific T1 and T2 contrast agents: (a) uptake by tumor proper without substantial uptake by the surrounding uninvolved liver; (b) enhanced tumor selectivity and sensitivity; (c) prolonged as well as immediate tumor uptake, for improved clinical flexibility of multi-site and multi-image acquisition without contrast fading or need for multiple contrast-agent injections; (d) improved contrast sharpness and brightness gradient at the tumor boundaries, for improved tumor staging and improved detection of small tumors; (e) improved detection of small metastases; and (f) improved detection of small invasive outgrowths, for enhanced prognostic and therapeutic monitoring information. Note that there is a minor blood-pool enhancement in the surrounding normal liver at all post-contrast times, strongly suggesting that an even lower dose than 0.155 mmol/kg would be highly effective, indicated and appropriate for optimal T1 imaging of Gd:MPD-DTPA:DS. This is because the Gd:MPD-DTPA chelate is substantially more potent [R1 = 7.8 (mmol.sec)⁻¹] than all of the others described herein, and hence, gives more of T2* darkening, as well as T1 brightening effects, per micromole of agent deposited in the tumor (see Example 26 for corroboration of this effect).

### EXAMPLE 27

### Gadolinium:N-methyll-1,3,propanediamine-DTPA:Dermatan Sulfate (Gd:MPD-DTPA:DS) Selective Contrast Agent: MRI Imaging of Prostate AT-1 Adenocarcinomas in Syngeneic Copenhagen Rats; Plus Correlation with Special Histochemical Stain

As shown in FIG. 20A, FIG. 20B, FIG. 20C, FIG. 20D, AND FIG. 20E, T1-weighted images (TR/Te = 250/80) are performed at 4.7 Tesla, before (Pre) and after (Post) intravenous (i.v.) injection of 0.155 mmol/Kg [Gd(III) dose] of the Gd:MPD-DTPA:DS (DS = 435 Type, Opocrin) selective contrast agent, as prepared in Examples 21 and 22, into AT-1 prostate adenocarcinomas grown in skin pouches of syngeneic Copenhagen rats (described and referenced in Example 18). Gd:MPD-DTPA:DS produces a rapid, extremely strong T1 contrast enhancement of the entire tumor at 7 minutes (FIG. 20B) and 20 minutes (FIG. 20C) post-injection (MPI), and a continued strong contrast enhancement of the tumor at 40 MPI (FIG. 20D) and 60 MPI (FIG. 20E), especially at the tumor rim and most especially at the basal tumor rim (where tumor host staging is typically assessed). Upon further experimental evaluation, the apparent moderate contrast darkening of central tumor regions which appears at 40 and 60 MPI, actually represents an overconcentration of the agent within these tumor regions, leading to T2* effects, which compete with the strong T1 brightening effects and artifactually darken the T1 contrast in these central tumor regions. This T2* artifact is detected and assessed by utilizing a T2 pulse sequence of TR/TE = 2500/250, (= selectively sensitive to T2* effects) and observing substantial contrast darkening at the more delayed post-contrast times. Hence, the very high R1 of Gd:MPD-DTPA:DS (relative to all of the preceding agents), in combination with an injected dose of 0.155 mmol/Kg, together with the very marked tumor uptake of agent and the paramagnetic response characteristics of the TR/TE = 250/80 pulse sequence at a 4.7 Tesla field, leads to an overly high local paramagnetic activity within the tumor as Gd:MPD-DTPA:DS accumulates over time, especially in the central regions of the tumor. The rim, and especially the basal rim, is relatively protected from this T2* darkening artifact, due to more rapid backdiffusion of agent into plasma at this basal site. The preceding results and considerations lead to the conclusion that a lower dose than 0.155 mmol/Kg is indicated for optimal T1 imaging with Gd:MPD-DTPA:DS, because the Gd:MPD-DTPA chelate is a substantially more potent T1 paramagnetic active than all of the others described herein. Note that in Example 25, there appears to be a slight overdose, as evidenced by the very slightly enhanced blood-pool background in the uninvolved liver surrounding the 3 liver tumor nodules. Nevertheless, these nodules are still exceptionally well visualized at all post-contrast times (7-60 MPI).

Correlation of these MRI images with a microwave augmented Prussian blue stain for Gd(III) metal ion is performed (as described in Example 18), for the Gd(III) of Gd:MPD-DTPA:DS which becomes localized in the outer 2/3 of the tumor mass excised at 60 MPI (and freshly frozen for sectioning and staining). (See FIG. 20). This shows strongly positive histochemical staining of almost all tumor cells, with a significant number of the tumor cells having positive staining of the nucleus as well (i.e., nuclear localization of the metal-ion marker). This very strong staining of nearly all tumor cells at 60 minutes, compared to the lighter staining of fewer numbers of (breast) tumor cells at 7 minutes (Example 18), and the additional nuclear localization seen here at 60 minutes but not in the (breast) tumor at 7 minutes (Example 18), strongly suggests that tumor-cell internalization proceeds over a 1-hour interval, and likely over the entire interval of time during which the dermatan-sulfate bound metal chelates remain at significant concentrations within the extracellular matrix is initially and rapidly loaded via local microvessels, by extremely rapid and selective extravasation across tumor-induced neovascular MRI endothelium -- see text above for tumor-selective induction and endothelial localization of GAG-binding receptors, including VEGF/VPF and others. The surprising and unexpected advantage of endothelial localization observed here for malignant prostate tumor, was also observed in Example 18 for malignant breast tumor. This corroborates the surprising and unexpected finding of Example 18 above, that tumor-induced neovascular endothelium, as well as tumor cells proper, are targets for binding, pumping, extravasation and tumor-cell internalization of the dermatan sulfate-bound (including non-covalently bound) classes of MRI contrast agents, and indeed for other active agents similarly bound to dermatan sulfates and GAGs. These findings of tumor endothelium, tumor matrix, tumor cell and nuclear localizations and accumulations, further provide the basis for selectively localizing therapeutic agents, whether metal chelates or other types of active substances.

### EXAMPLE 28

### Preparation of Deferioxamine (DFo)-Gallium:Dermatan Sulfate (DS)

Preparation of DFo-Ga:DS is accomplished in two parts using essentially purified Dermatan Sulfate (435 Type, Opocrin): first the preparation of a 70:30 (w/w) ratio of DFo:DS. Ratios are tested ranging from 1:10 to 10:1; the 70:30 ratio is one preferred formulation. The second step is to chelate gallium (Ga) with the DFo:DS complex to 61% of stoichiometry (chelation capacity). This is carried out by placing 1.15 gm (1.75 mmol) of deferioxamine mesylate (DFo-M) into a 20 ml beaker and dissolving the sample in 5 ml of water, separately weighing 401.7 mg of dermatan sulfate (DS), and dissolving in 1.5 mL of water. This entire DS solution is added to the 5 ml of DFo-M. The resulting mixture is incubated overnight at room temperature.

Chelation is accomplished by adding 335.7 mg (1.08 mmol) of Ga(NO₃)₃ 3H₂O to the DS:DFo-M mixture with stirring. The pH is adjusted to 7.5 using 5 N and 1N NaOH, and the entire batch is brought to a final volume of 10 ml. The prep is dialyzed sample is concentrated to appropriate metal concentration by nitrogen evaporation at 37°C. The final prep is a colorless or slightly yellow clear solution.

DFo is quantified by UV-visible spectrophotometry at 430 nm, by adding a FeCl₃ solution to the GaDFo:DS complex and monitoring the appearance of color at 430 nm as Fe transmetalates the Ga:DFo to form Fe:DFo is monitored at 430 nm. A standard curve of UV-visible absorption at 430 nm is used to quantify the total DFo concentration. The Ga is in turn, quantified by ICP and 61% occupancy of DFo by cold Ga confirmed. The DS concentration is evaluated on UV/VIS spectrophotometer at 620 nm by monitoring the decrease in optical density of azure A (cationic dye), based on its strong binding to Ds (as described above.)

### EXAMPLE 29

### Radiolabeling of Deferrioxamine (DFO)-Dermatan Sulfate (DS) Complex with Ga⁶⁷ and Ga⁶⁸

Radiolabeling is performed in two parts. conversion of ga⁶⁷ chloride (Syncor Nuclear Pharmacy) to Ga⁶⁷ citrate (similarly for Ga⁶⁸), and chelation of ga⁶⁷ by DFo:DS complex. To Ga⁶⁷ chloride solution (1 mCi/ml) is added 2.5 ml of 10 N HCl, with mixing. The mixture is extracted twice with 2.5 ml ether. From the ether extract, 1 mCi Ga⁶⁷ is removed into another test tube, and the ether is evaporated with nitrogen. The residue is dissolved with 0.075 ml Na-citrate (0.05 M) (13.3 mCi/ml), the pH is adjusted to 6.5 (2 ul of 1 N NaOH). Chelation of Ga⁶⁷ by the DFo:DS complex is accomplished by transferring 0.025 ml of the dissolved residue (Ga⁶⁷ citrate, 330 uCi) into a separate tube and adding 0.002 ml of DFo-DS complex (0.34 umoles -- already occupied by 61% cold Ga) to the Ga⁶⁷ citrate, and adjusting the pH to 6.5. This mixture is incubated for 10 minutes to allow complete chelation, and the material is ready for injection into test animals.

### EXAMPLE 30

### Radiolabeling of MPD-DTPA:Dermatan Sulfate (DS) Complex with In¹¹¹

The radiolabeling of MPD-DTPA complex is performed in two parts: conversion of In¹¹¹ chloride to In¹¹¹ acetate and chelation of In¹¹¹ by MPD-DTPA complex. Conversion of In¹¹¹ chloride salt (Syncor Nuclear Pharmacy) to In¹¹¹ acetate is performed by pipetting 0.005 ml of In¹¹¹ chloride (2 mCi) into a test tube and evaporating under nitrogen to dryness. The residue is resuspended in a known small volume of water.

Chelation of In¹¹¹ by MPD-DTPA is accomplished by transferring 0.025 ml of the dissolved residue (In¹¹¹ acetate) (330 uCi) to another test tube, adding 0.002 ml of MPD-DTPA (0.34 umoles) to the In¹¹¹ acetate, and adjusting the pH to 6.5. The sample mixture is incubated for 20 minutes to assure complete chelation.

### EXAMPLE 31

### Preparation of Deferioxamine (DFo) -Gallium:Dermatan Sulfate (DS)

Preparation of DFo-Ga:DS is accomplished in two parts using essentially purified Dermatan Sulfate (435 Type, Opocrin): first the preparation of a 70:30 (w/w) ratio of DFo:Ds. Ratios are tested ranging from 1:10 to 10:1; the 70:30 ratio is one preferred formulation. The second step is to chelate Gallium (Ga) with the DFo:DS complex to 61% of stoichiometry (chelation capacity). This is carried out by placing 1.15 gm (1.75 mmol) of deferioxamine mesylate (DFo-M) into a 20 ml beaker and dissolving the sample in 5 ml of water, separately weighing 401.7 mg of dermatan sulfate (DS), and dissolving in 1.5 mL of water. This entire DS solution is added to the 5 ml of DFo-M. The resulting mixture is incubated overnight at room temperature.

Chelation is accomplished by adding 335.7 mg (1.08 mmol) of Ga(NO₃)₃ 3H₂O to the DS:DFo-M mixture with stirring. The pH is adjusted to 7.5 using 5 N and 1N NaOH, and the entire batch is brought to a final volume of 10 ml. The prep is dialyzed against deionized water through 500 molecular weight cutoff bags for two hours to remove excess salt, and the dialyzed sample is concentrated to appropriate metal concentration by nitrogen evaporation at 37°C. The final prep is a colorless or slightly yellow clear solution.

DFo is quantified by UV-visible spectrophotometry at 430 nm, by adding a FeCl₃ solution to the GaDFo:DS complex and monitoring the appearance of color at 430 nm as Fe transmetalates the Ga:DFo to form Fe:DFo is monitored at 430 nm. A standard curve of UV-visible absorption at 430 nm is used to quantify the total DFo concentration. The Ga is in turn, quantified by ICP and 61% occupancy of DFo by cold Ga confirmed. The DS concentration is evaluated on UV/VIS spectrophotometer at 620 nm by monitoring the decrease in optical density of azure A (cationic dye), based on its strong binding to DS (as described above).

### EXAMPLE 32

### Radiolabeling of Deferioxamine (DFo)-Dermatan Sulfate (DS) Complex with Ga⁶⁷ and Ga⁶⁸

Radiolabeling is performed in two parts. conversion of Ga⁶⁷ chloride (Syncor Nuclear Pharmacy) to Ga⁶⁷ citrate (similarly for Ga⁶⁸), and chelation of ga⁶⁷ by DFo:DS complex. To Ga⁶⁷ chloride solution (1 mCi/ml) is added 2.5 ml of 10 N HCl, with mixing. The mixture is extracted twice with 2.5 ml ether. From the ether extract, 1 mCi Ga⁶⁷ is removed into another test tube, and the ether is evaporated with nitrogen. The residue is dissolved with 0.075 ml Na-citrate (0.05 M) (13.3 mCi/ml), the pH is adjusted to 6.5 (2 ul of 1 N NaOH). Chelation of Ga⁶⁷ by the DFo:DS complex is accomplished by transferring 0.025 ml of the dissolved residue (Ga⁶⁷ citrate, 330 uCi) into a separate tube and adding 0.002 ml of DFo-Ds complex (0.34 umoles -- already occupied by 61% cold Ga) to the ga⁶⁷ citrate, and adjusting the pH to 6.5. This mixture is incubated for 10 minutes to allow complete chelation, and the material is ready for injection into test animals.

### EXAMPLE 33

### Radiolabeling of MPD-DTPA:Dermatan Sulfate (DS) Complex with In¹¹¹

The radiolabeling of MPD-DTPA complex is performed in two parts: conversion of In¹¹¹ chloride to In¹¹¹ acetate and chelation of In¹¹¹ by MPD-DTPA complex. Conversion of In¹¹¹ chloride salt (Syncor Nuclear Pharmacy) to In¹¹¹ acetate is performed by pipetting 0.005 ml of In¹¹¹ chloride (2 mCi) into a test tube and evaporating under nitrogen to dryness. The residue is resuspended in a known small volume of water.

Chelation of In¹¹¹ by MNPD-DTPA is accomplished by transferring 0.025 ml of the dissolved residue (In¹¹¹ acetate) (330 uCi) to another test tube, adding 0.002 ml of MPD-DTPA (0.34 umoles) to the In¹¹¹ acetate, and adjusting the pH to 6.5. The sample mixture is incubated for 20 minutes to assure complete chelation.

### EXAMPLE 34

### Comparative In vivo Tumor Imaging with Three Different Forms of 67Ga and with One Form of In-111

Copenhagen rats with 2 to 5 cm AT-1 prostate adenocarcinomas grown in the skin pouch of the neck (as described above) are injected intravenously with ca. 0.35 umoles of the respective chelator, when present, and with 200 to 300 uCi/rat of the 67Ga isotope in various forms, and images are performed between 1 and 60 minutes, as follows.
(a) Ga-67 chloride (FIG. 21A, FIG. 21B). Blood pool labeling is strong and blood clearance is very slow, with very little clearance occurring by 60 minutes. Liver labeling (background) begins at an early time of 10-15 minutes and progresses as a function of transmetallation to transferrin and other plasma proteins.
(b) Ga-67:DFo:DS (435 Type, Opocrin) (FIG. 22A, FIG. 22B). The blood label clears very rapidly, and tumor uptake also occurs very rapidly, such that tumor is very well visualized at all times beyond 10 minutes of injection, with exceedingly good visualization at 30 to 60 minutes (and beyond). There is virtually no liver uptake or clearance. Instead, clearance is entirely by the renal route, leading rapidly to bladder activity. Renal clearance is essentially complete by 48 hours (image not shown).
(c) Ga-67:DFo (without DS) (FIG. 23A, FIG. 23B). The blood label clears very rapidly, but although a majority of the label clears renally, there is also moderate liver background detected as early as 11 minutes, which increases over the 60 minute imaging interval (as previously reported for DFo-metal clearance). Also, importantly the tumor uptake is markedly lower and more tansient than with DS present. By ROI quantification, there is minimal uptake over blood background.
   These results indicate that essentially purified dermatan sulfate formulations behave in the radionuclide mode, exactly as predicted from the MRI tumor-uptake and clearance data, above, namely that even at a 100-fold lower injected dose, binding to normal endothelium is negligible, tumor uptake is rapid and pronounced, and clearance is very rapid -- nearly as rapid as the Gd-67 chelator alone, but with complete avoidance of liver background -- a major surprising and unexpected advantage for imaging at this site. Hence, tumor-selectivity is confirmed and very advantageous. Also, optimal tumor imaging can be performed within about 1/2 hour of injection, another surprising an unexpected advantage for imaging efficacy, utility and safety.
(d) In-111:MPD-DTPA:DS (435 Type, Opocrin) versus In-111:MPD-DTPA without DS (FIG. 24). Imaging performed at 3 hours post-injection, shows continued very good visualization of the tumor with the full formulation In111:MPD-DTPA:DS, but not as good a visualization and much higher liver and bowel background with only the In111:MPD-DTPA chelate alone (i.e., without DS). This second study indicates, if a chelator is selected appropriate to the radionuclide ion (ionic radius and chelation mechanism), the essentially purified dermatan sulfate of the present invention is capable of the very rapid selective tumor-uptake and whole body clearance properties needed to approach both tumor imaging and therapy, with radionuclide and non-radionuclide therapeutic actives. These surprising and unexpected advantages are unsurpassed by any other currently available radionuclide imaging and therapeutic approaches.

### EXAMPLE 35

Clearance times of are assessed by quantifying the counts per pixel for regions of interest over heart and tumor, from the experiment of Example 34, (b), above.

FIG. 25A. The average blood clearance t1/2 is 18 minutes, with a very rapid alpha t1/2 of 8 minutes, and a slightly slower beta t1/2 of 35 minutes.

FIG. 25B. The single-component clearance t1/2 of tumor is 54 minutes, which is 7 times longer than the blood t1/2 alpha and is 3 times longer than the average blood t1/2. It is predicted from tumor-cell internalization of metal ions (above), that most of this early backdiffusion from tumor into plasma occurs from the extracellular matrix compartment of the tumor, and that a fraction of the internalized agent will have an extremely slow egress from the tumor. Additional studies are required to assess this.

### EXAMPLE 36

Additional radionuclide diagnostics and therapeutics enabled by the preceding Examples include (essentially purified dermatan sulfate = EPDS):
A. Tumor imaging agents
   (1) 67Ga:deferoxamine:EPDS (67Ga = single photon imaging agent, = SPECT)
   (2) 68Ga:deferoxamine:EPDS (68Ga = positron emission tomography agent, = PET)
   (3) 111In:deferoxamine:EPDS (111 Indium = SPECT)
   (4) 111In:N-methyl-MDP:EPDS
   (5) 123Iodine and 125Iodine -- via DIRECT LABELING of THE EPDS -- NOT A METAL CHELATE
   (6) 99mTc-basic metal chelates (non-peptides and peptides) :EPDS
   (7) lllIn-DTPA-octreotide:EPDS (octreotide = somatostatin receptor-binding peptide with exposed basic lysine group for binding to OSO3-groups of EPDS -- NOTE: octreotide binds tumor cell surfaces, hence, gives second addressing capabilities (i.e., "street" addresses), in addition to those of EPDS, within the tumor mass
   (8) 67 and 68Ga-ferrioxamine-octreotide:EPDS
   (9) 99mTc-basic metal chelate derivative of octreotide:EPDS
B. Tumor therapeutic agents
   (1) 1.a. (1), (3) and (4) w/ EPDS -- given in sufficiently high dose
   (2) 32P-labeled:EPDS -- DIRECT LABELING OF THE EPDS -- NOT A METAL CHELATE
      NOTE THE FOLLOWING FORMS OF PHOSPHATE ARE IMPORTANT POTENTIAL (PROPHETIC) RADIONUCLIDE FORMULATION OF EPDS:
      (a) General, Non-covalently bound to EPDS, via basic or amine derivatives of ethane-1-hydroxy-1,1-diphosphonic acids
      (b) Specific examples:
         (1) 3-amino-1-hydroxypropane-1,1-diphoschonic acid (APD):EPDS
         (2) Azacvcloheptvlidene-2,2-bisphosphonic acid
         (4) 4-amino-1-hydroxybutylidene-1,1-bisphosphonic acid (AHBuBP):EPDS
         (5) 6-amino-1-hydroxyhexylidene-1,1-bisphosphonic acid (AHHexBP):EPDS
      (b) General, covalently bound to EPDS
      (c) Specific examples:
         (1) Enzymatic phosphorylation of EPDS
         (2) Periodate-oxidized EPDS reacted covalently with H3PO4, KH2PO4, NaH2PO4
         (3) Amine-activated EPDS reacted covalently with H3PO4, KH2PO4, NaH2PO4
   (3) 90Y (90-Yttrium) chelated to:
      (a) most preferably, N-methyl-1,3-diamine-DTPA:EPDS
      (b) preferably, deferoxamine:EPDS
   (3) 131I-labeled EPDS -- DIRECT LABELING OF THE EPDS -- NOT A METAL CHELATE
   (4) 35S-labeled EPDS -- DIRECT LABELING OF THE EPDS BY RESULFATION REACTIONS, utilizing Sulfur-35 Na2SO4 -- NOT A METAL CHELATE
c. Tumor diagnostic and/or therapeutic agents
   (1) all of the radioisotopic forms of the metals of the present invention, except boron, which have sufficiently long and appropriate radioactive half lives for diagnostic or therapeutic applications in conjunction with the rapid targeting and clearance times of EPDS"
      [NOTE: boron (when included in a chemical boroleptic ("chemically bound form") which itself could bind EPDS, is potentially useful in accentuating external neutron-beam radiation therapy by accentuating neutron capture].

**Table 3**

| MULTIPLE MODELS OF TUMOR TARGETING WITH ACIDIC POLYSACCHARIDES AND GAGS | | |
|---|---|---|
| Animal Species | Tumor Type | Proof |
| 1. Rat | Breast (R3230) | MRI Histology |
| 2. Rat | Prostate (Dunning AT-1) | MRI Histology |
| 3. Rat | Hepatocellular Carcinoma (Morris 7777) | MRI Histology |
| 4. Mouse | Breast (MMT) (autochthonous) | MRI |
| 5. Mouse | Radiation-induced Fibrosarcoma (RIF) | MRI |
| 6. Nude Mouse | Melanoma (human) | MRI Histology |
| 7. Rabbit | Carcinoma (VX-2) | Histology |

### REFERENCES

Axen (1974) *Prostaglandins,* 5(1):45.

Bacchi *et al.* (1993) *Am. J. Pathol.,* 142:579.

Bartling *et al.* (1974) *Biotechnology and Bioengineering,* 16:1425.

Berse *et al.* (1992), *Molecular Biology of the Cell,* 3:211-220.

Bevilacqua *et al.* (1993) *J. Clin. Invest.,* 91:91.

Bevilaqua *et al.* (July 10, 1993) *Congress of Intl. Soc. of Thrombosis and Hemostasis.*

Boneu *et al.* (1992) *Heparin and Related Polysaccharides,* Plenum Press, NY, pg. 237.

Brechbiel *et al.* (1986) *Inorg. Chem.,* 25:2772-2781.

Connolly *et al.* (1989) *J. Clin. Invest.,* 84:1470-1478.

Cremers *et al.* (1994) *International Journal of Pharmaceutics,* 110:117-125.

Crumbliss *et al.* (1991), HANDBOOK OF MICROBIAL IRON CHELATES, Chapter 7, CRC Press.

Danishefsky *et al.* (1972) *Thrombosis Research,* 1:173.

Danishefsky *et al.* (1971) *Carbohydrate Research,* 16:199.

Dawes *et al.* (1989) *Thrombosis and Haemostasis,* 62 (3) :945-949.

Elices *et* al. (1990) *Cell,* 60:577-584.

Geraldes *et al.* (1985) *Proc. Soc. Mag. Res. Med.,* 2:860.

Grant *et al.* (1984) *Analytical Biochemistry,* 137:25-32.

Hahn *et al.* (1993) *Mag. Res. Imaging,* 11:1007.

Hashimoto *et al.* (1983) *J. Nucl. Med.* 24:123.

Huber *et al.* (1991) *Science,* 254:99-102.

Jakeman *et al.* (1992) *J. Clin. Invest.,* 89:244-253.

Janoki *et al.* 1983) *Int. J. Appl. Radiat. Isot.,* 34 (6) :871.

Kalishevskaya *et al.* (1988) *Eksp. Onkol.,* 10(4):59-62.

Kim *et al.* (1993) *Nature,* 362:841-844.

Kjellen *et al.* (1977) *Biochem. and Biophys. Res. Comm.,* 74:126-133.

Klein *et al.* (1982) *Analytical Biochemistry*, 124:59-64.

Landsberger (1987) U.S. Patent No. 4,710,493.

Levine (1993) *FASEB Journal,* 7:1242.

Li *et al.* (1993) *Bioconjugate Chem.* 4:275-283.

Lin *et al.* (1975) *Analytical Biochemistry*, 63:485.

Lindahl and Hook (1978) *Ann. Rev. Biochem.,* 47:385.

Lorant *et al.* (1993) *J. Clin. Invest.,* 92:559.

Lyle *et al.* (1963) *Talanta,* 10:1177.

Maeda *et al.* (1993) *Anti-Cancer Drugs,* 4:167-171.

Mascellani *et al.* (1994) *Thromb. Res.,* 74(6):605-615.

Mascellani *et al.* (1993) International Patent Application WO 93/05074.

Miller *et al.* (1994) *Am. J. Pathol.,* 145:574-584.

Montrucchio *et al.* (1993) *Am. J. Pathol.,* 142:471.

Munro *et al.* (1992) *Am. J. Pathol.,* 141:1397.

Nicosia *et al.* (1994) *Am. J. Pathol.,* 145:1023-1029.

Nikkari *et al.* (1993) *Am. J. Pathol.,* 143:1019.

Ramirez *et al.* (1973) *J. Macromol. Sci-Chem.,* A7(5):1035.

Ranney (1990) U.S. Patent No. 4,925,678.

Ranney (1992) U.S. Patent No. 5,108,759.

Ranney, Patent Application SN 07/642,033.

Ranney, Patent Application SN 07/803,595.

Ranney, Patent Application SN 07/880,660.

Ransohoff *et al.* (1993) *FASEB Journal,* 7:592.

Rice *et al.* (1991) *Am. J. Pathol.,* 138:385.

Sasseville *et al.* (1992) *Am. J. Pathol.,* 141:1021.

Senger *et al.* (1993) *Cancer and Metastasis Reviews,* 12:303-324.

Sessler *et al.,* U.S. 5,159,065.

Sessler *et al.,* U.S. 5,252,720.

Sessler *et al., U.S.* 4,935,498.

Sharon *et al.* (January 1993) *Scientific American,* pg. 83.

Sioussat *et al.* (1993) *Arch. Biochem. Biophys.,* 301:15-20.

Steinhoff *et al.* (1993) Am. *J. Pathol.* 142:481.

Strieter *et al.* (1992) *Am. J. Pathol.,* 141:1279.

Travis (1993) *Science,* 260:906.

Weindel *et al.* (1992) *BBRC,* 183(3):1167-1174.

Yamashiro *et al.* (1994) Am. *J. Pathol.,* 145:856-867.

## Claims

1. An agent comprising a radioisotopic metal-ion chelate having at least one excess basic or cationic group not involved in metal-ion chelation and being bound to selectively oversulfated dermatan sulfate, said oversulfated dermatan sulfate comprising oversulfated oligosaccharide sequences of L-iduronic acid-2-sulfate/galactosamine-4-sulfate or Ido-2-SO₃⁻/GalNAc-4-SO₃⁻ disaccharides.

2. The agent of claim 1, further defined as being at least about 15 weight percent metal-ion chelate.

3. The agent of claim 1, further defined as comprising a boron, magnesium, aluminum, gallium, germanium, zinc, cobalt, calcium, rubidium, yttrium, tcchnetium, ruthenium, rhenium, indium, iridium, platinum, thallium, tin or samarium metal-ion.

4. The agent of claim 1, wherein the metal ion is 67Ga, 68Ga, 111In, 99mTc, 90Yttrium, Indium-114m or platinum-193m.

5. The agent of claim 1, wherein said selectively oversulfated dermatan sulfate has a molecular weight of from about 8,000 daltons to about 45,000 daltons.

6. The agent of claim 1, wherein said selectively oversulfated dermatan sulfate is further defined as having a SO₃/COO⁻ ratio of between 0.7:1 and 1.8:1.

7. The agent of claim 1, wherein said metal-ion chelate has a formation constant for metal ions of at least about 10¹⁴.

8. The agent of claim 1, wherein the chelate includes a hydroxamate.

9. The agent of claim 1, wherein the chelate includes deferoxamine.

10. The agent of claim 1, wherein the chelate includes N-methyl-1,3-propanediamine-DTPA.

11. The agent of claim 1, wherein said metal-ion chelate is ferrioxamine.

12. The agent of claim 1, wherein said chclate includes a porphine, porphyrin, sapphyrin or texaphyrin.

13. The agent of claim 1, wherein said metal-ion chelate is 67Ga:deferoxamine, 68Ga:deferoxamine or 111In:N-methyl-1,3-propanediamine-DTPA.

14. The agent of claim 1, wherein said metal-ion chelate is further defined as comprising a polyaminocarboxylate or macrocyclic.

15. The agent of claim 14, wherein said polyaminocarboxylate is a basic or amine derivative of dicthylenetriaminctetraacetate.

16. The agent of claim 14, wherein said macrocyclic is a basic or aminc derivative of 1,4,7,10-tetraazacyclododecane-N,N',N",N"'-tetraacetate (DOTA).

17. The agent of claim 14, wherein said polyaminocarboxylate is N-methyl-1,3-propancdiamine-DTPA.

18. The agent of claim 1, wherein said oversulfated dermatan sulfate is defined further as selectively binding endothelial determinants induced at sites of disease.

19. An *in vivo* imaging agent chelate consisting of 67Ga: deferoxamine bound to a selectively oversulfated dermatan sulfate comprising oversulfated oligosaccharide sequences of L-iduronic acid-2-sulfate/ galactosamine-4-sulfate or Ido-2-SO₃⁻/GalNAc-4-SO₃⁻ disaccharides.

20. An *in vivo* imaging agent consisting or 68Ga: deferoxamine bound to a selectively oversulfated dermatan sulfate comprising oversulfated oligosaccharide sequences of L-iduronic acid-2-sulfate/galactosamine-4-sulfate or Ido-2-SO₃⁻/GalNAc-4-SO₃⁻ disaccharides.

21. An *in vivo* imaging agent comprising 111In:N-methyl-1,3-propanediamine-DTPA bound to selectively oversulfated dermatan sulfate, said oversulfated dermatan sulfate comprising selectively oversulfated oligosaccharide sequences of L-iduronic acid-2-sulfate/galactosamine-4-sulfate or Ido-2-SO₃⁻/GalNAc-4-SO₃⁻ disaccharides.

22. The agent of claim 1, wherein the metal ion of the metal-ion chelate is 99mTc.

23. The agent of claim 1, further comprising a peptide chelator of metal ions bound to said selectively oversulfated dermatan sulfate.

24. The agent of claim 23, where the peptide is octreotide.

25. The agent of claim 24, wherein the metal of said metal-ion chelatc is 67Ga, and the chelator is deferoxamine.

26. The agent of claim 24, wherein the metal of said metal-ion chelate is 68Ga and the chelator is deferoxamine.

27. The agent of claim 24, wherein the metal of said metal-ion chelate is 111In and the chelator is DTPA.

28. The agent of claim 1, wherein the chelator of said metal-ion chelate comprises a diaminc chemical side group or a diaminc chemical side group derivative.

29. An agent containing selectively oversulfated dermatan sulfate comprising oversulfated oligosaccharide sequences of L-iduronic acid-2-sulfate/ galactosamine-4-sulfate or Ido-2-SO₃⁻/CalNAc-4-SO₃⁻ disaccharides, said dermatan sulfate being bound to 123Iodine, 125Iodine, 131Iodine, 32Phosphorous or 35Sulfur.

30. An agent containing selectively oversulfated dermatan sutfate comprising oversulfated oligosaccharide sequences of L-iduronic acid-2-sulfate/ galactosamine-4-sulfate or ldo-2-SO₃⁻/GalNAc-4-SO₃⁻ disaccharides, said dermatan sulfate being bound to a radioactive basic or amine derivative of a 32P-diphosphonic acid.

31. The agent of claim 30, wherein the basic or amine derivative of 32P-diphosphonic acid is 3-amino-1-hydroxypropane-1,1-32P-diphosphonic acid, azacycloheptyliderie-2,2-32P-bisphosphonic acid, 4-amino-1-hydroxybutylidene-1,1-32P-bisphosphonic acid, or 6-amino-1-hydroxyhexylidene-1,1-32P-bispliosphonic acid.

32. The agent of claim 1, defined further as being in a combination with at least one of a buffer, saccharide, sulfated saccharide, or salt, to produce an osmotic strength suitable for parenteral administration, and as being an aqueous solution or a lyophilized or dry preparation suitable for aqueous reconstitution having the desired osmotic strength, and wherein said agent is aseptic or sterile.

33. The agent of claim 1, 19, 20, 21, 29 or 30 wherein said dermatan sulfate has at least about 220 U/mg of heparin cofactor II (HCII) activity.

## Patentansprüche

1. Mittel, enthaltend ein Chelat mit einem radioisotopischen Metallion und wenigstens einer überschüssigen basischen oder kationischen Gruppe, die nicht an der Chelatbildung mit dem Metallion beteiligt ist und an selektiv übersulfatiertes Dermatansulfat gebunden ist, wobei das übersulfatierte Dermatansulfat übersulfatierte Oligosaccharidsequenzen von L-Iduronsäure-2-sulfat/Galactosamin-4-sulfat- bzw. Ido-2-SO₃⁻/GalNAc-4-SO₃⁻-disacchariden umfaßt.

2. Mittel nach Anspruch 1, weiterhin dadurch definiert, daß es zu wenigstens etwa 15 Gew.-% aus Metallionchelat besteht.

3. Mittel nach Anspruch 1, weiterhin dadurch definiert, daß es ein Bor-, Magnesium-, Aluminium-, Gallium-, Germanium-, Zink-, Cobalt-, Calcium-, Rubidium-, Yttrium-, Technetium-, Ruthenium-, Rhenium-, Indium-, Iridium-, Platin-, Thallium-, Zinn- oder Samarium-Metallion enthält.

4. Mittel nach Anspruch 1, bei dem es sich bei dem Metallion um 67Ga, 68Ga, 111In, 99mTc, 90Yttrium, Indium-114m oder Platin-193m handelt.

5. Mittel nach Anspruch 1, bei dem das selektiv übersulfatierte Dermatansulfat ein Molekulargewicht von etwa 8.000 Dalton bis etwa 45.000 Dalton aufweist.

6. Mittel nach Anspruch 1, bei dem das selektiv übersulfatierte Dermatansulfat weiterhin dadurch definiert ist, daß es ein SO₃/COO⁻-Verhältnis zwischen 0,7:1 und 1,8:1 aufweist.

7. Mittel nach Anspruch 1, bei dem das Metallionchelat eine Bildungskonstante für Metallionen von wenigstens etwa 10¹⁴ aufweist.

8. Mittel nach Anspruch 1, bei dem das Chelat ein Hydroxamat umfaßt.

9. Mittel nach Anspruch 1, bei dem das Chelat Deferoxamin umfaßt.

10. Mittel nach Anspruch 1, bei dem das Chelat N-Methyl-1,3-propandiamin-DTPA umfaßt.

11. Mittel nach Anspruch 1, bei dem es sich bei dem Metallionchelat um Ferrioxamin handelt.

12. Mittel nach Anspruch 1, bei dem das Chelat ein Porphin, Porphyrin, Sapphyrin oder Texaphyrin umfaßt.

13. Mittel nach Anspruch 1, bei dem es sich bei dem Metallionchelat um 67Ga:Deferoxamin, 68Ga:Deferoxamin oder lllIn:N-Methyl-1,3-propandiamin-DTPA handelt.

14. Mittel nach Anspruch 1, bei dem das Metallionchelat weiterhin dadurch definiert ist, daß es ein Polyaminocarboxylat oder einen Macrozyklus umfaßt.

15. Mittel nach Anspruch 14, bei dem es sich bei dem Polyaminocarboxylat um ein basisches Derivat oder Aminderivat von Diethylentriamintetraacetat handelt.

16. Mittel nach Anspruch 14, bei dem es sich bei dem Macrozyklus um ein basisches Derivat oder Aminderivat von 1,4,7,10-Tetraazacyclododecan-N,N',N",N"'-tetraacetat (DOTA) handelt.

17. Mittel nach Anspruch 14, bei dem es sich bei dem Polyaminocarboxylat um N-Methyl-1,3-propandiamin-DTPA handelt.

18. Mittel nach Anspruch 1, bei dem das übersulfatierte Dermatansulfat weiterhin dadurch definiert ist, daß es sich selektiv an an Krankheitsherden induzierte Endotheldeterminanten bindet.

19. Chelatmittel zur in vivo-Bilderzeugung, enthaltend 67Ga:Deferoxamin, das an ein selektiv übersulfatiertes Dermatansulfat gebunden ist, welches übersulfatierte Oligosaccharidsequenzen von L-Iduronsäure-2-sulfat/Galactosamin-4-sulfat- bzw. Ido-2-SO₃⁻/GalNAc-4-SO₃⁻-disacchariden umfaßt.

20. Mittel zur in vivo-Bilderzeugung, enthaltend 68Ga:Deferoxamin, das an ein selektiv übersulfatiertes Dermatansulfat gebunden ist, welches übersulfatierte Oligosaccharidsequenzen von L-Iduronsäure-2-sulfat/Galactosamin-4-sulfat- bzw. Ido-2-SO₃⁻/GalNAc-4-SO₃⁻-disacchariden umfaßt.

21. Mittel zur in vivo-Bilderzeugung, enthaltend lllIn:N-Methyl-1,3-propandiamin-DTPA, das an ein selektiv übersulfatiertes Dermatansulfat gebunden ist, welches selektiv übersulfatierte Oligosaccharidsequenzen von L-Iduronsäure-2-sulfat/Galactosamin-4-sulfat- bzw. Ido-2-SO₃⁻/GalNAc-4-SO₃⁻-disacchariden umfaßt.

22. Mittel nach Anspruch 1, bei dem es sich bei dem Metallion des Metallionchelats um 99mTc handelt.

23. Mittel nach Anspruch 1, welches weiterhin einen Peptid-Chelatbildner umfaßt, der an das selektiv übersulfatierte Dermatansulfat gebundene Metallionen cheliert.

24. Mittel nach Anspruch 23, bei dem es sich bei dem Peptid um Octreotid handelt.

25. Mittel nach Anspruch 24, bei dem es sich bei dem Metall des Metallionchelats um 67Ga und bei dem Chelatbildner um Deferoxamin handelt.

26. Mittel nach Anspruch 24, bei dem es sich bei dem Metall des Metallionchelats um 68Ga und bei dem Chelatbildner um Deferoxamin handelt.

27. Mittel nach Anspruch 24, bei dem es sich bei dem Metall des Metallionchelats um 111In und bei dem Chelatbildner um DTPA handelt.

28. Mittel nach Anspruch 1, bei dem der Chelatbildner des Metallionchelats eine chemische Diaminseitengruppe oder ein chemisches Diamingruppenderivat umfaßt.

29. Mittel, enthaltend selektiv übersulfatiertes Dermatansulfat, welches übersulfatierte Oligosaccharidsequenzen von L-Iduronsäure-2-sulfat/Galactosamin-4-sulfat- bzw. Ido-2-SO₃⁻/GalNAc-4-SO₃⁻-disacchariden umfaßt, wobei das Dermatansulfat an 123Iod, 125Iod, 131Iod, 32Phosphor oder 35Schwefel gebunden ist.

30. Mittel, enthaltend selektiv übersulfatiertes Dermatansulfat, welches übersulfatierte Oligosaccharidsequenzen von L-Iduronsäure-2-sulfat/Galactosamin-4-sulfat- bzw. Ido-2-SO₃⁻/GalNAc-4-SO₃⁻-disacchariden umfaßt, wobei das Dermatansulfat an ein radioaktives basisches Derivat oder Aminderivat einer
32P-Diphosponsäure gebunden ist.

31. Mittel nach Anspruch 30, bei dem es sich bei dem basischen Derivat oder Aminderivat von 32P-Diphosphonsäure um 3-Amino-1-hydroxypropan-1,1-32P-diphosphonsäure, Azacycloheptyliden-2,2-32P-bisphosphonsäure, 4-Amino-1-hydroxybutyliden-1,1-32P-bisphosphonsäure oder 6-Amino-l-hydroxyhexyliden-1,1-32P-bisphosphonsäure handelt.

32. Mittel nach Anspruch 1, weiterhin dadurch definiert, daß es sich in einer Kombination mit wenigstens einem Puffer, Saccharid, sulfatierten Saccharid oder Salz befindet, wodurch sich eine für eine parenterale Verabreichung geeignete osmotische Stärke ergibt, und dadurch daß es sich um eine wäßrige Lösung oder eine für ein Wiederauflösen in Wasser geeignete lyophilisierte Zubereitung oder Trockenzubereitung mit der gewünschten osmotischen Stärke handelt, wobei das Mittel aseptisch bzw. steril ist.

33. Mittel nach Anspruch 1, 19, 20, 21, 29 oder 30, bei dem das Dermatansulfat eine Heparin-Cofaktor-II (HCII) Aktivität von wenigstens etwa 220 E/mg aufweist.

## Revendications

1. Agent comprenant un chélate d'ion métallique radioisotopique, ayant au moins un groupe basique ou cationique en excès qui n'est pas impliqué dans la chélation d'ion métallique, et qui est lié à un dermatan sulfate sélectivement sursulfaté, ledit dermatan sulfate sursulfaté comprenant des séquences oligosaccharidiques sursulfatées de disaccharides acide L-iduronique-2-sulfate/galactosamine-4-sulfate ou Ido-2-SO₃⁻/GalNAc-4-SO₃⁻.

2. Agent selon la revendication 1, défini en outre comme étant constitué d'au moins environ 15 % en poids de chélate d'ion métallique.

3. Agent selon la revendication 1, défini en outre comme comprenant un ion métallique de bore, magnésium, aluminium, gallium, germanium, zinc, cobalt, calcium, rubidium, yttrium, technétium, ruthénium, rhénium, indium, iridium, platine, thallium, étain ou samarium.

4. Agent selon la revendication 1, dans lequel l'ion métallique est l'ion 67Ga, 68Ga, 111In, 99mTc, 90Yttrium, indium-114m ou platine-193m.

5. Agent selon la revendication 1, dans lequel ledit dermatan sulfate sélectivement sursulfaté a une masse moléculaire d'environ 8000 daltons à environ 45 000 daltons.

6. Agent selon la revendication 1, dans lequel ledit dermatan sulfate sélectivement sursulfaté est en outre défini comme ayant un rapport SO₃/COO⁻ compris entre environ 0,7:1 et 1,8:1.

7. Agent selon la revendication 1, dans lequel ledit chélate d'ion métallique a une constante de formation des ions métalliques d'au moins environ 10¹⁴.

8. Agent selon la revendication 1, dans lequel le chélate comprend un hydroxamate.

9. Agent selon la revendication 1, dans lequel le chélate comprend la déféroxamine.

10. Agent selon la revendication 1, dans lequel le chélate comprend le N-méthyl-1,3-propanediamine-DTPA.

11. Agent selon la revendication 1, dans lequel ledit chélate d'ion métallique est la ferrioxamine.

12. Agent selon la revendication 1, dans lequel ledit chélate comprend une porphine, une porphyrine, une sapphyrine ou une texaphyrine.

13. Agent selon la revendication 1, dans lequel ledit chélate d'ion métallique est la 67Ga:déféroxamine, la 68Ga:déféroxamine ou le 111In:N-méthyl-1,3-propanediamine-DTPA.

14. Agent selon la revendication 1, dans lequel ledit chélate d'un ion métallique est défini en outre comme comprenant un polyaminocarboxylate ou un macrocyclique.

15. Agent selon la revendication 14, dans lequel ledit polyaminocarboxylate est un dérivé basique ou aminé du diéthylènetriaminetétraacétate.

16. Agent selon la revendication 14, dans lequel ledit macrocyclique est un dérivé basique ou aminé du 1,4,7,10-tétraazacyclododécane-N,N',N",N"'-tétraacétate (DOTA).

17. Agent selon la revendication 14, dans lequel ledit polyaminocarboxylate est le N-méthyl-1,3-propanediamine-DTPA.

18. Agent selon la revendication 1, dans lequel ledit dermatan sulfate sursulfaté est en outre défini comme liant d'une manière sélective les déterminants endothéliaux induits sur les sites d'une maladie.

19. Chélate formant agent d'imagerie in vivo, constitué de 67Ga:déféroxamine liée à un dermatanesulfate sélectivement sursulfaté comprenant des séquences oligosaccharidiques sursulfatées de disaccharides acide L-iduronique-2-sulfate/galactosamine-4-sulfate ou Ido-2-SO₃⁻/GalNAc-4-SO₃⁻.

20. Agent d'imagerie in vivo constitué de 68Ga:déféroxamine liée à un dermatan sulfate sélectivement sursulfaté comprenant des séquences oligosaccharidiques sursulfatées de disaccharides acide L-iduronique-2-sulfate/galactosamine-4-sulfate ou Ido-2-SO₃⁻/GalNAc-4-SO₃⁻.

21. Agent d'imagerie in vivo comprenant du 111In:N-méthyl-1,3-propanediamine-DTPA lié à du dermatan sulfate sélectivement sursulfaté, ledit dermatan sulfate sursulfaté comprenant des séquences oligosaccharidiques sélectivement sursulfatées de disaccharides acide L-iduronique-2-sulfate/galactosamine-4-sulfate ou Ido-2-SO₃⁻/GalNAc-4-SO₃⁻.

22. Agent selon la revendication 1, dans lequel l'ion métallique du chélate d'ion métallique est le 99mTc.

23. Agent selon la revendication 1, qui comprend en outre un chélatant peptidique d'ions métalliques liés audit dermatan sulfate sélectivement sursulfaté.

24. Agent selon la revendication 23, dans lequel le peptide est l'octréotide.

25. Agent selon la revendication 24, dans lequel le métal dudit chélate d'ion métallique est le 67Ga, et le chélatant est la déféroxamine.

26. Agent selon la revendication 24, dans lequel le métal dudit chélate d'ion métallique est le 68Ga, et le chélatant est la déféroxamine.

27. Agent selon la revendication 24, dans lequel le métal dudit chélate d'ion métallique est le 111In, et le chélatant est le DTPA.

28. Agent selon la revendication 1, dans lequel le chélatant dudit chélate d'ion métallique comprend un groupe latéral chimique de type diamine ou un dérivé d'un groupe latéral chimique de type diamine.

29. Agent contenant un dermatan sulfate sélectivement sursulfaté, comprenant des séquences oligosaccharidiques sursulfatées de disaccharides acide L-iduronique-2-sulfate/galactosamine-4-sulfate ou Ido-2-SO₃⁻/GalNAc-4-SO₃⁻, ledit dermatan sulfate étant lié à l'iode 123, à l'iode 125, à l'iode 131, au phosphore 32 ou au soufre 35.

30. Agent contenant un dermatan sulfate sélectivement sursulfaté comprenant des séquences oligosaccharidiques sursulfatées de disaccharides acide L-iduronique-2-sulfate/galactosamine-4-sulfate ou Ido-2-SO₃⁻/GalNAc-4-SO₃⁻, ledit dermatan sulfate étant lié à un dérivé radioactif basique ou aminé d'un acide 32P-diphosphonique.

31. Agent selon la revendication 30, dans lequel le dérivé basique ou aminé d'un acide 32P-diphosphonique est l'acide 3-amino-1-hydroxypropane-1,1-32P-diphosphonique, l'acide azacycloheptylidène-2,2-32P-bisphosphonique, l'acide 4-amino-1-hydroxybutylidène-1,1-32P-bisphosphonique ou l'acide 6-amino-1-hydroxyhexylidène-1,1-32P-bisphosphonique.

32. Agent selon la revendication 1, défini en outre comme étant en combinaison avec au moins un tampon, un saccharide, un saccharide sulfaté ou un sel, pour produire une force osmotique convenant à une administration parentérale, et comme étant une solution aqueuse ou une préparation lyophilisée ou sèche convenant à une reconstitution aqueuse ayant la force osmotique souhaitée, et où ledit agent est aseptique ou stérile.

33. Agent selon la revendication 1, 19, 20, 21, 29 ou 30, dans lequel ledit dermatan sulfate a une activité d'au moins environ 230 U/mg du co-facteur II de l'héparine (HCII).
